(19) **Europäisches Patentamt / European Patent Office / Office européen des brevets**

(11) **EP 4 737 482 A1**

(12) # EUROPEAN PATENT APPLICATION
published in accordance with Art. 153(4) EPC

(43) Date of publication:
06.05.2026 Bulletin 2026/19

(21) Application number: 24832429.5

(22) Date of filing: 26.06.2024

(51) International Patent Classification (IPC):
C07K 16/30 (2006.01)  C07K 14/725 (2006.01)
G01N 33/574 (2006.01)  A61P 35/00 (2006.01)
A61K 47/68 (2017.01)  C12N 15/113 (2010.01)
A61K 39/00 (2006.01)

(52) Cooperative Patent Classification (CPC):
A61K 39/00; A61K 47/68; A61P 35/00;
C07K 14/705; C07K 16/30; C12N 15/113;
G01N 33/575

(86) International application number:
PCT/KR2024/008897

(87) International publication number:
WO 2025/005651 (02.01.2025 Gazette 2025/01)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 27.06.2023  KR 20230082857
20.07.2023  KR 20230094413
25.06.2024  KR 20240082477

(71) Applicant: Atheon Bio, Inc.
Seongnam-si, Gyeonggi-do 13487 (KR)

(72) Inventors:
• CHOE, Soheui
Hwaseong-si Gyeonggi-do 18482 (KR)
• LEE, Hyun Soo
Seongnam-si Gyeonggi-do 13525 (KR)

(74) Representative: Dantz, Jan Henning et al
Loesenbeck - Specht - Dantz
Patent- und Rechtsanwälte
Am Zwinger 2
33602 Bielefeld (DE)

Remarks:
The complete document including Reference
Table(s) and the Sequence Listing(s) can be
downloaded from the EPO website

(54) **HUMANIZED ANTI-MYCT1 ANTIBODY AND USE THEREOF**

(57) The present invention relates to a humanized anti-MYCT1 antibody and a use thereof.

FIG. 21B

EP 4 737 482 A1

**Description**

[Technical Field]

The present invention relates to a humanized anti-MYCT1 antibody and use thereof.
The present invention claims priority from Korean Patent Application No. 2023-0082857, filed in the Republic of Korea on June 27, 2023, and Korean Patent Application No. 2023-0094413, filed in the Republic of Korea on July 20, 2023, the contents of which are incorporated herein by reference.

[Background Art]

Angiogenesis is a physiological process in which new blood vessels develop from pre-existing blood vessels. Angiogenesis is known to play a role in both normal and pathological processes. Angiogenesis is highly regulated by pro-angiogenic factors and anti-angiogenic factors, a process that is disrupted and dysregulated in cancer. Tumor-induced hypoxia increases the expression of angiogenic factors, which induce the formation of new blood vessels essential for tumor survival and proliferation. The vascular endothelial growth factor (VEGF) family, consisting of six growth factors (VEGFA-F), plays a key role in angiogenesis by binding to receptors VEGF receptors 1-3 (VEGFR1-3) and neuropilin. Angiogenesis may also be mediated independently of the VEGF pathway by the angiopoietin (Ang1-2)/Tie-2 pathway. Accordingly, over the past decade, drug development has focused on anti-angiogenesis as a strategy to deprive tumors of nutrients and inhibit tumor growth. However, despite the weak activity of these agents as single agents or in combination with chemotherapy, there still remains the problem that tumors overcome the effects and develop resistance.
Cancer immunotherapy emerged as an effective treatment for various cancers following the discovery of immune checkpoints. Immune checkpoint inhibitors (ICIs) have demonstrated long-lasting clinical activity against malignant tumors. ICIs activate tumor-fighting effector immune cells by blocking another mechanism hijacked by tumor "immune exhaustion." Primary resistance to ICIs occurs in tumors lacking tumor-infiltrating lymphocytes. In addition, tumors that initially respond to ICIs may develop secondary resistance due to defects in antigen presentation mechanisms and overexpression of co-inhibitory molecules.
Therefore, it is necessary to overcome the limitations of VEGF inhibitors and reprogram the tumor microenvironment (TME) by normalizing tumor blood vessels in order to facilitate immune cell migration into the TME.
To achieve this, the development of drugs targeting MYCT1 (MYC Target 1) is essential. Currently, no anti-MYCT1 antibody has been approved as a treatment for human diseases. Therefore, the development of anti-MYCT1 antibodies with strong binding affinity for MYCT1, excellent specificity, potent antibody-dependent cellular cytotoxicity (ADCC) and complement-dependent cytotoxicity (CDC) activities, and superior anti-tumor and anti-angiogenic activity through TME reprogramming is required.
[Non-patent document]
J. Cell. Mol. Med., 2016 Vol. 20, No. 3, pp. 471-481

[Disclosure]

[Technical Problem]

An object of the present invention is to provide a humanized antibody or antigen-binding fragment that binds to MYCT1.
Another object of the present invention is to provide a nucleic acid molecule encoding the antibody or antigen-binding fragment, an expression vector including the same, and a host cell transfected with the vector.
Still another object of the present invention is to provide an immunoconjugate including the antibody or antigen-binding fragment and a cytotoxic agent.
Yet another object of the present invention is to provide the antibody or antigen-binding fragment for use in the diagnosis or treatment of an MYCT1-associated disease.
Yet another object of the present invention is to provide the antibody or antigen-binding fragment for use in the diagnosis of sensitivity to immunotherapy.
Yet another object of the present invention is to provide a chimeric antigen receptor (CAR) including the antigen-binding fragment and an immune effector cell expressing the same.
However, the technical problems to be solved by the present invention are not limited to the above-mentioned problems, and other problems that are not mentioned may be clearly understood by those skilled in the art from the description below.

[Technical Solution]

In order to solve the above-described technical problems, one aspect of the present invention provides

an isolated humanized anti-MYCT1 antibody or antigen-binding fragment thereof including: at least one variable heavy chain region (VH) selected from the group consisting of SEQ ID NOs: 41 and 43 to 60; and at least one variable light chain region (VL) selected from the group consisting of SEQ ID NOs: 42 and 61 to 66; or
at least one VH selected from the group consisting of SEQ ID NOs: 92 and 94 to 101; and at least one VL selected from the group consisting of SEQ ID NOs: 93 and 102 to 115.

In one embodiment, the VH may include CDR1, CDR2, and CDR3 having amino acid sequences set forth in SEQ ID NOs: 3, 4, and 5, respectively, and the VL may include CDR1, CDR2, and CDR3 having amino acid sequences set forth in SEQ ID NOs: 6, 7, and 8, respectively.
In another embodiment, the VH may include CDR1, CDR2, and CDR3 having amino acid sequences set forth in SEQ ID NOs: 9, 10, and 11, respectively, and the VL may include CDR1, CDR2, and CDR3 having amino acid sequences set forth in SEQ ID NOs: 12, 13, and 14, respectively.
In still another embodiment, the antibody or antigen-binding fragment thereof may

include a VH sequence of SEQ ID NO: 43 and a VL sequence of SEQ ID NO: 42;
include a VH sequence of SEQ ID NO: 44 and a VL sequence of SEQ ID NO: 42;
include a VH sequence of SEQ ID NO: 45 and a VL sequence of SEQ ID NO: 42;
include a VH sequence of SEQ ID NO: 41 and a VL sequence of SEQ ID NO: 61;
include a VH sequence of SEQ ID NO: 46 and a VL sequence of SEQ ID NO: 62;
include a VH sequence of SEQ ID NO: 41 and a VL sequence of SEQ ID NO: 63;
include a VH sequence of SEQ ID NO: 44 and a VL sequence of SEQ ID NO: 63;
include a VH sequence of SEQ ID NO: 47 and a VL sequence of SEQ ID NO: 64;
include a VH sequence of SEQ ID NO: 48 and a VL sequence of SEQ ID NO: 64;
include a VH sequence of SEQ ID NO: 48 and a VL sequence of SEQ ID NO: 65;
include a VH sequence of SEQ ID NO: 49 and a VL sequence of SEQ ID NO: 65;
include a VH sequence of SEQ ID NO: 50 and a VL sequence of SEQ ID NO: 65;
include a VH sequence of SEQ ID NO: 51 and a VL sequence of SEQ ID NO: 65;
include a VH sequence of SEQ ID NO: 52 and a VL sequence of SEQ ID NO: 65;
include a VH sequence of SEQ ID NO: 53 and a VL sequence of SEQ ID NO: 65;
include a VH sequence of SEQ ID NO: 54 and a VL sequence of SEQ ID NO: 65;
include a VH sequence of SEQ ID NO: 55 and a VL sequence of SEQ ID NO: 64;
include a VH sequence of SEQ ID NO: 55 and a VL sequence of SEQ ID NO: 65;
include a VH sequence of SEQ ID NO: 56 and a VL sequence of SEQ ID NO: 65;
include a VH sequence of SEQ ID NO: 57 and a VL sequence of SEQ ID NO: 65;
include a VH sequence of SEQ ID NO: 58 and a VL sequence of SEQ ID NO: 65;
include a VH sequence of SEQ ID NO: 41 and a VL sequence of SEQ ID NO: 65;
include a VH sequence of SEQ ID NO: 58 and a VL sequence of SEQ ID NO: 42;
include a VH sequence of SEQ ID NO: 59 and a VL sequence of SEQ ID NO: 66; or
include a VH sequence of SEQ ID NO: 60 and a VL sequence of SEQ ID NO: 66.

In yet another embodiment, the antibody or antigen-binding fragment thereof may

include a VH sequence of SEQ ID NO: 92 and a VL sequence of SEQ ID NO: 102;
include a VH sequence of SEQ ID NO: 92 and a VL sequence of SEQ ID NO: 103;
include a VH sequence of SEQ ID NO: 92 and a VL sequence of SEQ ID NO: 104;
include a VH sequence of SEQ ID NO: 94 and a VL sequence of SEQ ID NO: 105;
include a VH sequence of SEQ ID NO: 95 and a VL sequence of SEQ ID NO: 105;
include a VH sequence of SEQ ID NO: 96 and a VL sequence of SEQ ID NO: 105;
include a VH sequence of SEQ ID NO: 97 and a VL sequence of SEQ ID NO: 105;
include a VH sequence of SEQ ID NO: 98 and a VL sequence of SEQ ID NO: 105;
include a VH sequence of SEQ ID NO: 99 and a VL sequence of SEQ ID NO: 106;
include a VH sequence of SEQ ID NO: 99 and a VL sequence of SEQ ID NO: 107;
include a VH sequence of SEQ ID NO: 99 and a VL sequence of SEQ ID NO: 108;
include a VH sequence of SEQ ID NO: 99 and a VL sequence of SEQ ID NO: 109;

include a VH sequence of SEQ ID NO: 99 and a VL sequence of SEQ ID NO: 110;
include a VH sequence of SEQ ID NO: 95 and a VL sequence of SEQ ID NO: 110;
include a VH sequence of SEQ ID NO: 100 and a VL sequence of SEQ ID NO: 110;
include a VH sequence of SEQ ID NO: 100 and a VL sequence of SEQ ID NO: 111;
include a VH sequence of SEQ ID NO: 101 and a VL sequence of SEQ ID NO: 111;
include a VH sequence of SEQ ID NO: 92 and a VL sequence of SEQ ID NO: 111;
include a VH sequence of SEQ ID NO: 101 and a VL sequence of SEQ ID NO: 112;
include a VH sequence of SEQ ID NO: 95 and a VL sequence of SEQ ID NO: 113;
include a VH sequence of SEQ ID NO: 100 and a VL sequence of SEQ ID NO: 114; or
include a VH sequence of SEQ ID NO: 95 and a VL sequence of SEQ ID NO: 115.

The antigen binding fragment may include Fd, Fab, Fab', F(ab')2, dsFv, scFv, and a single domain antibody (sdAb), but is not limited thereto, and any fragment that possesses at least antigen-binding property may be used without limitation.

In another aspect, the present invention provides a nucleic acid molecule encoding the antibody or antigen-binding fragment thereof.

In still another aspect, the present invention provides a recombinant expression vector including the nucleic acid molecule and a host cell transfected with the expression vector.

In yet another aspect, the present invention provides an immunoconjugate including the antibody or antigen-binding fragment and a cytotoxic agent.

In yet another aspect, the present invention provides a pharmaceutical composition for preventing or treating an MYCT1-associated disease, including the antibody or antigen-binding fragment as an active ingredient.

In one embodiment, the MYCT1-associated disease may be an angiogenesis-associated disease or cancer.

In another embodiment, the pharmaceutical composition may be administered in combination with at least one additional therapeutic agent or therapeutic procedure.

The at least one additional therapeutic agent or therapeutic procedure may be selected from one or more of chemotherapy, targeted anticancer therapy, an oncolytic drug, a cytotoxic agent, immune-based therapy, a cytokine, a surgical procedure, a radiation procedure, a vaccine, and cell therapy.

In yet another aspect, the present invention provides a composition for diagnosing an MYCT1-associated disease, including the antibody or antigen-binding fragment thereof as an active ingredient.

In yet another aspect, the present invention provides a pharmaceutical composition for enhancing sensitivity to an immunotherapy agent in cancer, including the antibody or antigen-binding fragment thereof as an active ingredient.

In yet another aspect, the present invention

provides a chimeric antigen receptor (CAR) including an antigen-binding domain that specifically binds to MYCT1. Specifically, the antigen-binding domain may include:
at least one variable heavy chain (VH) region selected from the group consisting of SEQ ID NOs: 41 and 43 to 60 and SEQ ID NOs: 92 and 94 to 101; and at least one variable light chain (VL) region selected from the group consisting of SEQ ID NOs: 42 and 61 to 66 and SEQ ID NOs: 93 and 102 to 115.

In yet another aspect, the present invention provides an immune effector cell expressing the chimeric antigen receptor (CAR).

In yet another aspect, the present invention provides a method of treating an MYCT1-associated disease, including a step of administering to a subject a composition including a therapeutically effective amount of the antibody or antigen-binding fragment.

In yet another aspect, the present invention provides a method of enhancing sensitivity of a subject to an immunotherapy agent, including a step of administering to the subject a composition including a therapeutically effective amount of the antibody or antigen-binding fragment.

However, the technical problems to be solved by the present invention are not limited to the above-mentioned problems, and other problems that are not mentioned may be clearly understood by those skilled in the art from the description below.

[Advantageous Effects]

The antibody or antigen-binding fragment specifically binding to MYCT1 provided in the present invention exhibits excellent affinity and binding strength for MYCT1, thereby enabling the development of antibody-drug conjugates, bispecific antibodies, or chimeric antigen receptors (CARs) including the antibody or antigen-binding fragment. Furthermore, the antibody or antigen-binding fragment according to the present invention can be usefully applied to the treatment or diagnosis of MYCT 1-associated diseases, such as cancer or angiogenesis-associated diseases. Furthermore, the antibody or antigen-binding fragment according to the present invention can be used in combination with immunotherapy and can enhance sensitivity to such agents.

The effects of the composition according to one embodiment of the present invention are not limited to the above-mentioned effects, and other effects that are not mentioned will be clearly understood by those skilled in the art from the description below.

[Description of Drawings]

FIG. 1 shows the results of sodium dodecyl sulfate-polyacrylamide gel electrophoresis (SDS-PAGE) to confirm the expression of a parent antibody (2B4) and a humanized antibody in a supernatant during small-scale production.
FIG. 2 shows the results of SDS-PAGE to confirm the expression of a parent antibody (11B6) and a humanized antibody in a supernatant during small-scale production.
FIGS. 3A, 3B, and 3C show SDS-PAGE gels of the parent antibody (2B4) purified under non-reducing and reducing conditions during small-scale production (R: reducing).
FIGS. 4A and 4B show SDS-PAGE gels of the parent antibody (11B6) purified under non-reducing and reducing conditions during small-scale production (R: reducing).
FIGS. 5A, 5B, 5C, 5D, 5E, and 5F show size exclusion chromatography-high-performance liquid chromatography (SEC-HPLC) chromatograms of the parent antibody (2B4) and humanized and post-translational modification (PTM) risk-eliminated variants purified during small-scale production.
FIGS. 6A, 6B, 6C, 6D, and 6E show SEC-HPLC chromatograms of the parent antibody (11B6) and humanized and PTM risk-eliminated variants purified during small-scale production.
FIG. 7 shows SDS-PAGE gels of the parent antibody (2B4) and lead antibodies (z2-p7-uIgG1K and z8-p7-uIgG1K) purified under non-reducing and reducing conditions during small-scale production (R: reducing).
FIG. 8 shows SEC-HPLC chromatograms of the parent antibody (2B4) and lead antibodies (z2-p7-uIgG1K and z8-p7-uIgG1K) purified during small-scale production.
FIG. 9 shows SDS-PAGE gels of the parent antibody (11B6) and lead antibodies (z10-p3, z12-p3, and z16-p3) purified under non-reducing and reducing conditions during small-scale production (R: reducing).
FIG. 10 shows SEC-HPLC chromatograms of the parent antibody (11B6) and lead antibodies (z10-p3, z12-p3, and z16-p3) purified under small-scale production.
FIG. 11 shows SDS-PAGE gels of the parent antibody (11B6) and lead antibodies (z10-p3, z12-p3, and z16-p3) purified under non-reducing and reducing conditions during scale-up production (R: reducing).
FIG. 12 shows SEC-HPLC chromatograms of the parent antibody (11B6) and lead antibodies (z10-p3, z12-p3, and z16-p3) purified during small-scale production.
FIGS. 13A, 13B and 13C show surface plasmon resonance (SPR) sensorgrams for the full curves of the parent antibody (2B4) and the PTM risk-eliminated variants against human MYCT1 (hMYCT1).
FIGS. 14A, 14B, 14C, 14D, 14E, and 14F show SPR sensorgrams for the full curves of the parent antibody (2B4) and the PTM risk-eliminated variants against hMYCT1.
FIG. 15 shows SPR sensorgrams for the full curves of the parent antibody (2B4) and the humanized and PTM risk-eliminated combination lead antibodies (z2-p7 and z8-p7) against hMYCT1.
FIG. 16 shows SPR sensorgrams for the full curves of the parent antibody (11B6) and the PTM risk-eliminated variants against hMYCT1.
FIG. 17 shows SPR sensorgrams for the full curves of the parent antibody (11B6) and lead antibodies (z10-p3, z12-p3, and z16-p3) against hMYCT1.
FIG. 18 shows differential scanning fluorimetry (DSF) profiles of the parent antibody (2B4) and lead antibodies.
FIGS. 19A and 19B show DSF profiles of the parent antibody (11B6) and lead antibodies.
FIG. 20 shows the results of an antibody toxicity evaluation according to one embodiment of the present invention.
FIGS. 21A and 21B show the results of a tube formation assay for an antibody according to one embodiment of the present invention.
FIG. 22 is a schematic diagram illustrating an antitumor activity experiment in a colon cancer cell line model of an antibody according to one embodiment of the present invention.
FIGS. 23A and 23B show the results illustrating the cancer growth inhibitory effect of an antibody according to one embodiment of the present invention in a colon cancer cell line model (Chimeric ATN001 is an 11B6 antibody, and hzATN001 is an 11B6-z16-p3 antibody).

[Modes of the Invention]

The antibody or antigen-binding fragment according to the present invention can overcome the limitations of existing immuno-oncology agents by specifically binding to MYCT1 and ultimately reprogramming the tumor microenvironment (TME) through i) inhibition of angiogenesis, ii) normalization of blood vessels, iii) increase in immune cell activity within the TME, and iv) increase in the infiltration of immune cells into the TME.

The MYCT1 (hMYCT1) has the following sequence (SEQ ID NO: 1):

MRTQVYEGLCKNYFSLAVLQRDRIKLLFFDILVFLSVFLLFLLFLVD

I**MANNTTSLGSPWPENFWED**LIMSFTVSMAIGLVLGGFIWAVFICLSRRR

RASAPISQWSSSRRSRSSYTHGLNRTGFYRHSGCERRSNLSLASLTFQRQA

SLEQANSFPRKSSFRASTFHPFLQCPPLPVETESQLVTLPSSNISPTISTSHSL

SRPDYWSSNSLRVGLSTPPPPAYESIIKAFPDS.

The SEQ ID NO: 1 is an example of an MYCT1 sequence, and all known MYCT1 sequences may be included in the present invention. The human antibody that specifically binds to MYCT1 provided in the present invention may specifically bind to the amino acid sequence at positions 49 to 67 of the above sequence (MANNTTSLGSPWPENFWED, SEQ ID NO: 2).

## Antibody or antigen-binding fragment

As used herein, the term "antibody" refers to a protein molecule that serves as a ligand that specifically recognizes an antigen, including an immunoglobulin molecule that immunologically has reactivity with a specific antigen, and includes a polyclonal antibody, a monoclonal antibody, a whole antibody, and an antibody fragment. In addition, the antibody in the present invention includes a chimeric antibody, a bivalent antibody, a bispecific molecule, a minibody, a domain antibody, a bispecific or multispecific antibody, an immune cell engaging bispecific or multispecific antibody, an antibody mimetic, a unibody, a diabody, a triabody, or a tetrabody. The term further includes a single-chain antibody having a binding function to neonatal Fc receptor (FcRn), a scaffold-based antibody mimetics (ScAb), a derivative of an antibody constant region, and a protein scaffold-based artificial antibody. A whole antibody has a structure having two full-length light chains (LC) and two full-length heavy chains (HC), each light chain being linked to a heavy chain by a disulfide bond. The whole antibody includes IgA, IgD, IgE, IgM, and IgG, and IgG includes IgG1, IgG2, IgG3, and IgG4 as subtypes. In one embodiment, the antibody provided by the present invention may be an IgG antibody.

In the present invention, "humanized antibody" refers to an antibody that exhibits reduced immunogenicity or non-immunogenicity in humans. For example, the humanized antibody may be produced by combining complementarity determining regions (CDRs) derived from a non-human organism (non-human species) with constant regions derived from human antibodies or framework regions (FRs) among variable regions derived from human antibodies. The humanized antibody may be produced by grafting the CDRs of a non-human species antibody between the FR sequences of a human antibody through a CDR-grafting method.

The term "monoclonal antibody" in the present invention refers to an antibody molecule of a single molecule composition obtained from a substantially identical antibody population, and such a monoclonal antibody exhibits a single binding specificity and affinity for a specific epitope. Typically, immunoglobulins have heavy and light chains, each of which contains a constant region and a variable region (also known as a domain). The variable regions of the light and heavy chains contain three variable regions, which are referred to as complementarity-determining regions (CDRs), and four structural regions.

The term "complementarity determining region" or "CDR region" or "CDR" as used herein refers to a highly variable region of sequence within an antibody variable region, which forms structurally defined loops ("hypervariable loops") and/or includes antigen contact residues ("antigen contact sites"). CDRs are primarily responsible for binding to antigenic epitopes. The CDRs are sandwiched between relatively conserved regions called framework regions (FRs). The heavy chain variable region (VH) and light chain variable region (VL) each consist of three CDRs and four FRs, arranged from amino-terminus to carboxy-terminus in the following order: FR1, CDR1, FR2, CDR2, FR3, CDR3, and FR4. The VH and VL contain binding domains that interact with antigens. The CDRs of the VH may be referred to as HCDR1, HCDR2, and HCDR3, the CDRs of the VL may be referred to as LCDR1, LCDR2, and LCDR3, the FRs of the VH may be referred to as HFR1, HFR2, HFR3, and HFR4, and the FRs of the VL may be referred to as LFR1, LFR2, LFR3, and LFR4. In this case, the regions are sequentially numbered from the N-terminus. The boundaries of the precise amino acid sequence of each CDR in given VL and VH amino acid sequences may be determined by using one of known CDR assignment systems including, for example, Chothia (Chothia et al. (1989) Nature 342: 877-883; Al-Lazikani et al., "Standard conformations for the canonical structures of immunoglobulins," Journal of Molecular Biology, 273, 927-948 (1997)) based on the three-dimensional structure of the antibody and the arrangement of the CDR loops, Kabat (Kabat et al., Sequences of Proteins of Immunological Interest, 4th ed., U.S. Department of Health and Human Services, National Institutes of Health (1987)) based on antibody sequence variability, AbM (University of Bath), Contact (University College London), International ImMunoGeneTics database (IMGT) (imgt.cines.fr/ on the World Wide Web), and North CDR definition based on affinity propagation clustering using multiple crystal structures, or a combination thereof.

The antibody of the present invention may include a constant region, and the constant region may be a constant region derived from IgG, IgA, IgD, IgE, IgM, or a combination thereof or a hybrid thereof, but is not limited thereto. Here, the term "combination" means that a polypeptide encoding a single-chain immunoglobulin constant region of the same origin forms a bond with a single-chain polypeptide of a different origin when forming a dimer or multimer. For example, a dimer or multimer may be formed from two or more constant regions selected from the group consisting of constant regions of IgG, IgA, IgD, IgE, and IgM. In addition, the term "hybrid" means that sequences corresponding to immunoglobulin heavy chain constant regions of two or more different origins are present within a single-chain immunoglobulin heavy chain constant region, for example, a hybrid of domains consisting of one to four domains selected from the group consisting of CH1, CH2, CH3 and CH4 of IgG, IgA, IgD, IgE, and IgM is possible.

The term "whole antibody" as used herein refers to a structure having two full-length light chains and two full-length heavy chains, each light chain being linked to a heavy chain by a disulfide bond, and includes IgA, IgD, IgE, IgM, and IgG. The IgG includes IgG1, IgG2, IgG3, and IgG4 as subtypes.

The terms "fragment," "binding fragment of polypeptide," and "antibody fragment" are used interchangeably herein to refer to any fragment of an antibody of the present invention that retains the antigen-binding activity of the antibody. Exemplary antibody fragments include Fd, Fab, Fab', F(ab')2, dsFv, scFv, or single domain antibodies (sdAb), but are not limited thereto. "Fd" refers to a heavy chain portion included in the Fab fragment. As used herein, the term "Fab" typically refers to a fragment including a VH and a VL, and further including a light chain constant domain and a heavy chain first constant domain (CH1). The term "Fab'" typically refers to a fragment that differs from a Fab by the addition of a few residues (including one or more cysteines from the antibody hinge region) to the carboxy terminus of the heavy chain CH1 domain. The term "F(ab')2" typically refers to a dimeric antibody fragment of Fab' including two Fab fragments linked by a disulfide bridge at the hinge region. The term "Fv" typically refers to the smallest antibody fragment including a complete antigen recognition and binding site. In certain cases, the fragment may be a dimer in which the VH and the VL are closely non-covalently bonded. The term "dsFv" typically refers to an Fv fragment in which the disulfide bond is stabilized, wherein the linkage between a single VL and a single VH is a disulfide bond. The term "dAb fragment" typically refers to an antibody fragment consisting of a VH domain. In the present invention, the term "scFv" typically refers to a monovalent molecule formed by covalent pairing of the VH and the VL of an antibody via a flexible peptide linker. These scFv molecules may have the general structure $NH_2$-VL-linker-VH-COOH or $NH_2$-VH-linker-VL-COOH. The term "single-domain antibody" refers to an antibody fragment, also known as a nanobody (e.g., sdAb, sdFv, nanobody), consisting of a single monomeric variable antibody domain.

The terms "humanized anti-MYCT1 antibody," "anti-MYCT1 antibody," "anti-MYCT1," "MYCT1 antibody," or "antibody that binds to MYCT1," as used herein, refer to an antibody capable of binding to MYCT1 with sufficient affinity such that the antibody can be used as a therapeutic agent targeting MYCT1. In one embodiment of the present invention, the MYCT1 antibody binds to MYCT1 with high affinity *in vitro* or *in vivo*. Here, the binding may be determined by radioimmunoassay (RIA), biolayer interferometry (BLI), meso scale discovery (MSD) analysis, surface plasmon resonance (SPR), or flow cytometry, but is not limited thereto.

The humanized anti-MYCT1 antibody or antigen-binding fragment of the present invention includes: at least one VH selected from the group consisting of SEQ ID NOs: 41 and 43 to 60; and at least one VL selected from the group consisting of SEQ ID NOs: 42 and 61 to 66; or
at least one VH selected from the group consisting of SEQ ID NOs: 92 and 94 to 101; and at least one VL selected from the group consisting of SEQ ID NOs: 93 and 102 to 115.

In one embodiment, the VH may include CDR1, CDR2, and CDR3 having amino acid sequences set forth in SEQ ID NOs: 3, 4, and 5, respectively, and the VL may include CDR1, CDR2, and CDR3 having amino acid sequences set forth in SEQ ID NOs: 6, 7, and 8, respectively.

In another embodiment, the VH may include CDR1, CDR2, and CDR3 having amino acid sequences set forth in SEQ ID NOs: 9, 10, and 11, respectively, and the VL may include CDR1, CDR2, and CDR3 having amino acid sequences set forth in SEQ ID NOs: 12, 13, and 14, respectively.

In still another embodiment, the antibody or antigen-binding fragment thereof may

include a VH sequence of SEQ ID NO: 43 and a VL sequence of SEQ ID NO: 42;
include a VH sequence of SEQ ID NO: 44 and a VL sequence of SEQ ID NO: 42;
include a VH sequence of SEQ ID NO: 45 and a VL sequence of SEQ ID NO: 42;

include a VH sequence of SEQ ID NO: 41 and a VL sequence of SEQ ID NO: 61;
include a VH sequence of SEQ ID NO: 46 and a VL sequence of SEQ ID NO: 62;
include a VH sequence of SEQ ID NO: 41 and a VL sequence of SEQ ID NO: 63;
include a VH sequence of SEQ ID NO: 44 and a VL sequence of SEQ ID NO: 63;
include a VH sequence of SEQ ID NO: 47 and a VL sequence of SEQ ID NO: 64;
include a VH sequence of SEQ ID NO: 48 and a VL sequence of SEQ ID NO: 64;
include a VH sequence of SEQ ID NO: 48 and a VL sequence of SEQ ID NO: 65;
include a VH sequence of SEQ ID NO: 49 and a VL sequence of SEQ ID NO: 65;
include a VH sequence of SEQ ID NO: 50 and a VL sequence of SEQ ID NO: 65;
include a VH sequence of SEQ ID NO: 51 and a VL sequence of SEQ ID NO: 65;
include a VH sequence of SEQ ID NO: 52 and a VL sequence of SEQ ID NO: 65;
include a VH sequence of SEQ ID NO: 53 and a VL sequence of SEQ ID NO: 65;
include a VH sequence of SEQ ID NO: 54 and a VL sequence of SEQ ID NO: 65;
include a VH sequence of SEQ ID NO: 55 and a VL sequence of SEQ ID NO: 64;
include a VH sequence of SEQ ID NO: 55 and a VL sequence of SEQ ID NO: 65;
include a VH sequence of SEQ ID NO: 56 and a VL sequence of SEQ ID NO: 65;
include a VH sequence of SEQ ID NO: 57 and a VL sequence of SEQ ID NO: 65;
include a VH sequence of SEQ ID NO: 58 and a VL sequence of SEQ ID NO: 65;
include a VH sequence of SEQ ID NO: 41 and a VL sequence of SEQ ID NO: 65;
include a VH sequence of SEQ ID NO: 58 and a VL sequence of SEQ ID NO: 42;
include a VH sequence of SEQ ID NO: 59 and a VL sequence of SEQ ID NO: 66; or
include a VH sequence of SEQ ID NO: 60 and a VL sequence of SEQ ID NO: 66.

In yet another embodiment, the antibody or antigen-binding fragment thereof may

include a VH sequence of SEQ ID NO: 92 and a VL sequence of SEQ ID NO: 102;
include a VH sequence of SEQ ID NO: 92 and a VL sequence of SEQ ID NO: 103;
include a VH sequence of SEQ ID NO: 92 and a VL sequence of SEQ ID NO: 104;
include a VH sequence of SEQ ID NO: 94 and a VL sequence of SEQ ID NO: 105;
include a VH sequence of SEQ ID NO: 95 and a VL sequence of SEQ ID NO: 105;
include a VH sequence of SEQ ID NO: 96 and a VL sequence of SEQ ID NO: 105;
include a VH sequence of SEQ ID NO: 97 and a VL sequence of SEQ ID NO: 105;
include a VH sequence of SEQ ID NO: 98 and a VL sequence of SEQ ID NO: 105;
include a VH sequence of SEQ ID NO: 99 and a VL sequence of SEQ ID NO: 106;
include a VH sequence of SEQ ID NO: 99 and a VL sequence of SEQ ID NO: 107;
include a VH sequence of SEQ ID NO: 99 and a VL sequence of SEQ ID NO: 108;
include a VH sequence of SEQ ID NO: 99 and a VL sequence of SEQ ID NO: 109;
include a VH sequence of SEQ ID NO: 99 and a VL sequence of SEQ ID NO: 110;
include a VH sequence of SEQ ID NO: 95 and a VL sequence of SEQ ID NO: 110;
include a VH sequence of SEQ ID NO: 100 and a VL sequence of SEQ ID NO: 110;
include a VH sequence of SEQ ID NO: 100 and a VL sequence of SEQ ID NO: 111;
include a VH sequence of SEQ ID NO: 101 and a VL sequence of SEQ ID NO: 111;
include a VH sequence of SEQ ID NO: 92 and a VL sequence of SEQ ID NO: 111;
include a VH sequence of SEQ ID NO: 101 and a VL sequence of SEQ ID NO: 112;
include a VH sequence of SEQ ID NO: 95 and a VL sequence of SEQ ID NO: 113;
include a VH sequence of SEQ ID NO: 100 and a VL sequence of SEQ ID NO: 114; or
include a VH sequence of SEQ ID NO: 95 and a VL sequence of SEQ ID NO: 115.

In one embodiment, the antibody provided in the present invention may be a monoclonal antibody.
In one embodiment, the antigen-binding fragment provided in the present invention may be an scFv.
The above-described amino acid sequences may include variants having different sequences due to deletion, insertion, or substitution of amino acid residues, or a combination thereof, as long as the structure, function, activity, and the like of the polypeptide including the amino acid sequences are not affected. In addition, the amino acid sequences may include amino acids that have undergone conventional modifications known in the art, and the amino acid modifications may be, for example, phosphorylation, sulfation, acrylation, glycosylation, methylation, farnesylation, and the like.
The antibody or antigen-binding fragment thereof of the present invention not only includes the above-described amino acid sequences, but also includes amino acid sequences substantially identical thereto or variants thereof. The term "substantially identical" means an amino acid sequence having at least 90%, at least 91%, at least 92%, at least 93%, at least 94%, at least 95%, at least 96%, at least 97%, at least 98%, at least 99%, or at least 99.5% homology to the above-described amino acid sequence, but is not limited thereto.

The antibody or antigen-binding fragment of the present invention may further include a linker between the VH and the VL. The linker may be a peptide linker and may have a length of about 10 to 25 amino acids. For example, the linker may include hydrophilic amino acids such as glycine and/or serine, but is not limited thereto.

Specifically, in the present invention, the linker may include, for example, $(GS)_n$, $(GGS)_n$, $(GSGGS)_n$, or $(G_nS)_m$ (n and m are each 1 to 10), and preferably may include GGGGS.

The antibody or antigen-binding fragment of the present invention may include not only the sequence of the anti-MYCT1 antibody or antigen-binding fragment of the present invention described herein, but also biological equivalents thereof, as long as it can specifically recognize MYCT1. For example, additional changes may be made to the amino acid sequence of the antibody to further improve the binding affinity and/or other biological properties of the antibody. Such modifications include, for example, deletion, insertion, and/or substitution of amino acid sequence residues of the antibody or antigen-binding fragment. Such amino acid mutations are made based on the relative similarity of amino acid side-chain substituents, for example, hydrophobicity, hydrophilicity, charge, size, and the like. Analysis of the size, shape, and type of amino acid side-chain substituents reveals that arginine, lysine, and histidine are all positively charged residues; alanine, glycine, and serine have similar sizes; and phenylalanine, tryptophan, and tyrosine have similar shapes. Therefore, based on these considerations, arginine, lysine, and histidine; alanine, glycine, and serine; and phenylalanine, tryptophan, and tyrosine may be considered biologically functional equivalents.

Considering the mutations having the above-described biological equivalent activity, the antibody or antigen-binding fragment of the present invention or the nucleic acid molecule encoding the same is interpreted to also include a sequence showing substantial identity with the sequence described in the sequence number. "Substantial identity" means that a sequence shows at least 90% homology, most preferably at least 95% homology, 96% or more, 97% or more, 98% or more, or 99% or more homology when the sequence of the present invention is aligned with any other sequence to the greatest extent possible and the aligned sequence is analyzed using an algorithm commonly used in the art. Alignment methods for sequence comparison are known in the art. NCBI Basic Local Alignment Search Tool (BLAST) is accessible from NBCI, or the like, and may be used in conjunction with sequence analysis programs such as blastp, blasm, blastx, tblastn, and tblastx on the Internet. BLAST is available at www.ncbi.nlm.nih.gov/BLAST/. Instructions for comparing sequence homology using this program may be found at www.ncbi.nlm.nih.gov/BLAST/blast help.html.

Based on this, the antibody or antigen-binding fragment of the present invention may have 90%, 91%, 92%, 93%, 94%, 95%, 96%, 97%, 98%, 99%, or more homology to the specified sequences or the entirety of the sequences described herein. Such homology may be determined by sequence comparison and/or alignment using methods known in the art. For example, the percentage sequence homology of the nucleic acids or proteins of the present invention may be determined using sequence comparison algorithms (e.g., BLAST or BLAST 2.0), manual alignment, or visual inspection.

The binding affinity of the anti-MYCT1 antibody or antigen-binding fragment provided in the present invention to MYCT1 is in the range of $10^{-5}$ M to $10^{-12}$ M. For example, the binding affinity of the anti-MYCT1 antibody or antigen-binding fragment to MYCT1 may be $10^{-6}$ M to $10^{-12}$ M, $10^{-7}$ M to $10^{-12}$ M, $10^{-8}$ M to $10^{-12}$ M, $10^{-9}$ M to $10^{-12}$ M, $10^{-5}$ M to $10^{-11}$ M, $10^{-6}$ M to $10^{-11}$ M, $10^{-7}$ M to $10^{-11}$ M, $10^{-8}$ M to $10^{-11}$ M, $10^{-9}$ M to $10^{-11}$ M, $10^{-10}$ M to $10^{-11}$ M, $10^{-5}$ M to $10^{-10}$ M, $10^{-6}$ M to $10^{-10}$ M, $10^{-7}$ M to $10^{-10}$ M, $10^{-8}$ M to $10^{-10}$ M, $10^{-9}$ M to $10^{-10}$ M, $10^{-5}$ M to $10^{-9}$ M, $10^{-6}$ M to $10^{-9}$ M, $10^{-7}$ M to $10^{-9}$ M, $10^{-8}$ M to $10^{-9}$ M, $10^{-5}$ M to $10^{-8}$ M, $10^{-6}$ M to $10^{-8}$ M, $10^{-7}$ M to $10^{-8}$ M, $10^{-5}$ M to $10^{-7}$ M, $10^{-6}$ M to $10^{-7}$ M, or $10^{-5}$ M to $10^{-6}$ M, but is not limited thereto.

According to yet another embodiment of the present invention, the present invention relates to a nucleic acid molecule encoding an antibody or antigen-binding fragment provided in the present invention.

As used herein, the term "nucleic acid" encompasses DNA (gDNA and cDNA) and RNA molecules. Nucleotides, the basic structural units of nucleic acids, include not only natural nucleotides but also analogues with modified sugar or base moieties. The sequences of nucleic acids encoding the VH and VL of the present invention may be modified. The modifications include addition, deletion, non-conservative substitution, or conservative substitution of nucleotides.

The DNA encoding the antibody or antigen-binding fragment of the present invention can be easily isolated or synthesized using conventional molecular biological techniques (e.g., by using an oligonucleotide probe capable of specifically binding to the DNA encoding the antibody and the heavy and light chains). The nucleic acid is isolated and inserted into a replicable vector for further cloning (DNA amplification) or further expression.

According to another embodiment, the present invention relates to a recombinant expression vector including the nucleic acid molecule provided in the present invention.

The term "vector" in the present invention refers to a recombinant vector capable of expressing a target protein upon transfection into a suitable host cell, which is a genetic construct including essential regulatory elements operably linked to enable expression of a gene insert. The term "operably linked" herein means that a nucleic acid expression regulatory sequence and a nucleic acid sequence encoding a target protein are functionally linked to perform a general function. Operable linkage with a recombinant vector may be achieved using genetic recombination techniques well known in the art, and site-specific DNA cleavage and ligation may be easily achieved using enzymes generally known in the art.

In the present invention, various types of vectors, such as nanoparticles, plasmids, viruses, and cosmids, may be used as recombinant expression vectors for inserting the foreign gene. The type of recombinant vector is not particularly limited, as long as it can express a desired gene and produce a desired protein in various host cells, both prokaryotic and eukaryotic. Specifically, vectors capable of producing large quantities of the foreign protein in a form similar to that of the natural form, while possessing a promoter exhibiting strong activity as well as strong expression capacity, can be used.

Various gene delivery vehicles are well known in the art, including both viral and non-viral (e.g., naked DNA, plasmid) vectors. Viral vectors suitable for gene delivery are well known to those skilled in the art. Non-limiting examples of the viral vectors may include retroviral vectors (derived from Moloney murine leukemia virus vector (MoMLV), murine stem cell virus (MSCV), spleen focus-forming virus (SFFV), myeloproliferative sarcoma virus (MPSV), Sin Nombre virus (SNV), etc.), lentiviral vectors (e.g., derived from human immunodeficiency virus (HIV)-1, HIV-2, simian immunodeficiency virus (SIV), bovine immunodeficiency virus (BIV), feline immunodeficiency virus (FIV), etc.), adenovirus (Ad) vectors including replication competent, replication deficient, and emasculated forms thereof, adeno-associated virus (AAV) vectors, simian virus 40 (SV-40) vectors, bovine papilloma virus vectors, Epstein-Barr virus vectors, herpes virus vectors, varicella virus vectors, Harvey murine sarcoma virus vectors, mouse mammary tumor virus vectors, Rous sarcoma virus (RSV) vectors, parvovirus vectors, poliovirus vectors, vesicular stomatitis virus vectors, Maraba virus vectors and group B adenovirus enadenotucirev vectors.

Non-viral vectors for gene transfer include naked DNA, plasmids, transposons, mRNA, and the like. Non-limiting examples include pKK plasmid (Clonetech), pUC plasmid, pET plasmid (Novagen, Inc., Madison, Wis.), pRSET or pREP plasmids (Invitrogen, San Diego, Calif.), and pMAL plasmid (New England Biolabs, Beverly, Mass.).

The vector of the present invention may be introduced into many suitable host cells using methods disclosed or cited herein or otherwise known to those skilled in the art.

Suitable expression vectors of the present invention may include, in addition to expression regulation elements such as a promoter, start codon, stop codon, polyadenylation signal, or enhancer, a nucleotide sequence encoding a signal peptide for membrane targeting or secretion. The start codon and stop codon are generally considered to be part of a nucleotide sequence encoding an immunogenic target protein, and must be functional in a subject when the genetic construct is administered and must be in frame with the coding sequence.

The term "promoter" as used herein refers to any sequence that regulates the expression of a coding sequence, such as a gene. A promoter may be, for example, constitutive, inducible, repressible, or tissue-specific. A promoter is a control sequence, which is a region of a polynucleotide sequence where the initiation and rate of transcription are controlled. Non-limiting examples of the promoter in the present invention include RSV long terminal repeat (LTR) promoter (optionally with RSV enhancer), cytomegalovirus (CMV) promoter, SV40 promoter, dihydrofolate reductase promoter, $\beta$-actin promoter, phosphoglycerate kinase (PGK) promoter, U6 promoter, EF1alpha short (EFS) promoter, human polypeptide chain elongation factor (EF1a) promoter, P5 promoter, ubiquitin C (Ubc) promoter, CMV enhancer and chicken $\beta$-actin promoter (CAG) promoter, tetracycline response element (TRE) promoter, upstream activating sequence (UAS) promoter, action 5C (Ac5) promoter, polyhedrin promoter, calcium/calmodulin-dependent protein kinase IIa (CaMKIIa) promoter, Gall promoter, translation elongation factor 1 (TEF1) promoter, glutamine synthetase (GDS) promoter, alcohol dehydrogenase 1 (ADH1) promoter, cauliflower mosaic virus 35S (CaMV35S) promoter, ubiquitin (Ubi) promoters, such as ubiquitin C (UbiC), H1 promoter, U6 promoter, alpha-1-antitrypsin promoter, SFFV promoter, and the like.

In addition, in the present invention, the promoter may be coupled to an enhancer to increase transcription efficiency. Non-limiting examples of the enhancer include an RSV enhancer, a CMV enhancer, or an $\alpha$-fetoprotein MERII enhancer, but are not limited thereto. The vector of the present invention may also be fused with other sequences to facilitate purification of antibodies expressed therefrom. Examples of the fused sequences include glutathione S-transferase (Pharmacia, USA), maltose binding protein (New England Biolabs, USA), FLAG (IBI Scientific, USA), and hexahistidine (6x Hi; Quiagen Inc., USA), but are not limited thereto.

The vector of the present invention includes an antibiotic resistance gene commonly used as a selectable marker in the technical field, and may include resistance genes for ampicillin, gentamicin, carbenicillin, chloramphenicol, streptomycin, kanamycin, geneticin, neomycin, and tetracycline, but is not limited thereto.

Yet another embodiment of the present invention relates to a host cell transfected with an expression vector provided in the present invention.

The term "host cell" as used herein includes an individual cell or cell culture that may be or has been a recipient of vector(s) for incorporation of a polypeptide insert. A host cell includes progeny of a single host cell, and the progeny may not necessarily be completely identical (morphologically or in genomic DNA complement) to the original parent cell due to natural, accidental, or intentional mutation. A host cell includes cells transfected *in vivo* with the polypeptide(s) of the present invention.

In the present invention, the host cell may include cells of mammalian, plant, insect, fungal, or cellular origin, such as bacterial cells such as *Escherichia coli, Streptomyces,* and *Salmonella typhimurium;* yeast cells such as *Pichia*

*pastoris;* insect cells such as *Drosophila* or *Spodoptera* Sf9 cells; animal cells such as Chinese hamster ovary (CHO) cells, SP2/0 (mouse myeloma), human lymphoblastoid, COS, NSO (mouse myeloma), 293T, Bowes melanoma cells, HT-1080, baby hamster kidney (BHK) cells, human embryonic kidney (HEK) cells or primary embryonic retinal C6 (PER.C6) (human retinal cells); or plant cells, but are not limited thereto, and any cell that may be used as a host cell line known to those of ordinary skill in the art may be used.

In the present invention, the transfection method is any method for injecting a desired vector into the host cell, any known method capable of injecting a vector into a host cell may be included, and for example, a method using CaCl$_2$, electroporation, microinjection, calcium phosphate precipitation, electroporation, liposome-mediated transfection, diethylaminoethyl (DEAE)-dextran treatment, gene bombardment, and virus-based transfection may be used, but the method is not limited thereto.

According to yet another embodiment, the present invention relates to a method of producing an antibody or antigen-binding fragment that specifically binds to MYCT1, including: a step of culturing the host cell to produce an antibody or antigen-binding fragment; and isolating and purifying the produced antibody or antigen-binding fragment.

The host cell of the present invention may be cultured in various media. Any commercially available media may be used as a culture medium without limitation. Any other necessary supplements known to those skilled in the art may be included at appropriate concentrations. Culture conditions, such as temperature, pH, and the like, are already used with host cells selected for expression and will be apparent to those skilled in the art.

In the present invention, the antibody or antigen-binding fragment thereof may be recovered by removing impurities, for example, by centrifugation or ultrafiltration, and the resulting product may be purified, for example, by affinity chromatography. Additional purification techniques, such as anion or cation exchange chromatography, hydrophobic interaction chromatography, and hydroxylapatite chromatography, may also be used.

According to yet another embodiment of the present invention, a bispecific or multispecific antibody including an antibody or antigen-binding fragment provided by the present invention is provided.

The term "bispecific antibody" as used herein refers to an antibody that has binding or antagonistic activity toward one or more targets, and includes a form in which antibodies with binding or antagonistic activity toward two different targets are bound, or an antibody in which an antibody with binding activity toward one target is bound to a substance with antagonistic activity toward another target.

The term "multispecific antibody" as used herein refers to an antibody that has binding specificities for at least three different antigens. Multispecific antibodies may include trispecific or higher antibodies, such as trispecific antibodies, tetraspecific antibodies, or antibodies targeting more than four targets.

In the present invention, antibodies belonging to the bispecific or multispecific antibody category may be categorized into scFv-based antibodies, Fab-based antibodies, and IgG-based antibodies. Bispecific or multispecific antibodies can simultaneously suppress or amplify two or more signals, making them more effective than antibodies suppressing or amplifying single signals. Compared to treating each signal with a separate signal suppressor, lower dosing is possible, and two or more signals can be suppressed/amplified within the same time and space.

Methods for producing bispecific or multispecific antibodies are well known. Traditionally, recombinant production of bispecific antibodies is based on the co-expression of two or more immunoglobulin heavy chain/light chain pairs under the condition that the two or more heavy chains possess different specificities.

In the present invention, in the case of a bispecific or multispecific antibody based on scFv, the VL and VH of different scFvs may be combined to produce a hybrid scFv in a heterodimeric form to produce a diabody, different scFvs may be linked to produce a tandem ScFv, CH1 and CL of Fab may be expressed at the ends of each scFv to produce a heterodimeric miniantibody, and some amino acids of the CH3 domain, which is a homodimeric domain of Fc, may be substituted to change the structure into a heterodimeric structure in the form of a "knob into hole," and these changed CH3 domains may be expressed at the ends of different scFvs to produce a minibody in the form of a heterodimeric scFv.

In the present invention, in the case of a bispecific or multispecific antibody based on Fab, individual Fabs for specific antigens may be combined using a disulfide bond or a mediator to produce a heterodimeric Fab form, and by expressing scFvs for different antigens at the terminals of the heavy or light chains of a specific Fab, an antibody having an antigen-binding valency of two may be produced, or by providing a hinge region between the Fab and scFv, an antibody having an antigen-binding valency of four may be produced in a homodimeric form. In addition, a dual-target bibody having an antigen-binding valency of three may be obtained by fusing scFvs for different antigens to the light and heavy chain terminals of the Fab, and a tri-target bibody having an antigen-binding valency of three may be obtained by fusing different scFvs to each of the light and heavy chain terminals of the Fab or by chemically conjugating three different Fabs.

In the present invention, in the case of IgG-based bispecific or multispecific antibodies, a method of producing bispecific antibodies by producing hybrid hybridomas, also known as quadromas, by crossbreeding mouse and rat hybridomas again developed by Trion Pharma is known. In addition, bispecific antibodies may be produced in the so-called "holes and knob" form, which is produced in a heterodimeric form by modifying some amino acids in the CH3 homodimeric domain of Fc for different heavy chains while sharing the light chain portion. In addition to the heterodimeric form of bispecific antibodies, two different scFvs may be fused and expressed in the constant domains of each of the light and heavy chains of IgG instead of the variable domains, thereby producing (scFv)4-IgG in a homodimeric form. In addition, ImClone reported the production of a bispecific antibody by fusing only a single variable domain for mouse platelet-derived growth factor receptor-α to the light chain amino terminus of IMC-1C11, a chimeric monoclonal antibody against human VEGF receptor 2 (VEGFR-2). In addition, antibodies with multiple antigen-binding valencies for CD20 may be produced through the so-called "dock and lock (DNL)" method using the dimerization and docking domain (DDD) of the R subunit of protein kinase A (PKA) and the anchoring domain of PKA.

In the present invention, a wide variety of recombinant antibody formats, for example, bivalent, trivalent, or tetravalent bispecific or multispecific antibodies, have been developed. For example, the antibodies include bivalent, trivalent, or tetravalent antibodies disclosed in International Patent Application Publication Nos. WO2001/077342, WO2009/080251, WO2009/080252, WO2009/080253, WO2009/080254, WO2010/112193, WO2010/115589, WO2010/136172, WO2010/145792, WO2010/145793, and WO2011/117330. An antibody being bivalent or more, trivalent or more, or tetravalent or more indicates that two or more binding domains, three or more binding domains, or four or more binding domains are present in the antibody molecule, respectively.

The bispecific or multispecific antibody according to the present invention may include the anti-MYCT1 antibody or antigen-binding fragment specifically in the form of a whole IgG antibody or a fragment thereof, for example, a single-chain Fv, a VH domain and/or a VL domain, a Fab or (Fab)2. In addition, the bispecific or multispecific antibody according to the present invention may include the antibody targeting MYCT1 and an antibody binding to a target other than the antibody targeting MYCT1, for example, an antibody targeting one or more selected from the group consisting of PD-1, PD-L1, BTLA, CTLA-4, VISTA, LAG3, TIM3, CD137 (4-1BB), VISTA, CD258 (LIGHT), TIGIT, CD134 (OX40), CD28, CD278 (ICOS), CD27, CD154 (CD40L), CD357 (GITR), CD30, DR3, CD226 (DNAM1), CD96, CD200, CD200R, transferrin receptor, c-Met, EGFR, HER2, KDR, PDGFRa, NRP1, and MARCO, specifically in the form of a whole IgG antibody or a fragment thereof, for example, a single-chain Fv, a VH domain and/or a VL domain, a Fab or (Fab)2.

Through the bispecific or multispecific antibodies according to the present invention, additional binding specificities induced or mediated by targets other than MYCT1 may be obtained.

For example, the bispecific antibody according to the present invention may simultaneously target MYCT1 and one or more selected from the group consisting of PD-1, PD-L1, BTLA, CTLA-4, VISTA, LAG3, TIM3, CD137 (4-1BB), VISTA, CD258 (LIGHT), TIGIT, CD3, CD20, CD134 (OX40), CD28, CD278 (ICOS), CD27, CD154 (CD40L), CD357 (GITR), CD30, DR3, CD226 (DNAM1), CD96, CD200, CD200R, transferrin receptor, c-Met, EGFR, HER2, KDR, PDGFRa, NRP1, MARCO, FcRH5, HER2, LYPD1, LY6G6D, PMEL17, LY6E, NKG2D, CD3, CD16a, CD19, CD33, CD22, CD79A, CD79B, EDAR, GFRA1, MRP4, RET, Steap1, and TenB2.

For example, the multispecific antibody according to the present invention may simultaneously target MYCT1 and two or more selected from the group consisting of PD-1, PD-L1, BTLA, CTLA-4, VISTA, LAG3, TIM3, CD137 (4-1BB), VISTA, CD258 (LIGHT), TIGIT, CD3, CD20, CD134 (OX40), CD28, CD278 (ICOS), CD27, CD154 (CD40L), CD357 (GITR), CD30, DR3, CD226 (DNAM1), CD96, CD200, CD200R, transferrin receptor, c-Met, EGFR, HER2, KDR, PDGFRa, NRP1, MARCO, FcRH5, HER2, LYPD1, LY6G6D, PMEL17, LY6E, NKG2D, CD3, CD16a, CD19, CD33, CD22, CD79A, CD79B, EDAR, GFRA1, MRP4, RET, Steap1, and TenB2.

According to yet another embodiment of the present invention, the present invention relates to an immune cell-engaging bispecific or multispecific antibody including an antigen-binding fragment (scFv) provided by the present invention and one or more scFvs binding to an immune cell-activating antigen.

In the present invention, the immune cell-engaging bispecific or multispecific antibody temporarily induces a cytolytic synapse between a cytotoxic T cell or NK cell and a cancer target cell, thereby releasing a toxic substance.

In the present invention, a T cell-activating antigen may be CD3, TCRα, TCRβ, TCRγ, TCRξ, HVEM, LIGHT, CD40, 4-1BB, OX40, DR3, GITR, CD30, TIM1, SLAM, CD2, or CD226, but is not limited thereto.

In the present invention, an NK cell activating antigen may be NKp30, NKp40, NKp44, NKp46, NKG2D, DNAM1, DAP10, CD16 (e.g., CD16a, CD16b), CRTAM, CD27, PSGL1, CD96, CD100 (SEMA4D), NKp80, CD244 (SLAMF4 or 2B4), SLAMF6, SLAMF7, KIR2DS2, KIR2DS4, KIR3DS1, KIR2DS3, KIR2DS5, KIR2DS1, CD94, NKG2C, NKG2E, or CD160, but is not limited thereto.

Other immune cell engagers are specifically described in U.S. Patent Application Publication No. US 2017/0368169, which is incorporated herein by reference.

Specifically, the immune cell-engaging bispecific or multispecific antibody includes a tandem scFv and may bind to the following antigens and cancer cell surface antigens. The cancer cell surface antigen is MYCT1, which is targeted by the antibody according to the present invention.

In the present invention, the immune cell engaging bispecific or multispecific antibody may include a structure in the form of, for example, VL(MYCT1)-VH(MYCT1)-VH(CD3 or CD16A)-VL(CD3 or CD16A), VH(MYCT1)-VL(MYCT1)-VH(CD3 or CD16A)-VL(CD3 or CD16A), VH(CD3 or CD16A)-VL(CD3 or CD16A)-VH(MYCT1)-VL(MYCT1), or VH(CD3 or CD16A)-VL(CD3 or CD16A)-VL(MYCT1)-VH(MYCT1).

The present invention may further include a linker between the VH and the VL. The linker may be a peptide linker and have a length of about 10 to 25 amino acids. For example, the linker may include hydrophilic amino acids such as glycine and/or serine, but is not limited thereto. Specifically, the linker may include (GS)n, (GGS)n, (GSGGS)n, or (GnS)m (where n and m are each 1 to 10), preferably GGGGS, and preferably GGGGSGGGGSGGGGS, but is not limited thereto.

Examples of the immune cell-engaging bispecific or multispecific antibodies in the present invention include blinatumomab (Amgen Inc.), which binds to CD3 and CD19; solitomab (Amgen Inc.), which binds to CD3 and EpCAM; MEDI 565 (MedImmune, Amgen Inc.), which binds to CD3 and CEA; and BAY2010112 (Bayer AG, Amgen Inc.), which binds to CD3 and PSMA. Exemplary dual affinity retargeting (DART) antibodies include MGD006 (Macrogenics Inc.), which binds to CD3 and CD123; and MGD007 (Macrogenics Inc.), which binds to CD3 and gpA33. Exemplary tandem diabodies (TandAbs) may include AFM11 (Affimed Therapeutics AG), which binds to CD3 and CD19; and AFM13 (Affimed Therapeutics AG), which binds to CD30 and CD16A.

## Immunoconjugates

According to another embodiment of the present invention, the present invention relates to an immunoconjugate in which the antibody or antigen-binding fragment is combined with a cytotoxic agent.

In the present invention, the cytotoxic agent may be one or more selected from the group consisting of a chemotherapeutic agent or chemotherapeutic drug, a growth inhibitor, a toxin (e.g., a protein toxin, an enzymatically active toxin or fragment thereof derived from bacteria, fungi, plants, or animals), and a radioisotope.

In one embodiment, the immunoconjugate provided in the present invention may be an antibody-drug conjugate (ADC) in which the antibody or antigen-binding fragment is conjugated to a drug.

The term "ADC" as used herein refers to a form in which a drug and an antibody are chemically linked without diminishing the biological activity of the antibody and the drug. In the present invention, the ADC has a form in which a drug is bound to an amino acid residue at the N-terminus of the heavy chain and/or light chain of an antibody, specifically, a form in which a drug is bound to an $\alpha$-amine group at the N-terminus of the heavy chain and/or light chain of an antibody.

In the present invention, the antibody or antigen-binding fragment may be linked to a drug via a linker. The linker, serving as a linking moiety between the antibody and the drug, must have a cleavable form under intracellular conditions, that is, must allow the drug to be released from the antibody in the intracellular environment. In addition, reflecting the antibody's long half-life, the linker must be stable when the antibody undergoes systemic circulation, and the linker-drug binding must not affect the stability or pharmacokinetics of the antibody.

In the present invention, the linker may include, for example, a cleavable linker or a non-cleavable linker. Cleavable linkers, like peptide linkers, may be cleaved by intracellular peptidases or proteases, such as lysosomal or endosomal proteases. Non-cleavable linkers, such as thioether linkers, may release the drug after the antibody is non-selectively degraded by intracellular hydrolysis.

In the present invention, the cleavable linker may include a peptide linker. The peptide linker may be at least two amino acids long. For example, the peptide linker may include a Val-Cit, Val-Ala, or Val-Cit dipeptide, or Phe-Leu or Gly-Phe-Leu-Gly. Examples of linkers are specifically disclosed in International Patent Application Publication No. WO2004/010957, which is incorporated herein by reference.

In the present invention, the antibody region of the ADC binds to an antigen on a target cancer cell, thereby forming an ADC-antigen complex, which is then internalized into cancer cells via an endosome-lysosome pathway. In this case, the intracellular release of the cytotoxic drug is controlled by the internal environment of the endosome/lysosome.

In the present invention, the cleavable linker is pH-sensitive and may be sensitive to hydrolysis at a specific pH value. Generally, a linker being pH-sensitive indicates that it may be hydrolyzed under acidic conditions. For example, a pH-sensitive linker may be an acid-labile linker that may be hydrolyzed in lysosomes, such as a hydrazone, a semicarbazone, a thiosemicarbazone, a cis-aconitic amide, an orthoester, an acetal, a ketal, and the like.

In the present invention, the linker may also be cleaved under reducing conditions, and a disulfide linker may be an example thereof. Various disulfide bonds may be formed using N-succinimidyl-S-acetylthioacetate (SATA), N-succinimidyl-3-(2-pyridyldithio)propionate (SPDP), N-succinimidyl-3-(2-pyridyldithio)butyrate (SPDB), and N-succinimidyl-oxycarbonyl-alpha-methyl-alpha-(2-pyridyl-dithio)toluene (SMPT). These disulfide linkers may be cleaved by disulfide exchange with the thiol of intracellular glutathione.

In the present invention, the drug and/or drug-linker may be randomly conjugated via lysine of the antibody, or via cysteine exposed upon reduction of the disulfide bond chain. In some cases, the linker-drug conjugate may be formed via a cysteine present in a genetically engineered tag, for example, a peptide or protein. The genetically engineered tag, for example, a peptide or protein, may include an amino acid motif recognizable by, for example, an isoprenoid transferase. The peptide or protein may have a deletion at the carboxyl terminus of the peptide or protein, or may have a covalent addition of a spacer unit to the carboxyl (C) terminus of the peptide or protein.

In the present invention, the peptide or protein may be covalently linked directly to the amino acid motif, or may be covalently linked to the spacer unit to be linked to the amino acid motif. The above amino acid spacer unit includes 1 to 20 amino acids, among which a glycine unit is preferable.

In the present invention, the linker may include a $\beta$-glucuronide linker that is recognized and hydrolyzed by $\beta$-

glucuronidase, which is abundant in lysosomes or overexpressed in some tumor cells. Unlike peptide linkers, the β-glucuronide linker has high hydrophilicity, and thus has the advantage of increasing the solubility of ADCs when combined with highly hydrophobic drugs. In this regard, the β-glucuronide linker disclosed in International Patent Application Publication No. WO2015/182984, for example, a β-glucuronide linker including a self-immolative group, may be used, and the disclosure is incorporated herein by reference.

In the present invention, the linker may be, for example, a non-cleavable linker, and the drug is released through a single step of antibody hydrolysis within the cell, thereby producing, for example, an amino acid-linker-drug conjugate. This type of linker may be a thioether group or maleimidocaproyl group, and may maintain stability in blood.

In the present invention, the linker-drug conjugate may be formed through randomly linking a linker and a drug through a cysteine that is exposed when the disulfide bond chain of the antibody or antigen-binding fragment is reduced, or by introducing an antibody-terminal binding peptide having the sequence GGGGGGGCVIM.

In the present invention, the drug is a pharmacologically effective agent that may be bound to an antibody, and may be, specifically, a chemotherapeutic agent, a toxin, microRNA (miRNA), small interfering RNA (siRNA), short hairpin RNA (shRNA), or a radioactive isotope. The chemotherapeutic agent may be, for example, a cytotoxic agent or an immunosuppressant. Specifically, the chemotherapeutic agent may include a microtubulin inhibitor, a mitotic inhibitor, a topoisomerase inhibitor, or a chemotherapeutic agent that may function as a DNA intercalator. In addition, the chemotherapeutic agent may include a drug for treating cancer, inflammation, or immune diseases. For example, the chemotherapeutic agent may include an immunomodulatory compound, an anticancer agent, an antiviral agent, an antibacterial agent, an antifungal agent, an anthelmintic agent, or a combination thereof.

In the present invention, the anticancer agent may be, for example, one or more selected from the group consisting of maytansinoids, auristatin (including monomethyl auristatin E (MMAE) and monomethyl auristatin F (MMAF)), aminopterin, actinomycin, bleomycin, talisomycin, camptothecin, N8-acetyl spermidine, 1-(2-chloroethyl)-1,2-dimethyl sulfonyl hydrazide, esperamicin, etoposide, 6-mercaptopurine, dolastatin, trichothecenes, calicheamicin, taxol, taxane, paclitaxel, docetaxel, methotrexate, vincristine, vinblastine, doxorubicin, melphalan, mitomycin A, mitomycin C, chlorambucil, duocarmycin, L-asparaginase, mercaptopurine, thioguanine, hydroxyurea, cytarabine, cyclophosphamide, ifosfamide, nitrosourea, cisplatin, carboplatin, mitomycin, dacarbazine, procarbazine, topotecan, nitrogen mustard, cytoxan, etoposide, 5-fluorouracil, bischloroethyl nitrosourea (CNU), irinotecan, camptothecin, bleomycin, idarubicin, daunorubicin, dactinomycin, plicamycin, mitoxantrone, asparaginase, vinorelbine, chlorambucil, melphalan, carmustine, lomustine, busulfan, treosulfan, decarbazine, etoposide, teniposide, topotecan, 9-aminocamptothecin, crisnatol, mitomycin C, trimetrexate, mycophenolic acid, tiazofurin, ribavirin, 5-ethynyl-1-beta-ribofuranosylimidazole-4-carboxamide (EICAR), hydroxyurea, deferoxamine, floxuridine, doxifluridine, raltitrexed, cytarabine (ara-C), cytosine arabinoside, fludarabine, tamoxifen, raloxifene, megestrol, goserelin, leuprolide acetate, flutamide, bicalutamide, EB1089, CB1093, KH1060, verteporfin, phthalocyanine, photosensitizer Pe4, demethoxy-hypocrellin A, interferon-α, interferon-γ, tumor necrosis factors, gemcitabine, velcade, revamid, thalomid, lovastatin, 1-methyl-4-phenylpyridinium ion, staurosporine, actinomycin D, dactinomycin, bleomycin A2, bleomycin B2, peplomycin, epirubicin, pirarubicin, zorubicin, mitoxantrone, verapamil, thapsigargin, and nucleases and toxins of bacterial, animal or plant origin, but is not limited thereto.

In the present invention, the anti-inflammatory agent may include a steroid preparation that reduces inflammation or swelling by binding to a glucocorticoid receptor, a non-steroidal anti-inflammatory drug (NSAID) that relieves pain by responding to cyclic oxygenase (COX) that synthesizes inflammation-causing prostaglandins, or an immune-selective anti-inflammatory derivative (ImSAID) that alters the activation and transport of inflammatory cells, but is not limited thereto.

In the present invention, the immune disease treatment drug may include azathioprine, chlorambucil, cyclophosphamide, cyclosporine, mycophenolate, azathioprine, or methotrexate, but is not limited thereto.

In the present invention, in some cases, the drug may include one or more nucleophilic groups selected from the group consisting of amine, thiol, hydroxyl, hydrazide, oxime, hydrazine, thiosemicarbazone, hydrazine carboxylate, and arylhydrazide groups that may react to form a covalent bond with an electrophilic group on a linker and a linker reagent, but is not limited thereto.

## Chimeric antigen receptor (CAR)

According to yet another embodiment of the present invention, the present invention relates to a CAR including the antigen-binding fragment.

The term "CAR" as used herein refers to an engineered receptor including an extracellular antigen-binding domain and an intracellular signaling domain. While the most common type of CAR includes an scFv derived from a monoclonal antibody fused to the transmembrane and intracellular domains of a T cell coreceptor, such as the CD3ζ chain, the invention described herein is not limited to these domains.

The term "CAR" as used herein refers to any receptor engineered to express any intracellular signaling molecule and an extracellular antigen-binding domain fused or linked thereto.

In the present invention, the binding domain may include an antigen-binding fragment provided in the present invention, preferably an scFv.

The CAR of the present invention may further include at least one of a hinge region (or spacer) and a signaling domain.

In the present invention, the hinge region, also referred to as a "spacer," is a portion that connects an antigen-binding domain and a transmembrane domain, and has the purpose of extending the antigen-binding domain from a T cell membrane or an NK cell membrane.

In the present invention, the hinge region may be obtained from any suitable sequence from any genus including, for example, human or a part thereof, or may include a hinge region of a human protein including all or part of CD8, D28, 4-1BB, OX40, CD3 zeta (ζ) chain, T cell receptor α or β chain, CD28, CD3ε, CD45, CD4, CD5, CD8, CD9, CD16, CD22, CD33, CD37, CD64, CD80, CD86, CD134, CD137, ICOS, CD154, functional derivatives thereof, or combinations thereof, which are commonly used in the art, but is not limited thereto.

In addition, in the present invention, the hinge region may include one selected from immunoglobulins (e.g., IgG1, IgG2, IgG3, IgG4, and IgD), but is not limited thereto.

In the present invention, the signaling domain is a portion of a CAR that is found or engineered to be found within a T cell. In the present invention, the signaling domain may or may not include a transmembrane domain that serves to anchor the CAR to a plasma membrane of a T cell.

In the present invention, the transmembrane domain and the signaling domain may be derived from the same protein (e.g., CD3ζ), or the transmembrane domain and the signaling domain may be derived from different proteins (e.g., the transmembrane domain of CD28 and the intracellular signaling domain of a CD3ζ molecule, or vice versa).

In the present invention, the transmembrane domain includes a hydrophobic polypeptide that spans the cell membrane. Specifically, the transmembrane domain may span from one surface of the cell membrane (extracellular) to the other surface (intracellular or cytoplasmic).

In the present invention, the transmembrane domain may be in the form of an alpha helix, a beta barrel, or a combination thereof. In addition, the transmembrane domain may include a multicomponent protein having multiple transmembrane segments, each including an alpha helix, a beta sheet, or a combination thereof.

In the present invention, the transmembrane domain may include, for example, a T cell receptor α or β chain, all or part of CD3 zeta (ζ) chain, CD28, CD3ε, CD45, CD4, CD5, CD8, CD9, CD16, CD22, CD33, CD37, CD64, CD80, CD86, CD134, CD137, ICOS, CD154, functional derivatives thereof, or combinations thereof, but is not limited thereto.

In the present invention, the transmembrane domain, for example, may be a polypeptide primarily including hydrophobic residues such as leucine and valine. In one embodiment of the present invention, a triad of phenylalanine, tryptophan, and valine may be found at each terminus of the synthetic transmembrane domain.

In the present invention, at least one costimulatory domain may be further included between the transmembrane domain and the signaling domain. In the present invention, the costimulatory domain, as a region through which costimulatory signals are transmitted, may serve to transmit signals so that CAR-T cells or CAR-NK cells that recognize a specific antigen bound to the antigen-binding domain induce an immune response, help self-proliferation, and increase the residual time in the body.

In the present invention, the costimulatory domain may include a functional signaling domain derived from a polypeptide including 4-1BB (CD137); OX40; CD27; CD28; CD30; CD40; PD-1; CD2; CD7; CD258; natural killer group 2 member C (NKG2C); natural killer group 2 member D (NKG2D); B7-H3; CD83; ICAM-1; a ligand that binds to LFA-1 (CD11a/CD18) or ICOS; an active fragment thereof; a functional derivative thereof; or a combination thereof, but is not limited thereto.

In the present invention, the signaling domain may include a polypeptide that provides activation of an immune cell to stimulate or activate at least a portion of an immune cell signaling pathway.

In the present invention, the signaling domain may include a functional signaling domain derived from a polypeptide including all or part of CD3 zeta (ζ), common FcR gamma (FcER1G), FcgammaRIIIa, FcRbeta (FcεRIβ), CD3 gamma, CD3 delta, CD3 epsilon, CD79a, CD79b, DNAX-activating protein 10 (DAP10), DNAX-activating protein 12 (DAP12), an active fragment thereof, a functional derivative thereof, or a combination thereof, but is not limited thereto, and such signaling domains are known in the art.

In addition, the CAR of the present invention may further include a signal peptide.

In the present invention, the "signal peptide" includes a peptide sequence, which is a type of peptide of any secreted or transmembrane protein, and may direct the transport of the CAR of the present invention to the cell membrane and cell surface, and provide precise localization of the CAR of the present invention. In particular, the signal peptide in the present invention anchors the CAR of the present invention to the cell membrane, such that the extracellular portion of the CAR is displayed on the cell surface, the transmembrane portion spans the plasma membrane, and the active domain is located in the cytoplasmic portion or within the cell.

In the present invention, any peptide that performs the above-described function may be used as the signal peptide without limitation. For example, the signal peptide may be an N-terminal CD8α signal peptide, but is not limited thereto.

According to yet another embodiment of the present invention, the present invention relates to a nucleic acid molecule encoding a CAR according to the present invention.

The nucleic acid molecule encoding the CAR according to the present invention may be readily prepared from the amino acid sequence of the CAR specified by any conventional method. The base sequence encoding the amino acid sequence may be obtained for each domain from the above-mentioned NCBI RefSeq ID or GenBenk accession number, and the nucleic acid of the present disclosure may be prepared using standard molecular biological and/or chemical procedures. For example, a polynucleotide based on the base sequence may be synthesized, and the nucleic acid molecule of the present disclosure may be prepared by combining DNA fragments obtained from a cDNA library using polymerase chain reaction (PCR).

The nucleic acid molecule of the present invention may be a gene or part of an expression or cloning cassette, but is not limited thereto.

According to yet another embodiment of the present invention, the present invention relates to an expression cassette including a nucleic acid molecule provided by the present invention.

The expression cassette of the present invention may include expression control sequences, such as a promoter, an enhancer, a polyadenylation signal, a transcription terminator, and an internal ribosome entry site (IRES), which regulate the expression of the nucleic acid molecule of the present invention.

In the present invention, the promoter may include the SFFV promoter, the elongation factor $1\alpha$ (EF 1a) promoter, or the CAG (chicken beta-actin promoter with a CMV enhancer) promoter, but is not limited thereto.

The expression cassette of the present invention may further include a gene sequence encoding a surrogate marker useful for monitoring expression, such as a cell surface marker (e.g., a truncated cell surface marker), which may be used to confirm transduction or manipulation of cells expressing the receptor.

Exemplary marker genes in the present invention may include base sequences encoding truncated epidermal growth factor receptor (EGFRt), prostate-specific membrane antigen (PSMA), CD34, nerve growth factor receptor (NGFR), or variant forms thereof, but are not limited thereto.

In addition, in the present invention, the marker gene may include a base sequence encoding a fluorescent protein, for example, green fluorescent protein (GFP), enhanced green fluorescent protein (EGFP), for example, super-fold GFP (sfGFP), red fluorescent protein (RFP), for example, tdTomato, mCherry, mStrawberry, AsRed2, DsRed or DsRed2, cyan fluorescent protein (CFP), blue green fluorescent protein (BFP), enhanced blue fluorescent protein (EBFP), and yellow fluorescent protein (YFP), species variants, monomer variants, and codon-optimized and/or enhanced variants of the fluorescent proteins, but is not limited thereto.

In addition, in the present invention, the marker gene is or includes a base sequence encoding an enzyme, for example, luciferase, lacZ gene derived from *Escherichia coli,* a base sequence encoding alkaline phosphatase, secreted embryonic alkaline phosphatase (SEAP), or chloramphenicol acetyl transferase (CAT). Exemplary luminescent reporter genes may include a base sequence encoding luciferase (luc), $\beta$-galactosidase, CAT, $\beta$-glucuronidase (GUS), or a variant thereof, but are not limited thereto.

In addition, in the present invention, the marker gene is a gene encoding a polypeptide that gives resistance to an exogenous agent or drug. For example, the marker gene may be or include a puromycin resistance gene, a hygromycin resistance gene, a blasticidin resistance gene, a neomycin resistance gene, a geneticin resistance gene, or a zeocin resistance gene, or a modified form thereof, but is not limited thereto.

In addition, in the present invention, the marker gene and one terminal of the polynucleotide of the present invention may be linked by a polynucleotide encoding a cleavable peptide.

In the present invention, the cleavable peptide corresponds to a self-cleavage site, and may be or include, for example, a P2A, F2A, E2A, or T2A peptide, or a combination thereof, but is not limited thereto.

According to yet another embodiment of the present invention, the present invention relates to a recombinant expression vector including the expression cassette provided in the present invention.

The description of the recombinant expression vector in the present invention overlaps with the description of the above-described antibody or antigen-binding fragment, and thus the description thereof is omitted here.

According to yet another embodiment of the present invention, the present invention relates to an immune effector cell transduced with the recombinant expression vector provided in the present invention.

The immune effector cell in the present invention may be an allogeneic or autologous immune cell. In addition, the immune effector cell in the present invention may be derived from T cells, dendritic cells, killer dendritic cells, mast cells, NK cells, NKT cells, macrophages, monocytes, B cells, and stem cells, but is not limited thereto.

The term "natural killer cells (NK cells)" as used herein is defined as large granular lymphocytes (LGL), which constitute three types of cells that differentiate from B and T lymphocytes produced from common lymphoid progenitors. NK cells are known to differentiate and mature in the bone marrow, lymph nodes, spleen, tonsils, and thymus and enter the circulatory system. In the present invention, the NK cells may include any type of NK cells without limitation, and may include cultured NK cells, for example, primary NK cells, NK cells from a cultured NK cell line, or NK cells obtained from a mammal, but are not limited thereto. When the NK cells are obtained from a mammal, the NK cells may be obtained from a number of sources, including, but not limited to, blood, bone marrow, lymph nodes, thymus, or other tissues or fluids. The NK cells may be concentrated or purified. The NK cells may preferably be human NK cells (e.g., isolated from a human). NK cell lines may be obtained from, for example, the American Type Culture Collection (ATCC), and include, for example, NK-92 cells (ATCC CRL-2407), NK92MI cells (ATCC CRL-2408), or derivatives thereof.

According to yet another embodiment of the present invention, the present invention relates to a polypeptide expressed from an immune effector cell provided in the present invention.

**Therapeutic use**

According to yet another embodiment of the present invention, the present invention relates to an antibody or antigen-binding fragment, or a pharmaceutical composition including the antibody or antigen-binding fragment, for preventing, ameliorating, or treating an MYCT1-associated disease.

The antibody or antigen-binding fragment of the present invention exhibits excellent affinity and binding strength to MYCT1. Specifically, the antibody or antigen-binding fragment of the present invention binds specifically to tumor endothelial cells expressing MYCT1 and reprograms the TME, and thereby can be effectively used for the treatment of diseases associated with MYCT1 overexpression, such as cancer, and particularly solid tumors.

The MYCT1-associated disease may be a disease associated with MYCT1 expression or overexpression, such as cancer. Accordingly, the MYCT1-associated disease is preferably cancer or a tumor, but is not limited thereto.

The term "cancer" as used herein refers to or indicates a physiological condition typically characterized by uncontrolled cell growth in mammals.

Cancers or carcinomas that may be treated with the composition of the present invention are not particularly limited and include both solid cancers and hematological cancers. Examples of such cancers include skin cancer such as melanoma, liver cancer, hepatocellular carcinoma, gastric cancer, breast cancer, lung cancer, ovarian cancer, bronchial cancer, nasopharyngeal cancer, laryngeal cancer, pancreatic cancer, bladder cancer, colorectal cancer, colon cancer, cervical cancer, brain cancer, prostate cancer, bone cancer, skin cancer, thyroid cancer, parathyroid cancer, kidney cancer, esophageal cancer, biliary tract cancer, testicular cancer, rectal cancer, head and neck cancer, cervical spine cancer, ureteral cancer, osteosarcoma, neuroblastoma, fibrosarcoma, rhabdomyosarcoma, astrocytoma, neuroblastoma, and glioma, but are not limited thereto.

More preferably, the cancer is characterized by expression of the MYCT1 protein, and may be breast cancer, pancreatic cancer, prostate cancer, lung cancer, thyroid cancer, stomach cancer, ovarian cancer, colon cancer, liver cancer, gallbladder cancer, kidney cancer, cervical cancer, or bladder cancer, but is not limited thereto. The cancer may be primary cancer or metastatic cancer.

In the present invention, the MYCT1-associated disease may be an angiogenesis-associated disease. The angiogenesis-associated disease may be one or more selected from the group consisting of rheumatoid arthritis, osteoarthritis, septic arthritis, psoriasis, corneal ulcer, age-related macular degeneration, diabetic retinopathy, proliferative vitreoretinopathy, immature retinopathy, ophthalmic inflammation, keratoconus, Sjögren's syndrome, myopic ophthalmic tumor, corneal graft rejection, abnormal wound healing, bone diseases, proteinuria, abdominal aortic aneurysm diseases, degenerative cartilage loss due to traumatic joint injury, demyelinating diseases of the nervous system, liver cirrhosis, glomerular diseases, premature rupture of the gestational membrane, inflammatory bowel diseases, periodontal diseases, arteriosclerosis, restenosis, inflammatory diseases of the central nervous system, Alzheimer's disease, and skin aging, but is not limited thereto.

The term "angiogenesis" is used herein to encompass all aspects of blood vessel maintenance and generation. Therefore, angiogenesis encompasses not only the maintenance and regulation of existing vasculature and small blood vessels, but also the formation of new capillaries, leading to neovascularization. Angiogenesis is a complex process involving a series of sequential steps, including endothelial cell-mediated degradation of the vascular basement membrane and interstitial matrix, endothelial cell migration, endothelial cell proliferation, and formation of capillary loops by endothelial cells. Angiogenesis encompasses the growth and/or development of new blood vessels (also referred to as neovascularization), the dilatation of small blood vessels, excessive or prolonged vessel growth, and the maintenance of existing vasculature. MYCT1 is known to be involved in the regulation of angiogenesis (see the non-patent document).

The term "angiogenesis-associated disease" is used herein to refer to a specific pathological process in humans in which angiogenesis is abnormally prolonged. This further encompasses angiogenic diseases and conditions, such as diseases and conditions related to, caused by, or associated with angiogenesis. Non-limiting examples of such diseases include various forms of cancer and metastases characterized by angiogenesis/neovascularization. The antibody and antigen-binding fragment thereof disclosed herein may be used to treat angiogenesis-associated diseases by binding to MYCT1 and inhibiting angiogenesis.

In the present invention, the term "prevention" may include, without limitation, any action that may block, suppress, or delay symptoms caused by an MYCT1-associated disease using the composition of the present invention.

The terms "amelioration" and "treatment" in the present invention may include, without limitation, any action that may ameliorate or benefit symptoms caused by an MYCT1-associated disease using the composition of the present invention.

The pharmaceutical composition of the present invention may be formulated and used in the form of oral dosage forms such as powder, granules, capsules, tablets, and aqueous suspensions, as well as topical preparations, suppositories, and sterile injection solutions, according to conventional methods, but is not limited thereto. Preferably, the pharmaceutical composition may be formulated for intratracheal or inhalational administration; or for use as an injection, but is not limited thereto.

The pharmaceutical composition of the present invention may include a pharmaceutically acceptable carrier. For oral administration, the pharmaceutically acceptable carrier may include a binder, a lubricant, a disintegrant, an excipient, a solubilizer, a dispersant, a stabilizer, a suspending agent, a colorant, a flavoring agent, and the like. For injections, the pharmaceutically acceptable carrier may include a mixture of buffers, preservatives, analgesics, solubilizers, isotonic agents, and stabilizers. For topical administration, the pharmaceutically acceptable carrier may include a base, an excipient, a lubricant, a preservative, and the like. Formulations of the pharmaceutical composition of the present invention may be prepared in various forms by mixing the composition with a pharmaceutically acceptable carrier as described above. For example, for oral administration, formulations may be prepared in the form of tablets, troches, capsules, elixirs, suspensions, syrups, wafers, and the like, and for injections, formulations may be prepared in the form of unit dose ampoules or forms of multiple doses. In addition, the pharmaceutical composition may be formulated as a solution, a suspension, a tablet, a capsule, a sustained-release preparation, and the like.

Examples of carriers, excipients, and diluents suitable for the formulation of the present invention include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, or mineral oils. In addition, fillers, anti-coagulants, lubricants, wetting agents, fragrances, emulsifiers, preservatives, and the like may be additionally included.

The administration routes of the pharmaceutical composition of the present invention include, but are not limited to, oral, intravenous, intramuscular, intraarterial, intramedullary, intrathecal, intracardiac, transdermal, subcutaneous, intraperitoneal, intranasal, enteral, topical, sublingual, or rectal administration. Administration may be oral or parenteral, and is preferably oral, but is not limited thereto.

The parenteral administration of the present invention includes subcutaneous, intradermal, intravenous, intramuscular, intraarticular, intrasynovial, intrasternal, intrathecal, intralesional, and intracranial injection or infusion techniques. The pharmaceutical composition of the present invention may also be administered in the form of a suppository for rectal administration.

The pharmaceutical composition of the present invention may vary depending on various factors including the activity of the specific compound used, age, body weight, general health, sex, diet, administration time, administration route, excretion rate, drug combination, and severity of the specific disease to be prevented or treated, and the dosage of the pharmaceutical composition may vary depending on the patient's condition, body weight, severity of disease, form of medicine, administration route, and period, but may be appropriately selected by a person skilled in the art, and may be administered at 0.0001 to 50 mg/kg or 0.001 to 50 mg/kg per day. The administration may be performed once a day or divided into several times. The administration amount does not limit the scope of the present invention in any way. The pharmaceutical composition according to the present invention may be formulated as a pill, a dragee, a capsule, a liquid, a gel, a syrup, a slurry, or a suspension.

According to yet another specific embodiment of the present invention, the present invention relates to a method of preventing, ameliorating, or treating an MYCT1-associated disease, including a step of administering to a subject a therapeutically effective amount of an antibody or antigen-binding fragment provided by the present invention; or a bispecific or multispecific antibody including the antibody or antigen-binding fragment.

In the present invention, the subject is a subject diagnosed with or suspected of having an MYCT1-associated disease (particularly a disease caused by overexpression of MYCT1). The subject suspected of having the disease is any animal, including humans, monkeys, cows, horses, sheep, pigs, chickens, turkeys, quails, cats, dogs, mice, rats, rabbits, or guinea pigs, that has or may develop the disease, but any subject treatable with the active ingredient provided by the present invention is included without limitation.

The term "pharmaceutically effective amount" as used herein is an amount sufficient to stop or alleviate the physiological effects of a subject or patient caused by an MYCT1-associated disease. An appropriate effective amount may be determined by a treating physician within the scope of sound medical judgment, and may be administered once or in several divided doses. However, for the purpose of the present invention, a specific therapeutically effective amount for a specific patient is preferably applied differently depending on various factors such as the type and degree of response to be achieved, whether other agents are used in some cases, the composition containing the specific active ingredient, the patient's age, weight, general health condition, sex, and diet, administration time, administration route, the number of administrations, the secretion rate of the composition containing the active ingredient, the treatment period, drugs used together or concurrently with the specific composition, and similar factors well known in the medical field.

A total effective amount of the composition of the present invention may be administered to a patient as a single dose, or may be administered by a fractionated treatment protocol in which multiple doses are administered over a long period of time. The content of the active ingredient in the composition of the present invention may vary depending on the severity of the disease. Specifically, a preferred total dosage of the composition of the present invention may be about 0.0001 mg to 500 mg per kg of patient body weight per day. However, since the dosage of the composition is determined by taking into consideration various factors such as the administration route of the pharmaceutical composition and the number of treatments, as well as the patient's age, weight, health condition, sex, severity of the disease, diet, and excretion rate, a person having ordinary skill in the art will be able to determine an appropriate effective dosage according to a specific use of the composition of the present invention. The formulation, administration route, or administration method of the pharmaceutical composition according to the present invention is not particularly limited as long as the effects of the present invention are exhibited.

**Diagnostic use**

According to yet another embodiment of the present invention, the present invention relates to an antibody or antigen-binding fragment, or a composition for diagnosing an MYCT1-associated disease, including the antibody or antigen-binding fragment.

The present invention may be used as a diagnostic marker for assessing the progression of angiogenesis-associated diseases, such as cancer or diseases characterized by angiogenesis, and the prognosis before and after treatment by detecting MYCT1 using the antibody or antigen-binding fragment.

The antibody or antigen-binding fragment of the present invention may be used in any known analytical method, such as competitive binding assay, direct and indirect sandwich assays, and immunoprecipitation assay.

The antibody or antigen-binding fragment of the present invention may be labeled with a label that: (i) provides a detectable signal; (ii) reacts with a first or second label, for example, a second label to modify a detectable signal provided by Fluorescence Resonance Energy Transfer (FRET); (iii) affects mobility, for example, electrophoretic mobility, by charge, hydrophobicity, shape, or other physical parameters; or (iv) provides a capture moiety, such as an affinity, antibody/antigen, or ion complex. Suitable labels include fluorescent labels, luminescent labels, chromophore labels, radioactive labels, isotope labels, isobaric labels, enzymatic labels, particulate labels, in particular metal particle labels, magnetic particle labels, and polymer particle labels, small organic molecules such as biotin, receptor ligands or binding molecules such as cell adhesion proteins or lectins, label-sequences including nucleic acid and/or amino acid residues that are detectable by use of a binding agent, and the like.

In the present invention, the specific type of the label is not particularly limited, but examples thereof include enzymes that utilize hydrogen peroxide to oxidize a dye precursor, such as horseradish peroxidase (HRP), lactoperoxidase, or microperoxidase, biotin/avidin, spin labels, bacteriophage labels, radioisotopes 32P, 14C, 125I, 3H and 131I linked to stable free radicals, and the like, fluorophores, such as rare earth chelates or fluorescein and derivatives thereof, rhodamine and derivatives thereof, dansyl, umbelliferone, luciferases, such as firefly luciferase and bacterial luciferase (U.S. Patent No. 4,737,456), luciferin, 2,3-dihydrophthalazinedione, alkaline phosphatase, $\beta$-galactosidase, glucoamylase, lysozyme, saccharide oxidases such as glucose oxidase, galactose oxidase, and glucose-6-phosphate dehydrogenase, and heterocyclic oxidases such as uricase and xanthine oxidase, but are not limited thereto.

According to yet another embodiment of the present invention, the present invention relates to a kit for diagnosing an MYCT1-associated disease, including the diagnostic composition of the present invention.

The term "kit" in the present invention refers to a tool capable of assessing the expression level of a biomarker by labeling a probe or antibody that specifically binds to a biomarker component with a detectable label. This includes not only direct labeling of a detectable substance related to the probe or antibody through reaction with a substrate, but also indirect labeling in which a chromogenic label that exhibits color development through reactivity with another directly labeled reagent is conjugated. The kit may include a chromogenic substrate solution that undergoes a chromogenic reaction with the label, a washing solution, and other solutions, and may be manufactured to include the reagent components to be used. The kit in the present invention may include essential components for performing reverse transcription PCR (RT-PCR), and may include, in addition to each primer pair specific to the marker gene, a test tube, a reaction buffer, deoxynucleotides (dNTPs), Taq polymerase, reverse transcriptase, DNase, an RNase inhibitor, sterile water, and the like. In addition, the kit may be a kit for detecting genes for cancer diagnosis, including the essential elements necessary for performing a DNA chip assay. The DNA chip kit may include a substrate to which a cDNA corresponding to a gene or a fragment thereof is attached as a probe, and the substrate may include a cDNA corresponding to a quantitative control gene or a fragment thereof. The kit of the present invention is not limited thereto, as long as it is known in the art.

In the present invention, the kit may be an RT-PCR kit, a DNA chip kit, an enzyme-linked immunosorbent assay (ELISA) kit, a protein chip kit, a rapid kit, or a multiple reaction monitoring (MRM) kit.

The kit of the present invention may further include one or more other component compositions, solutions, or devices suitable for the analytical method. For example, the kit may further include essential elements necessary for performing an RT-PCR. The RT-PCR kit includes a primer pair specific to a gene encoding a marker protein. The primers are nucleotides having a sequence specific to the nucleic acid sequence of the gene and may have a length of about 7 to 50 bp, more preferably about 10 to 30 bp. The kit may also include a primer specific to the nucleic acid sequence of a control gene. Other RT-PCR kits may include test tubes or other appropriate containers, reaction buffers (with varying pH and magnesium concentrations), dNTPs, enzymes such as Taq polymerase and reverse transcriptase, DNase, RNase inhibitor, diethylpyrocarbonate (DEPC)-water, sterile water, and the like.

In addition, the diagnostic kit of the present invention may include essential elements necessary for performing a DNA chip assay. The DNA chip kit may include a substrate to which cDNA or oligonucleotides corresponding to a gene or fragment thereof are attached, and reagents, agents, enzymes, and the like for producing a fluorescently labeled probe. The substrate may also include cDNA or oligonucleotides corresponding to a control gene or fragment thereof.

In addition, the diagnostic kit of the present invention may include essential elements necessary for performing an ELISA. The ELISA kit includes an antibody specific to the protein. The antibody has high specificity and affinity for the marker protein and little cross-reactivity with other proteins, and may be a monoclonal antibody, polyclonal antibody, or recombinant antibody. The ELISA kit may also include an antibody specific to a control protein. Other ELISA kits may include reagents capable of detecting bound antibodies, such as labeled secondary antibodies, chromophores, enzymes (e.g., conjugated to the antibody) and their substrates or other substances capable of binding to the antibody.

In the present invention, the fixative for the antigen-antibody binding reaction may include a nitrocellulose membrane, a polyvinylidene fluoride (PVDF) membrane, a well plate synthesized from a polyvinyl resin or a polystyrene resin, a glass slide glass, and the like, but is not limited thereto.

In the present invention, the washing solution preferably contains a phosphate buffer solution, NaCl, and Tween 20, and a buffer solution composed of a 0.02 M phosphate buffer solution, 0.13 M NaCl, and 0.05% Tween 20 (phosphate buffer + Tween 20 (PBST)) is more preferred. After the antigen-antibody binding reaction, and a subsequent reaction with the secondary antibody, the washing solution is added to the fixative in an appropriate amount to wash three to six times. A sulfuric acid solution ($H_2SO_4$) may preferably be used as a reaction stopping solution.

According to yet another specific embodiment of the present invention, a method for providing information for diagnosing an MYCT1-associated disease is provided, including a step of bringing a biological sample isolated from a subject of interest into contact with an antibody or antigen-binding fragment provided in the present invention; or a bispecific or multispecific antibody including the antibody or antigen-binding fragment.

In the present invention, the subject is a subject diagnosed with or suspected of having an MYCT1-associated disease (particularly a disease caused by overexpression of MYCT1). The subject suspected of having the disease is any animal, including humans, monkeys, cows, horses, sheep, pigs, chickens, turkeys, quails, cats, dogs, mice, rats, rabbits, or guinea pigs, that has or may develop the disease, but any subject diagnosable with the active ingredient provided by the present invention is included without limitation.

In the present invention, the biological sample may include whole blood, leukocytes, peripheral blood mononuclear cells, buffy coat, plasma, serum, sputum, tears, mucus, nasal washes, nasal aspirates, breath, urine, semen, saliva, peritoneal washes, pelvic fluids, cystic fluids, meningeal fluids, amniotic fluids, glandular fluids, pancreatic fluids, lymph fluids, pleural fluids, nipple aspirates, bronchial aspirates, synovial fluids, joint aspirates, organ secretions, cells, cell extracts, or cerebrospinal fluid, but is not limited thereto.

The information providing method of the present invention may include a step of measuring the expression level of the MYCT1 protein in the biological sample using the antibody or antigen-binding fragment provided in the present invention.

The measuring of the expression level of the protein in the present invention may be performed using western blotting, ELISA, competitive binding assay, or similar methods.

The information providing method of the present invention may include a step of predicting the onset or high probability of an MYCT1-associated disease when the measured MYCT1 protein expression level in the biological sample is increased compared to a control group. In this case, the control group may refer to the expression level of MYCT1 in a healthy normal control group, but is not limited thereto.

## Combination therapy

According to yet another embodiment of the present invention, the present invention relates to a pharmaceutical composition including an antibody or antigen-binding fragment; an immunoconjugate; a CAR; or an immune effector cell expressing a CAR as an active ingredient, provided in the present invention, as a combination therapy, which is administered in combination with at least one additional therapeutic agent or therapeutic procedure.

The at least one additional therapeutic agent or therapeutic procedure may be selected from one or more of chemotherapy, targeted anticancer therapy, oncolytic drugs, cytotoxic agents, immune-based therapy, a cytokine, a surgical procedure, a radiation procedure, a vaccine, or cell therapy, but is not limited thereto.

The at least one therapeutic agent may be immunotherapy.

The at least one therapeutic agent may be a PD-1 inhibitor, such as a PD-1 antibody. Specifically, the PD-1 inhibitor may be selected from PDR001, nivolumab, pembrolizumab, pidilizumab, MEDI0680, REGN2810, TSR-042, PF-06801591, or AMP-224, but is not limited thereto.

The at least one therapeutic agent may be a PD-L1 inhibitor, such as a PD-L1 antibody. Specifically, the PD-1 inhibitor may be selected from FAZ053, atezolizumab, avelumab, durvalumab, or BMS-936559, but is not limited thereto.

In the present invention, the term "combination" should be understood to refer to simultaneous, separate, or sequential administration. When the administration is sequential or separate, the interval between administrations of the secondary components should be such that the beneficial effects of the combination are not lost.

**Use in enhancing sensitivity to immunotherapy**

According to yet another embodiment of the present invention, the present invention relates to the use of the antibody or antigen-binding fragment provided in the present invention to enhance the sensitivity to immunotherapy in various cancer types.

The term "sensitivity" as used herein refers to whether a specific drug (e.g., an immunotherapy agent) is effective against an individual patient's cancer.

The term "resistance," as used herein when mentioning sensitivity, refers to a lack of sensitivity to the drug, resulting in the drug's ineffectiveness.

For example, the specific drug is primarily immunotherapy, and these anticancer drugs may or may not be effective depending on the type of cancer. Furthermore, even for cancer types on which an anticancer drug is recognized as effective, it is known that the anticancer drug may or may not be effective in individual patients. Whether an anticancer drug is effective against an individual patient's cancer is referred to as anticancer drug sensitivity. Therefore, when the present invention is capable of predicting patients who are expected to respond (responders) and those who are not expected to respond (non-responders) prior to the initiation of treatment, highly effective and safe chemotherapy may be implemented.

A composition include a therapeutically effective amount of the antibody or antigen-binding fragment of the present invention may be administered to a subject resistant to immunotherapy to enhance the subject's sensitivity to the immunotherapy.

The terminology used in the embodiments is for the purpose of describing particular embodiments only and is not intended to limit the present invention. Singular forms include plural forms, unless the context clearly indicates otherwise. Terms such as "comprise" or "have" as used herein should be understood as specifying the presence of features, steps, operations, components, parts or combinations thereof described herein, not precluding the possibility of the presence or addition of one or more other features, steps, operations, components, parts or combinations thereof.

Unless otherwise defined, all terms, including technical and scientific terms used herein, have the same meaning as generally understood by one of ordinary skill in the art to which the embodiments pertain. Terms, such as those defined in commonly used dictionaries, should be interpreted as having a meaning that is consistent with their meaning in the context of the relevant art and are not interpreted in an idealized or overly formal sense unless clearly so defined in the present invention

When describing components of an embodiment, terms such as "first," "second," "A," "B," "(a)," "(b)," and the like may be used. These terms are only intended to distinguish the components from other components, and the nature, order, or sequence of the components is not limited by the terms. When a component is described as being "connected," "coupled," or "linked" to another component, it should be understood that the component may be directly connected or linked to the other component, but that another component may also be "connected," "coupled," or "linked" between the components.

Hereinafter, embodiments are described in detail with reference to the attached drawings. However, the embodiments may be modified in various ways, and the scope of the patent application is not limited or restricted by these embodiments. It should be understood that all modifications, equivalents, or alternatives to the embodiments are included within the scope of the patent application.

In addition, when described with reference to the accompanying drawings, identical components will be assigned the same reference numerals throughout the drawings, and redundant descriptions thereof will be omitted. When describing embodiments, when a detailed description of a related known technology is deemed to unnecessarily obscure the gist of the embodiment, the detailed description will be omitted.

The present invention is susceptible to various modifications and embodiments. Specific embodiments are illustrated in the drawings and described in detail in the following detailed description. However, this is not intended to limit the present invention to specific embodiments, but rather to encompass all modifications, equivalents, and alternatives falling within the spirit and technical scope of the present invention. In describing the present invention, detailed descriptions of related known technologies will be omitted when they are deemed to obscure the gist of the present invention.

**Example 1. Methods of preparing and analyzing humanized anti-MYCT1 antibody**

**1-1. Preparation of humanized anti-MYCT1 antibody**

The present inventors produced humanized antibodies based on mouse antibodies. Specifically, an anti-MYCT1 mouse parental antibody (2B4 clone) having a VH including SEQ ID NO: 41 and a VL including SEQ ID NO: 42; and an anti-MYCT1 mouse parental antibody (11B6 clone) having a VH including SEQ ID NO: 92 and a VL including SEQ ID NO: 93 were obtained. In Table 1 below, CDRs (underlined and bold) are annotated using the Kabat CDR definition.

**[Table 1]**

| Clone | Construct | | SEQ ID NO. |
|---|---|---|---|
| 2B4 | VH | EVQLQQSGPELVKPGASVKMSCKASGYTFT**DYNMH**WVRQSHGKGLEWIG**YINPNNGGSSYNRKFKG**KATLTVNKSSRSASMELRSLTSEDSAVYYCAT**RFDV**WGTGTTVTVSS | 41 |
| | VL | DVVMTQTPLTLSVTIGHPASISC**KSSQSLLFSDGKTYL N**WLLQRPGQSPKRLIY**LVSKLDS**GVPDRFTGSGSGTDFTLKISRVEAEDLGVYYC**WQGTHFPQT**FGGGTKLEIK | 42 |
| 11B6 | VH | EVQLVESGGGLVKPGGSLKLSCAASGFTFS**DFGIH**WVRQAPEKGLEWVG**YISSGSRTIHYADTVKG**RFTISRDNAKNTLFLQMTNLRSEDTAIYYCAR**RDWDPEWLAY**WGQGTLVTISA | 92 |
| | VL | DVVMTQAPLSLPVSLGDQASISC**RSSLSLVHSNGHTYL H**WYLQKPGQSPKLLIY**KVSNRFS**GVPDRFSGSGSGTDFTLKIRRVEAEDLGVYFC**SQSTHVPFT**FGSGTKLEIK | 93 |

By analyzing these mouse antibody sequences, a humanization process was performed in which certain sequences were humanized and other sequences were maintained to minimize the risk of immunogenicity while maintaining binding affinity. Antibodies are composed of variable and constant regions, which are further divided into CDRs that directly bind antigens and FRs that support the CDR loops. Humanized antibodies are initially produced through CDR grafting, a process of grafting mouse CDR loops onto human antibodies (chimerization). However, when simple CDR grafting alone is performed, the affinity of humanized antibodies is reduced. Therefore, several key framework amino acid residues expected to affect the CDR structure were replaced with those of mouse antibodies to increase the affinity to a level similar to that of the original mouse antibody (humanization).

The specific humanization process was outsourced to Wuxi Biologics, and a total of 47 humanized antibodies were produced. Plasmids for IgG antibodies were synthesized by Genewiz, including one plasmid containing a VH in which a VH domain was fused to human IgG1 CH1, a hinge, CH2, and CH3 segments in the heavy chain; and another plasmid containing a VL in which a VL domain was fused to human Igκ CK domain in the light chain. Each construct contained identical constant segments, various VH or VL domains, and various site mutations. A human Igκ monopeptide was fused to the 5'-terminus of the VH or VL for secretory expression.

The CDR sequences of the antibodies produced in this manner are shown in Table 2, and the sequences of the VH and VL are summarized in Tables 2 to 6 below.

**[Table 2]**

| Clone | Construct | Sequence | SEQ ID NO. |
|---|---|---|---|
| 2B4 | CDR-H1 | DYNMH | 3 |
| | CDR-H2 | YINPNNGGSSYNRKFKG | 4 |
| | CDR-H3 | RFDV | 5 |
| | CDR-L1 | KSSQSLLFSDGKTYLN | 6 |

(continued)

| Clone | Construct | Sequence | SEQ ID NO. |
|---|---|---|---|
| | CDR-L2 | LVSKLDS | 7 |
| | CDR-L3 | WQGTHFPQT | 8 |
| 11B6 | CDR-H1 | DFGIH | 9 |
| | CDR-H2 | YISSGSRTIHYADTVKG | 10 |
| | CDR-H3 | RDWDPEWLAY | 11 |
| | CDR-L1 | RSSLSLVHSNGHTYLH | 12 |
| | CDR-L2 | KVSNRFS | 13 |
| | CDR-L3 | SQSTHVPFT | 14 |

[Table 3]

| Clone | Construct | Sequence | SEQ ID NO. |
|---|---|---|---|
| 2B4 | 2B4 FR-H1 | EVQLQQSGPELVKPGASVKMSCKASGYTFT | 15 |
| | 2B4 FR-H1 | QVQLVQSGAEVKKPGASVKVSCKASGYTFT | 16 |
| | 2B4 FR-H1 | QVQLVQSGAEVKKPGASVKMSCKASGYTFT | 17 |
| | 2B4 FR-H2 | WVRQSHGKGLEWIG | 18 |
| | 2B4 FR-H2 | WVRQAPGQGLEWMG | 19 |
| | 2B4 FR-H2 | WVRQAPGQGLEWIG | 20 |
| | 2B4 FR-H3 | KATLTVNKSSRSASMELRSLTSEDSAVYYCAT | 21 |
| | 2B4 FR-H3 | RVTMTTDTSTSTAYMELRSLRSDDTAVYYCAR | 22 |
| | 2B4 FR-H3 | RVTMTTDTSTSTAYMELRSLRSDDTAVYYCAT | 23 |
| | 2B4 FR-H3 | RVTLTTDTSTSTAYMELRSLRSDDTAVYYCAT | 24 |
| | 2B4 FR-H3 | RVTMTVDTSTSTAYMELRSLRSDDTAVYYCAT | 25 |
| | 2B4 FR-H3 | RVTMTTDKSTSTAYMELRSLRSDDTAVYYCAT | 26 |
| | 2B4 FR-H3 | RVTMTVDKSTRTAYMELRSLRSDDTAVYYCAT | 27 |
| | 2B4 FR-H3 | RVTLTVDKSTRTAYMELRSLRSDDTAVYYCAT | 28 |
| | 2B4 FR-H4 | WGTGTTVTVSS | 29 |
| | 2B4 FR-H4 | WGTGTTVTVS | 30 |
| | 2B4 FR-H4 | WGQGTTVTVSS | 31 |
| | 2B4 FR-L1 | DVVMTQTPLTLSVTIGHPASISC | 32 |
| | 2B4 FR-L1 | DVVMTQSPLSLPVTLGQPASISC | 33 |
| | 2B4 FR-L2 | WLLQRPGQSPKRLIY | 34 |
| | 2B4 FR-L2 | WFQQRPGQSPRRLIY | 35 |
| | 2B4 FR-L2 | WLQQRPGQSPRRLIY | 36 |
| | 2B4 FR-L3 | GVPDRFTGSGSGTDFTLKISRVEAEDLGVYYC | 37 |
| | 2B4 FR-L3 | GVPDRFSGSGSGTDFTLKISRVEAEDVGVYYC | 38 |
| | 2B4 FR-L4 | FGGGTKLEIK | 39 |
| | 2B4 FR-L4 | FGGGTKVEIK | 40 |

[Table 4]

| Clone | Construct | Sequence | SEQ ID NO. |
|---|---|---|---|
| 11B6 | 11B6 FR-H1 | EVQLVESGGGLVKPGGSLKLSCAASGFTFS | 67 |
| | 11B6 FR-H1 | EVQLVESGGGLVKPGGSLRLSCAASGFTFS | 68 |
| | 11B6 FR-H2 | WVRQAPEKGLEWVG | 69 |
| | 11B6 FR-H2 | WVRQAPGKGLEWVS | 70 |
| | 11B6 FR-H2 | WVRQAPGKGLEWVG | 71 |
| | 11B6 FR-H3 | RFTISRDNAKNTLFLQMTNLRSEDTAIYYCAR | 72 |
| | 11B6 FR-H3 | RFTISRDNAKNSLYLQMNSLRAEDTAVYYCAR | 73 |
| | 11B6 FR-H3 | RFTISRDNAKNTLYLQMNSLRAEDTAVYYCAR | 74 |
| | 11B6 FR-H3 | RFTISRDNAKNSLFLQMNSLRAEDTAVYYCAR | 75 |
| | 11B6 FR-H3 | RFTISRDNAKNSLYLQMNSLRAEDTAIYYCAR | 76 |
| | 11B6 FR-H3 | RFTISRDNAKNTLFLQMNSLRAEDTAIYYCAR | 77 |
| | 11B6 FR-H4 | WGQGTLVTISA | 78 |
| | 11B6 FR-H4 | WGQGTLVTVSS | 79 |
| | 11B6 FR-L1 | DVVMTQAPLSLPVSLGDQASISC | 80 |
| | 11B6 FR-L1 | DIVMTQSPLSLPVTPGEPASISC | 81 |
| | 11B6 FR-L1 | DVVMTQSPLSLPVTPGEPASISC | 82 |
| | 11B6 FR-L2 | WYLQKPGQSPKLLIY | 83 |
| | 11B6 FR-L2 | WYLQKPGQSPQLLIY | 84 |
| | 11B6 FR-L3 | GVPDRFSGSGSGTDFTLKIRRVEAEDLGVYFC | 85 |
| | 11B6 FR-L3 | GVPDRFSGSGSGTDFTLKISRVEAEDVGVYYC | 86 |
| | 11B6 FR-L3 | GVPDRFSGSGSGTDFTLKIRRVEAEDVGVYYC | 87 |
| | 11B6 FR-L3 | GVPDRFSGSGSGTDFTLKISRVEAEDVGVYFC | 88 |
| | 11B6 FR-L3 | GVPDRFSGSGSGTDFTLKIRRVEAEDVGVYFC | 89 |
| | 11B6 FR-L4 | FGSGTKLEIK | 90 |
| | 11B6 FR-L4 | FGQGTKLEIK | 91 |

[Table 5]

| Clone | Antibody number | VH sequence | SEQ ID NO. | VL sequence | SEQ ID NO. |
|---|---|---|---|---|---|
| 2B4 | p1-uIgG1K | EVQLQQSGPELVKPGASVKMSCKASGYTFTDYNMHWVRQSHGKGLEWIGYINPNQGGSSYNRKFKGKATLTVNKSSRSASMELRSLTSEDSAVYYCATRFDVWGTGTTVTVSS | 43 | DVVMTQTPLTLSVTIGHPASISCKSSQSLLFSDGKTYLNWLLQRPGQSPKRLIYLVSKLDSGVPDRFTGSGSGTDFTLKISRVEAEDLGVYYCWQGTHFPQTFGGGTKLEIK | 42 |
| | p2-uIgG1K | EVQLQQSGPELVKPGASVKMSCKASGYTFTDYNMHWVRQSHGKGLEWIGYINPNSGGSSYNRKFKGKATLTVNKSSRSASMELRSLTSEDSAVYYCATRFDVWGTGTTVTVSS | 44 | DVVMTQTPLTLSVTIGHPASISCKSSQSLLFSDGKTYLNWLLQRPGQSPKRLIYLVSKLDSGVPDRFTGSGSGTDFTLKISRVEAEDLGVYYCWQGTHFPQTFGGGTKLEIK | 42 |

(continued)

| Clone | Antibody number | VH sequence | SEQ ID NO. | VL sequence | SEQ ID NO. |
|---|---|---|---|---|---|
| | p3-uIgG1K | EVQLQQSGPELVKPGASVKM SCKASGYTFTDYNMHWVRQ SHGKGLEWIGYINPNNAGSSY NRKFKGKATLTVNKSSRSAS MELRSLTSEDSAVYYCATRF DVWGTGTTVTVSS | 45 | DVVMTQTPLTLSVTIGHPASISC KSSQSLLFSDGKTYLNWLLQRP GQSPKRLIYLVSKLDSGVPDRFT GSGSGTDFTLKISRVEAEDLGV YYCWQGTHFPQTFGGGTKLEIK | 42 |
| | p4-uIgG1K | EVQLQQSGPELVKPGASVKM SCKASGYTFTDYNMHWVRQ SHGKGLEWIGYINPNNGGSSY NRKFKGKATLTVNKSSRSAS MELRSLTSEDSAVYYCATRF DVWGTGTTVTVSS | 41 | DVVMTQTPLTLSVTIGHPASISC KSSQSLLFSEGKTYLNWLLQRP GQSPKRLIYLVSKLDSGVPDRFT GSGSGTDFTLKISRVEAEDLGV YYCWQGTHFPQTFGGGTKLEIK | 61 |
| | p5-uIgG1K | EVQLQQSGPELVKPGASVKM SCKASGYTFTDYNMHWVRQ SHGKGLEWIGYINPNNGGSSY NRKFKGKATLTVNKSSRSAS MELRSLTSEDSAVYYCATRF DVWGTGTTVTVS | 46 | DVVMTQTPLTLSVTIGHPASISC KSSQSLLFSQGKTYLNWLLQRP GQSPKRLIYLVSKLDSGVPDRFT GSGSGTDFTLKISRVEAEDLGV YYCWQGTHFPQTFGGGTKLEIK | 62 |
| | p6-uIgG1K | EVQLQQSGPELVKPGASVKM SCKASGYTFTDYNMHWVRQ SHGKGLEWIGYINPNNGGSSY NRKFKGKATLTVNKSSRSAS MELRSLTSEDSAVYYCATRF DVWGTGTTVTVSS | 41 | DVVMTQTPLTLSVTIGHPASISC KSSQSLLFSDAKTYLNWLLQRP GQSPKRLIYLVSKLDSGVPDRFT GSGSGTDFTLKISRVEAEDLGV YYCWQGTHFPQTFGGGTKLEIK | 63 |
| | p7-uIgG1K | EVQLQQSGPELVKPGASVKM SCKASGYTFTDYNMHWVRQ SHGKGLEWIGYINPNSGGSSY NRKFKGKATLTVNKSSRSAS MELRSLTSEDSAVYYCATRF DVWGTGTTVTVSS | 44 | DVVMTQTPLTLSVTIGHPASISC KSSQSLLFSDAKTYLNWLLQRP GQSPKRLIYLVSKLDSGVPDRFT GSGSGTDFTLKISRVEAEDLGV YYCWQGTHFPQTFGGGTKLEIK | 63 |
| | z0-uIgG1K | QVQLVQSGAEVKKPGASVKV SCKASGYTFTDYNMHWVRQ APGQGLEWMGYINPNNGGSS YNRKFKGRVTMTTDTSTSTA YMELRSLRSDDTAVYYCARR FDVWGQGTTVTVSS | 47 | DVVMTQSPLSLPVTLGQPASISC KSSQSLLFSDGKTYLNWFQQRP GQSPRRLIYLVSKLDSGVPDRFS GSGSGTDFTLKISRVEAEDVGV YYCWQGTHFPQTFGGGTKVEIK | 64 |
| | z1-uIgG1K | QVQLVQSGAEVKKPGASVKV SCKASGYTFTDYNMHWVRQ APGQGLEWMGYINPNNGGSS YNRKFKGRVTMTTDTSTSTA YMELRSLRSDDTAVYYCATR FDVWGQGTTVTVSS | 48 | DVVMTQSPLSLPVTLGQPASISC KSSQSLLFSDGKTYLNWFQQRP GQSPRRLIYLVSKLDSGVPDRFS GSGSGTDFTLKISRVEAEDVGV YYCWQGTHFPQTFGGGTKVEIK | 64 |
| | z2-uIgG1K | QVQLVQSGAEVKKPGASVKV SCKASGYTFTDYNMHWVRQ APGQGLEWMGYINPNNGGSS YNRKFKGRVTMTTDTSTSTA YMELRSLRSDDTAVYYCATR FDVWGQGTTVTVSS | 48 | DVVMTQSPLSLPVTLGQPASISC KSSQSLLFSDGKTYLNWLQQRP GQSPRRLIYLVSKLDSGVPDRFS GSGSGTDFTLKISRVEAEDVGV YYCWQGTHFPQTFGGGTKVEIK | 65 |

(continued)

| Clone | Antibody number | VH sequence | SEQ ID NO. | VL sequence | SEQ ID NO. |
|---|---|---|---|---|---|
| | z3-uIgG1K | QVQLVQSGAEVKKPGASVKVSCKASGYTFTDYNMHWVRQAPGQGLEWMGYINPNNGGSSYNRKFKGRVTLTTDTSTSTAYMELRSLRSDDTAVYYCATRFDVWGQGTTVTVSS | 49 | DVVMTQSPLSLPVTLGQPASISCKSSQSLLFSDGKTYLNWLQQRPGQSPRRLIYLVSKLDSGVPDRFSGSGSGTDFTLKISRVEAEDVGVYYCWQGTHFPQTFGGGTKVEIK | 65 |
| | z4-uIgG1K | QVQLVQSGAEVKKPGASVKVSCKASGYTFTDYNMHWVRQAPGQGLEWMGYINPNNGGSSYNRKFKGRVTMTVDTSTSTAYMELRSLRSDDTAVYYCATRFDVWGQGTTVTVSS | 50 | DVVMTQSPLSLPVTLGQPASISCKSSQSLLFSDGKTYLNWLQQRPGQSPRRLIYLVSKLDSGVPDRFSGSGSGTDFTLKISRVEAEDVGVYYCWQGTHFPQTFGGGTKVEIK | 65 |
| | z5-uIgG1K | QVQLVQSGAEVKKPGASVKVSCKASGYTFTDYNMHWVRQAPGQGLEWMGYINPNNGGSSYNRKFKGRVTMTTDKSTSTAYMELRSLRSDDTAVYYCATRFDVWGQGTTVTVSS | 51 | DVVMTQSPLSLPVTLGQPASISCKSSQSLLFSDGKTYLNWLQQRPGQSPRRLIYLVSKLDSGVPDRFSGSGSGTDFTLKISRVEAEDVGVYYCWQGTHFPQTFGGGTKVEIK | 65 |
| | z6-uIgG1K | QVQLVQSGAEVKKPGASVKVSCKASGYTFTDYNMHWVRQAPGQGLEWMGYINPNNGGSSYNRKFKGRVTMTTDTSTRTAYMELRSLRSDDTAVYYCATRFDVWGQGTTVTVSS | 52 | DVVMTQSPLSLPVTLGQPASISCKSSQSLLFSDGKTYLNWLQQRPGQSPRRLIYLVSKLDSGVPDRFSGSGSGTDFTLKISRVEAEDVGVYYCWQGTHFPQTFGGGTKVEIK | 65 |
| | z7-uIgG1K | QVQLVQSGAEVKKPGASVKMSCKASGYTFTDYNMHWVRQAPGQGLEWMGYINPNNGGSSYNRKFKGRVTMTTDTSTSTAYMELRSLRSDDTAVYYCATRFDVWGQGTTVTVSS | 53 | DVVMTQSPLSLPVTLGQPASISCKSSQSLLFSDGKTYLNWLQQRPGQSPRRLIYLVSKLDSGVPDRFSGSGSGTDFTLKISRVEAEDVGVYYCWQGTHFPQTFGGGTKVEIK | 65 |
| | z8-uIgG1K | QVQLVQSGAEVKKPGASVKVSCKASGYTFTDYNMHWVRQAPGQGLEWIGYINPNNGGSSYNRKFKGRVTMTTDTSTSTAYMELRSLRSDDTAVYYCATRFDVWGQGTTVTVSS | 54 | DVVMTQSPLSLPVTLGQPASISCKSSQSLLFSDGKTYLNWLQQRPGQSPRRLIYLVSKLDSGVPDRFSGSGSGTDFTLKISRVEAEDVGVYYCWQGTHFPQTFGGGTKVEIK | 65 |
| | z9-uIgG1K | QVQLVQSGAEVKKPGASVKVSCKASGYTFTDYNMHWVRQAPGQGLEWMGYINPNNGGSSYNRKFKGRVTMTVDKSTRTAYMELRSLRSDDTAVYYCATRFDVWGQGTTVTVSS | 55 | DVVMTQSPLSLPVTLGQPASISCKSSQSLLFSDGKTYLNWFQQRPGQSPRRLIYLVSKLDSGVPDRFSGSGSGTDFTLKISRVEAEDVGVYYCWQGTHFPQTFGGGTKVEIK | 64 |
| | z10-uIgG1K | QVQLVQSGAEVKKPGASVKVSCKASGYTFTDYNMHWVRQAPGQGLEWMGYINPNNGGSSYNRKFKGRVTMTVDKSTRTAYMELRSLRSDDTAVYYCATRFDVWGQGTTVTVSS | 55 | DVVMTQSPLSLPVTLGQPASISCKSSQSLLFSDGKTYLNWLQQRPGQSPRRLIYLVSKLDSGVPDRFSGSGSGTDFTLKISRVEAEDVGVYYCWQGTHFPQTFGGGTKVEIK | 65 |

(continued)

| Clone | Antibody number | VH sequence | SEQ ID NO. | VL sequence | SEQ ID NO. |
|---|---|---|---|---|---|
| | z11-uIgG1K | QVQLVQSGAEVKKPGASVKV SCKASGYTFTDYNMHWVRQ APGQGLEWMGYINPNNGGSS YNRKFKGRVTLTVDKSTRTA YMELRSLRSDDTAVYYCATR FDVWGQGTTVTVSS | 56 | DVVMTQSPLSLPVTLGQPASISC KSSQSLLFSDGKTYLNWLQQRP GQSPRRLIYLVSKLDSGVPDRFS GSGSGTDFTLKISRVEAEDVGV YYCWQGTHFPQTFGGGTKVEIK | 65 |
| | z12-uIgG1K | QVQLVQSGAEVKKPGASVK MSCKASGYTFTDYNMHWVR QAPGQGLEWMGYINPNNGGS SYNRKFKGRVTLTVDKSTRT AYMELRSLRSDDTAVYYCAT RFDVWGQGTTVTVSS | 57 | DVVMTQSPLSLPVTLGQPASISC KSSQSLLFSDGKTYLNWLQQRP GQSPRRLIYLVSKLDSGVPDRFS GSGSGTDFTLKISRVEAEDVGV YYCWQGTHFPQTFGGGTKVEIK | 65 |
| | z13-uIgG1K | QVQLVQSGAEVKKPGASVK MSCKASGYTFTDYNMHWVR QAPGQGLEWIGYINPNNGGSS YNRKFKGRVTLTVDKSTRTA YMELRSLRSDDTAVYYCATR FDVWGQGTTVTVSS | 58 | DVVMTQSPLSLPVTLGQPASISC KSSQSLLFSDGKTYLNWLQQRP GQSPRRLIYLVSKLDSGVPDRFS GSGSGTDFTLKISRVEAEDVGV YYCWQGTHFPQTFGGGTKVEIK | 65 |
| | z14-uIgG1K | EVQLQQSGPELVKPGASVKM SCKASGYTFTDYNMHWVRQ SHGKGLEWIGYINPNNGGSSY NRKFKGKATLTVNKSSRSAS MELRSLTSEDSAVYYCATRF DVWGTGTTVTVSS | 41 | DVVMTQSPLSLPVTLGQPASISC KSSQSLLFSDGKTYLNWLQQRP GQSPRRLIYLVSKLDSGVPDRFS GSGSGTDFTLKISRVEAEDVGV YYCWQGTHFPQTFGGGTKVEIK | 65 |
| | z15-uIgG1K | QVQLVQSGAEVKKPGASVK MSCKASGYTFTDYNMHWVR QAPGQGLEWIGYINPNNGGSS YNRKFKGRVTLTVDKSTRTA YMELRSLRSDDTAVYYCATR FDVWGQGTTVTVSS | 58 | DVVMTQTPLTLSVTIGHPASISC KSSQSLLFSDGKTYLNWLLQRP GQSPKRLIYLVSKLDSGVPDRFT GSGSGTDFTLKISRVEAEDLGV YYCWQGTHFPQTFGGGTKLEIK | 42 |
| | z2-p7-uIgG1K | QVQLVQSGAEVKKPGASVKV SCKASGYTFTDYNMHWVRQ APGQGLEWMGYINPNSGGSS YNRKFKGRVTMTTDTSTSTA YMELRSLRSDDTAVYYCATR FDVWGQGTTVTVSS | 59 | DVVMTQSPLSLPVTLGQPASISC KSSQSLLFSDAKTYLNWLQQRP GQSPRRLIYLVSKLDSGVPDRFS GSGSGTDFTLKISRVEAEDVGV YYCWQGTHFPQTFGGGTKVEIK | 66 |
| | z8-p7-uIgG1K | QVQLVQSGAEVKKPGASVKV SCKASGYTFTDYNMHWVRQ APGQGLEWIGYINPNSGGSSY NRKFKGRVTMTTDTSTSTAY MELRSLRSDDTAVYYCATRF DVWGQGTTVTVSS | 60 | DVVMTQSPLSLPVTLGQPASISC KSSQSLLFSDAKTYLNWLQQRP GQSPRRLIYLVSKLDSGVPDRFS GSGSGTDFTLKISRVEAEDVGV YYCWQGTHFPQTFGGGTKVEIK | 66 |

[Table 6]

| Clone | Antibody number | VH sequence | SEQ ID NO. | VL sequence | SEQ ID NO. |
|---|---|---|---|---|---|
| 11B6 | p1-uIgG1K | EVQLVESGGGLVKPGGSLKLSCAASGFTFSDFGIHWVRQAPEKGLEWVGYISSGSRTIHYADTVKGRFTISRDNAKNTLFLQMTNLRSEDTAIYYCARRDWDPEWLAYWGQGTLVTISA | 92 | DVVMTQAPLSLPVSLGDQASISCRSSLSLVHSQGHTYLHWYLQKPGQSPKLLIYKVSNRFSGVPDRFSGSGSGTDFTLKIRRVEAEDLGVYFCSQSTHVPFTFGSGTKLEIK | 102 |
| | p2-uIgG1K | EVQLVESGGGLVKPGGSLKLSCAASGFTFSDFGIHWVRQAPEKGLEWVGYISSGSRTIHYADTVKGRFTISRDNAKNTLFLQMTNLRSEDTAIYYCARRDWDPEWLAYWGQGTLVTISA | 92 | DVVMTQAPLSLPVSLGDQASISCRSSLSLVHSSGHTYLHWYLQKPGQSPKLLIYKVSNRFSGVPDRFSGSGSGTDFTLKIRRVEAEDLGVYFCSQSTHVPFTFGSGTKLEIK | 103 |
| | p3-uIgG1K | EVQLVESGGGLVKPGGSLKLSCAASGFTFSDFGIHWVRQAPEKGLEWVGYISSGSRTIHYADTVKGRFTISRDNAKNTLFLQMTNLRSEDTAIYYCARRDWDPEWLAYWGQGTLVTISA | 92 | DVVMTQAPLSLPVSLGDQASISCRSSLSLVHSNAHTYLHWYLQKPGQSPKLLIYKVSNRFSGVPDRFSGSGSGTDFTLKIRRVEAEDLGVYFCSQSTHVPFTFGSGTKLEIK | 104 |
| | z0-uIgG1K | EVQLVESGGGLVKPGGSLRLSCAASGFTFSDFGIHWVRQAPGKGLEWVSYISSGSRTIHYADTVKGRFTISRDNAKNSLYLQMNSLRAEDTAVYYCARRDWDPEWLAYWGQGTLVTVSS | 94 | DIVMTQSPLSLPVTPGEPASISCRSSLSLVHSNGHTYLHWYLQKPGQSPQLLIYKVSNRFSGVPDRFSGSGSGTDFTLKISRVEAEDVGVYYCQSTHVPFTFGQGTKLEIK | 105 |
| | z1-uIgG1K | EVQLVESGGGLVKPGGSLRLSCAASGFTFSDFGIHWVRQAPGKGLEWVGYISSGSRTIHYADTVKGRFTISRDNAKNSLYLQMNSLRAEDTAVYYCARRDWDPEWLAYWGQGTLVTVSS | 95 | DIVMTQSPLSLPVTPGEPASISCRSSLSLVHSNGHTYLHWYLQKPGQSPQLLIYKVSNRFSGVPDRFSGSGSGTDFTLKISRVEAEDVGVYYCQSTHVPFTFGQGTKLEIK | 105 |
| | z2-uIgG1K | EVQLVESGGGLVKPGGSLRLSCAASGFTFSDFGIHWVRQAPGKGLEWVSYISSGSRTIHYADTVKGRFTISRDNAKNTLYLQMNSLRAEDTAVYYCARRDWDPEWLAYWGQGTLVTVSS | 96 | DIVMTQSPLSLPVTPGEPASISCRSSLSLVHSNGHTYLHWYLQKPGQSPQLLIYKVSNRFSGVPDRFSGSGSGTDFTLKISRVEAEDVGVYYCQSTHVPFTFGQGTKLEIK | 105 |
| | z3-uIgG1K | EVQLVESGGGLVKPGGSLRLSCAASGFTFSDFGIHWVRQAPGKGLEWVSYISSGSRTIHYADTVKGRFTISRDNAKNSLFLQMNSLRAEDTAVYYCARRDWDPEWLAYWGQGTLVTVSS | 97 | DIVMTQSPLSLPVTPGEPASISCRSSLSLVHSNGHTYLHWYLQKPGQSPQLLIYKVSNRFSGVPDRFSGSGSGTDFTLKISRVEAEDVGVYYCQSTHVPFTFGQGTKLEIK | 105 |
| | z4-uIgG1K | EVQLVESGGGLVKPGGSLRLSCAASGFTFSDFGIHWVRQAPGKGLEWVSYISSGSRTIHYADTVKGRFTISRDNAKNSLYLQMNSLRAEDTAIYYCARRDWDPEWLAYWGQGTLVTVSS | 98 | DIVMTQSPLSLPVTPGEPASISCRSSLSLVHSNGHTYLHWYLQKPGQSPQLLIYKVSNRFSGVPDRFSGSGSGTDFTLKISRVEAEDVGVYYCQSTHVPFTFGQGTKLEIK | 105 |

(continued)

| Clone | Antibody number | VH sequence | SEQ ID NO. | VL sequence | SEQ ID NO. |
|---|---|---|---|---|---|
| | z5-uIgG1K | EVQLVESGGGLVKPGGSLRLSCAASGFTFSDFGIHWVRQAPGKGLEWVSYISSGSRTIHYADTVKGRFTISRDNAKNSLYLQMNSLRAEDTAVYYCARRDWDPEWLAYWGQGTLVTVSS | 99 | DVVMTQSPLSLPVTPGEPASISCRSSLSLVHSNGHTYLHWYLQKPGQSPQLLIYKVSNRFSGVPDRFSGSGSGTDFTLKISRVEAEDVGVYYCSQSTHVPFTFGQGTKLEIK | 106 |
| | z6-uIgG1K | EVQLVESGGGLVKPGGSLRLSCAASGFTFSDFGIHWVRQAPGKGLEWVSYISSGSRTIHYADTVKGRFTISRDNAKNSLYLQMNSLRAEDTAVYYCARRDWDPEWLAYWGQGTLVTVSS | 99 | DIVMTQSPLSLPVTPGEPASISCRSSLSLVHSNGHTYLHWYLQKPGQSPKLLIYKVSNRFSGVPDRFSGSGSGTDFTLKISRVEAEDVGVYYCSQSTHVPFTFGQGTKLEIK | 107 |
| | z7-uIgG1K | EVQLVESGGGLVKPGGSLRLSCAASGFTFSDFGIHWVRQAPGKGLEWVSYISSGSRTIHYADTVKGRFTISRDNAKNSLYLQMNSLRAEDTAVYYCARRDWDPEWLAYWGQGTLVTVSS | 99 | DIVMTQSPLSLPVTPGEPASISCRSSLSLVHSNGHTYLHWYLQKPGQSPQLLIYKVSNRFSGVPDRFSGSGSGTDFTLKIRRVEAEDVGVYYCSQSTHVPFTFGQGTKLEIK | 108 |
| | z8-uIgG1K | EVQLVESGGGLVKPGGSLRLSCAASGFTFSDFGIHWVRQAPGKGLEWVSYISSGSRTIHYADTVKGRFTISRDNAKNSLYLQMNSLRAEDTAVYYCARRDWDPEWLAYWGQGTLVTVSS | 99 | DIVMTQSPLSLPVTPGEPASISCRSSLSLVHSNGHTYLHWYLQKPGQSPQLLIYKVSNRFSGVPDRFSGSGSGTDFTLKISRVEAEDVGVYFCSQSTHVPFTFGQGTKLEIK | 109 |
| | z9-uIgG1K | EVQLVESGGGLVKPGGSLRLSCAASGFTFSDFGIHWVRQAPGKGLEWVSYISSGSRTIHYADTVKGRFTISRDNAKNSLYLQMNSLRAEDTAVYYCARRDWDPEWLAYWGQGTLVTVSS | 99 | DVVMTQSPLSLPVTPGEPASISCRSSLSLVHSNGHTYLHWYLQKPGQSPQLLIYKVSNRFSGVPDRFSGSGSGTDFTLKISRVEAEDVGVYFCSQSTHVPFTFGQGTKLEIK | 110 |
| | z10-uIgG1K | EVQLVESGGGLVKPGGSLRLSCAASGFTFSDFGIHWVRQAPGKGLEWVGYISSGSRTIHYADTVKGRFTISRDNAKNSLYLQMNSLRAEDTAVYYCARRDWDPEWLAYWGQGTLVTVSS | 95 | DVVMTQSPLSLPVTPGEPASISCRSSLSLVHSNGHTYLHWYLQKPGQSPQLLIYKVSNRFSGVPDRFSGSGSGTDFTLKISRVEAEDVGVYFCSQSTHVPFTFGQGTKLEIK | 110 |
| | z11-uIgG1K | EVQLVESGGGLVKPGGSLRLSCAASGFTFSDFGIHWVRQAPGKGLEWVGYISSGSRTIHYADTVKGRFTISRDNAKNSLYLQMNSLRAEDTAIYYCARRDWDPEWLAYWGQGTLVTVSS | 100 | DVVMTQSPLSLPVTPGEPASISCRSSLSLVHSNGHTYLHWYLQKPGQSPQLLIYKVSNRFSGVPDRFSGSGSGTDFTLKISRVEAEDVGVYFCSQSTHVPFTFGQGTKLEIK | 110 |
| | z12-uIgG1K | EVQLVESGGGLVKPGGSLRLSCAASGFTFSDFGIHWVRQAPGKGLEWVGYISSGSRTIHYADTVKGRFTISRDNAKNSLYLQMNSLRAEDTAIYYCARRDWDPEWLAYWGQGTLVTVSS | 100 | DVVMTQSPLSLPVTPGEPASISCRSSLSLVHSNGHTYLHWYLQKPGQSPKLLIYKVSNRFSGVPDRFSGSGSGTDFTLKIRRVEAEDVGVYFCSQSTHVPFTFGQGTKLEIK | 111 |

(continued)

| Clone | Antibody number | VH sequence | SEQ ID NO. | VL sequence | SEQ ID NO. |
|---|---|---|---|---|---|
| | z13-uIgG1K | EVQLVESGGGLVKPGGSLRLSCAASGFTFSDFGIHWVRQAPGKGLEWVGYISSGSRTIHYADTVKGRFTISRDNAKNTLFLQMNSLRAEDTAIYYCARRDWDPEWLAYWGQGTLVTVSS | 101 | DVVMTQSPLSLPVTPGEPASISCRSSLSLVHSNGHTYLHWYLQKPGQSPKLLIYKVSNRFSGVPDRFSGSGSGTDFTLKIRRVEAEDVGVYFCSQSTHVPFTFGQGTKLEIK | 111 |
| | z14-uIgG1K | EVQLVESGGGLVKPGGSLKLSCAASGFTFSDFGIHWVRQAPEKGLEWVGYISSGSRTIHYADTVKGRFTISRDNAKNTLFLQMTNLRSEDTAIYYCARRDWDPEWLAYWGQGTLVTISA | 92 | DVVMTQSPLSLPVTPGEPASISCRSSLSLVHSNGHTYLHWYLQKPGQSPKLLIYKVSNRFSGVPDRFSGSGSGTDFTLKIRRVEAEDVGVYFCSQSTHVPFTFGQGTKLEIK | 111 |
| | z15-uIgG1K | EVQLVESGGGLVKPGGSLRLSCAASGFTFSDFGIHWVRQAPGKGLEWVGYISSGSRTIHYADTVKGRFTISRDNAKNTLFLQMNSLRAEDTAIYYCARRDWDPEWLAYWGQGTLVTVSS | 101 | DVVMTQAPLSLPVSLGDQASISCRSSLSLVHSNGHTYLHWYLQKPGQSPKLLIYKVSNRFSGVPDRFSGSGSGTDFTLKIRRVEAEDLGVYFCSQSTHVPFTFGSGTKLEIK | 112 |
| | z10-p3-uIgG1K | EVQLVESGGGLVKPGGSLRLSCAASGFTFSDFGIHWVRQAPGKGLEWVGYISSGSRTIHYADTVKGRFTISRDNAKNSLYLQMNSLRAEDTAVYYCARRDWDPEWLAYWGQGTLVTVSS | 95 | DVVMTQSPLSLPVTPGEPASISCRSSLSLVHSNAHTYLHWYLQKPGQSPQLLIYKVSNRFSGVPDRFSGSGSGTDFTLKISRVEAEDVGVYFCSQSTHVPFTFGQGTKLEIK | 113 |
| | z12-p3-uIgG1K | EVQLVESGGGLVKPGGSLRLSCAASGFTFSDFGIHWVRQAPGKGLEWVGYISSGSRTIHYADTVKGRFTISRDNAKNSLYLQMNSLRAEDTAIYYCARRDWDPEWLAYWGQGTLVTVSS | 100 | DVVMTQSPLSLPVTPGEPASISCRSSLSLVHSNAHTYLHWYLQKPGQSPKLLIYKVSNRFSGVPDRFSGSGSGTDFTLKIRRVEAEDVGVYFCSQSTHVPFTFGQGTKLEIK | 114 |
| | z16-p3-uIgG1K | EVQLVESGGGLVKPGGSLRLSCAASGFTFSDFGIHWVRQAPGKGLEWVGYISSGSRTIHYADTVKGRFTISRDNAKNSLYLQMNSLRAEDTAVYYCARRDWDPEWLAYWGQGTLVTVSS | 95 | DIVMTQSPLSLPVTPGEPASISCRSSLSLVHSNAHTYLHWYLQKPGQSPKLLIYKVSNRFSGVPDRFSGSGSGTDFTLKISRVEAEDVGVYFCSQSTHVPFTFGQGTKLEIK | 115 |

## 1-2. Expression of humanized anti-MYCT1 antibody

Day 1: Typically, ExpiCHO cells ($\geq 6.0 \times 10^6$/mL) with a viability of $\geq$95% in 100 mL of cell culture medium were prepared for transfection. 71.2 $\mu$g of plasmid DNA and 228.5 $\mu$L of ExpiFectamine™ CHO reagent were diluted in 2.54 mL and 2.51 mL of OptiPRO™ serum-free medium (SFM), respectively, and mixed. The cells were added to the ExpiCHO cell culture medium and incubated on a platform shaker set to 110 rpm, 37 °C, and 8% $CO_2$.

Day 2: 18 to 22 hours after transfection, 428.5 $\mu$L of enhancer and 21.5 mL of feed were added to the culture medium to support high-density transient transfection and enhance protein production.

After 10 days of culture, cells were pelleted by centrifugation at 4000 rpm (3724 g, BECKMAN ALLERGA X-15 CENTRIFUGE, catalog number: B31016) at 25 °C for 10 minutes. The supernatant was collected and used for purification and gel electrophoresis. Sodium dodecyl sulfate-polyacrylamide gel electrophoresis (SDS-PAGE) samples were prepared by mixing 15 $\mu$L of the supernatant with 5 $\mu$L of 4X loading buffer and boiling for 5 minutes. 15 $\mu$L of the sample was loaded onto NuPAGE™ 4% to 12% Bis-Tris Protein Gels (ThermoFisher), and SDS-PAGE was run at a constant 200 V for 35 minutes using 2-(N-morpholino)ethanesulfonic acid (MES) running buffer. The proteins in the gel were then stained with Coomassie blue. The molecular weight of the antibodies was determined using the PageRuler™ Unstained Protein Ladder (ThermoFisher) with the antibody samples.

**1-3. Purification of humanized anti-MYCT1 antibody**

The Protein A column was prepacked with 5 mL MabSelectSure resin (GE Life Sciences, Cat. # 17543803). The column was equilibrated with 0.1 M Tris, pH 7.0, before loading the cell culture supernatant containing the expressed IgG. After loading, the column was washed with 10 CV of 0.1 M Tris, pH 7.0, and eluted with 0.1 M glycine, pH 3.5. The eluate was then neutralized with 0.1 M Tris, pH 9.0, and dialyzed against 1X PBS buffer (Sangon Biotech CO., LTD., B548117-0500).

Purification process (protein A column):

A. Buffer preparation

1) Buffer A: 0.1 M Tris, pH 7.0
2) Buffer B: 0.1 M Glycine, pH 3.5
3) PBS: 137 mM NaCl, 2.68 mM KCl, 1.76 mM $KH_2PO_4$, 10 mM $Na_2HPO_4$

B. Purification

1) A collection tube was prepared.
2) The column was equilibrated with Wash Buffer A at 1 mL/min with 10 CV.
3) The sample was loaded onto the column.
4) The column was washed with 10 CV of Buffer A.
5) The protein was eluted with 10 CV of Buffer B. Fractions with UV intensities of 100 mAU or more were collected. 1 mL and 2 to 3 mL fractions of each fraction were collected, analyzed by SDS-PAGE, and combined.

C. Dialysis

The protein was dialyzed against PBS buffer.

**1-4. Characterization of humanized anti-MYCT1 antibody**

To obtain the antibody concentration, absorbance at 280 nm was measured using a Nanodrop. Antibody purity was assessed by SDS-PAGE and size exclusion chromatography-high-performance liquid chromatography (SEC-HPLC). SDS-PAGE samples were prepared by mixing 15 μL of protein sample with 5 μL of 4X loading buffer and boiling the resulting mixture for five minutes. 15 μL of the mixture was loaded onto a NuPAGE Bis-Tris Mini Gels 4% to 12% gel, and SDS-PAGE was run at 200 V for 35 minutes using MES running buffer. Proteins on the gel were then stained with Coomassie blue. For SEC-HPLC, 80 μL of purified antibody was loaded onto a TSKgel G3000SWxL column connected to a 1260 Infinity II HPLC system using a running buffer of 50 mM sodium phosphate, 150 mM NaCl, pH 7.0. The runtime was 20 minutes. Protein peaks were monitored with a UV detector at 280 nm. Peak retention times were analyzed using ChemStation software (V2.99.2.0).

**1-5. Binding affinity measurement by surface plasmon resonance (SPR)**

**1-5-1. $k_{off}$ (dissociation rate constant) ranking**

The activator was prepared by mixing 400 mM 1-ethyl-3-(3-dimethylaminopropyl)carbodiimide (EDC) and 100 mM N-hydroxysuccinimide (NHS) immediately before injection. The CM5 sensor chip was activated with this mixture at a flow rate of 10 μL/min for 420 seconds. Thereafter, 30 μg/mL anti-human Fc IgG in 10 mM NaAc (pH 4.5) was injected into the chip at a flow rate of 10 μL/min for 420 seconds. The chip was then deactivated with 1 M ethanolamine-HCl (GE) at a flow rate of 10 μL/min for 420 seconds.

Parent antibody, humanized, and post-translational modification (PTM)-eliminated variants were captured to Fc2 via anti-human Fc IgG at 13 to 20 μg/mL in running buffer 1X HBS-EP+ (10 mM 2-[4-(2-hydroxyethyl)-1-piperazinyl]-ethanesulfonic acid (HEPES), 150 mM NaCl, 3 mM ethylenediaminetetraacetic acid (EDTA), 0.05% Tween 20, pH 7.4). Fc IgG was injected at a flow rate of 10 μL/min for 60 seconds. Antigen, MYCT1 (10 nM), and running buffer were injected at a flow rate of 100 μL/min across channels 1 to 8 during a 120 sec association step, followed by a 300 sec dissociation step. 10 mM glycine pH 1.5 was injected as a regeneration buffer after each dissociation step. The chip was regenerated with 10 mM glycine pH 1.5.

The surface channel Fc1, which contained no captured ligand, was used as a control surface for baseline subtraction. The final data for each interaction was subtracted from the reference Fc1 and buffer channel data. The experimental data was analyzed using Biacore 8K (version 3.0.12.15655) evaluation software, using a 1:1 binding model.

## 1-5-2. SPR full kinetics test

The activator was prepared by mixing 400 mM EDC and 100 mM NHS immediately before injection. The CM5 sensor chip was activated with this mixture at a flow rate of 10 μL/min for 420 seconds. Thereafter, 30 μg/mL anti-human Fc IgG in 10 mM NaAc (pH 4.5) was injected into the chip at a flow rate of 10 μL/min for 420 seconds. The chip was then deactivated with 1 M ethanolamine-HCl (GE) at a flow rate of 10 μL/min for 420 seconds.

3 μg/mL parent antibody and lead molecules diluted in running buffer 1X HBS-EP+ were captured to Fc2 via anti-human Fc IgG at a flow rate of 10 μL/min for 150 seconds. Multicycle kinetics were used to perform the analysis. Analyte at various concentrations (0.625, 1.25, 2.5, 5, and 10 nM) and running buffer were sequentially injected into Fc1-Fc2 at a flow rate of 100 μL/min for a 120-second association step, followed by a 300-second dissociation step. After each dissociation step, 10 mM glycine pH 1.5 was injected as a regeneration buffer. The chip was regenerated with 10 mM glycine pH 1.5.

The surface channel Fc1, which contained no captured ligand, was used as a control surface for baseline subtraction. The final data for each interaction was subtracted from the reference Fc1 and buffer channel data. The experimental data was analyzed using Biacore 8K (version 3.0.12.15655) evaluation software, using a 1:1 binding model.

## 1-6. Thermal stability evaluated by differential scanning fluorimetry (DSF)

The Tm of the lead antibody was investigated using the QuantStudio™ 7 Flex Real-Time PCR System (Applied Biosystems). 19 μL of antibody solution was mixed with 1 μL of 62.5X SYPRO Orange solution (Invitrogen) and transferred to a 96-well plate (Biosystems S. A.). The plate was heated from 26 °C to 95 °C at a rate of 0.9 °C/min, and the resulting fluorescence data was collected. The negative derivative of the fluorescence change at different temperatures was calculated, and the maximum value was defined as the melting temperature (Tm). Data collection and Tm calculation were performed automatically by the operating software (QuantStudio™ Real-Time PCR Software v1.3).

## Example 2. Analytical results

### 2-1. Expression of parent and humanized antibodies

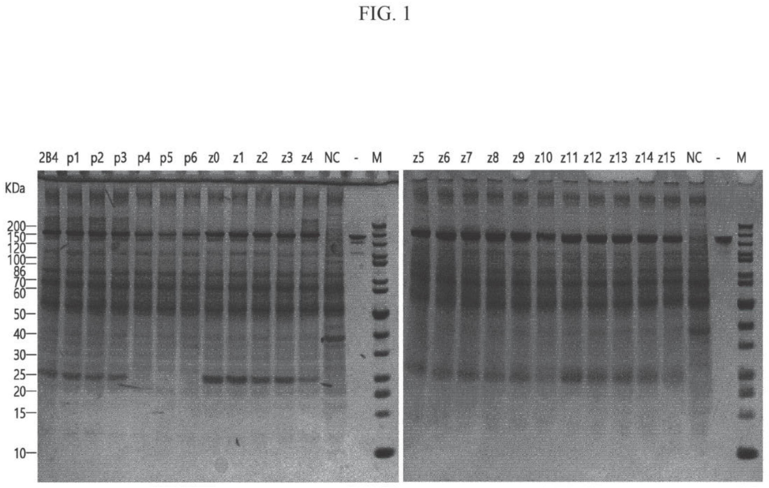

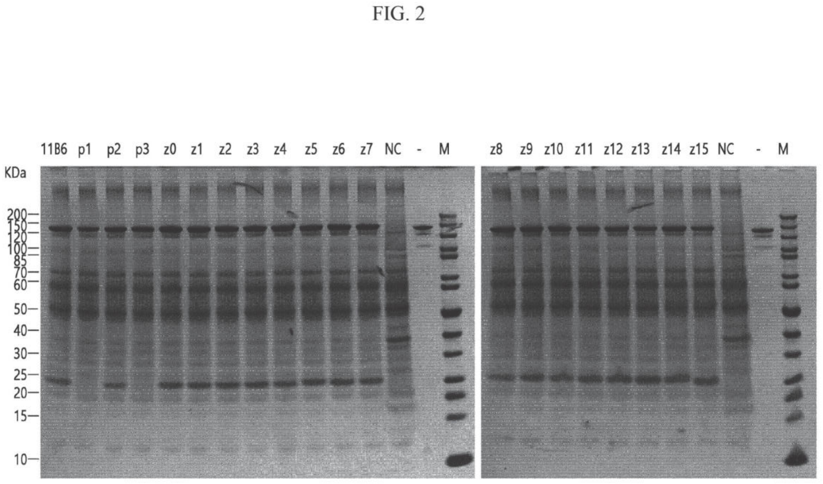

Supernatant expression was detected by SDS-PAGE (FIG. 1). Twenty antibodies (11B6) were expressed using the pcDNA3.4 expression vector and ExpiCHO cells. Supernatant expression was detected by SDS-PAGE (FIG. 2).

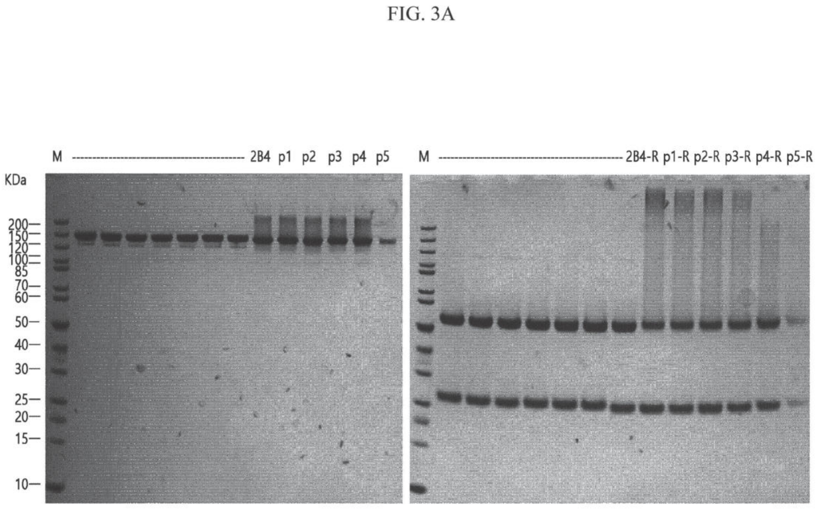

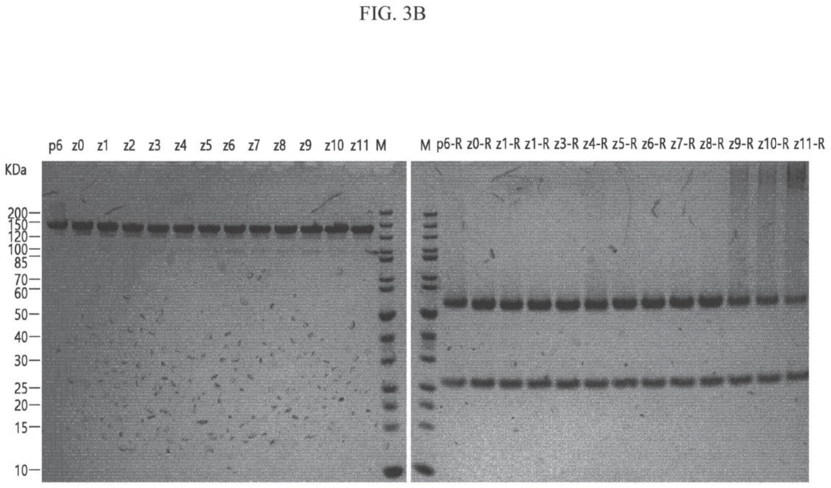

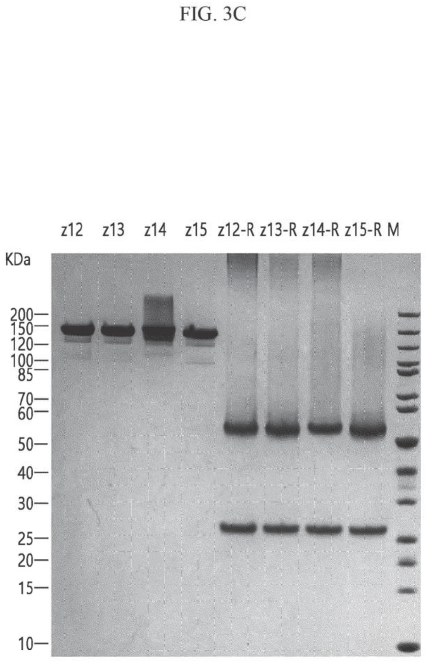

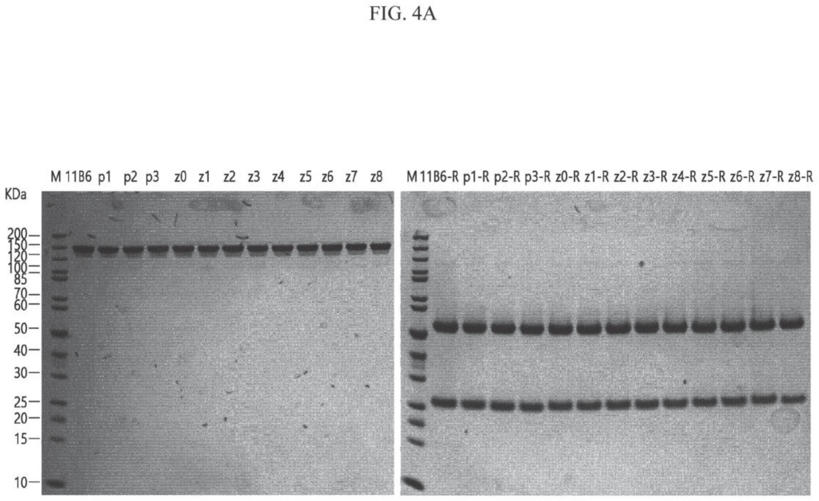

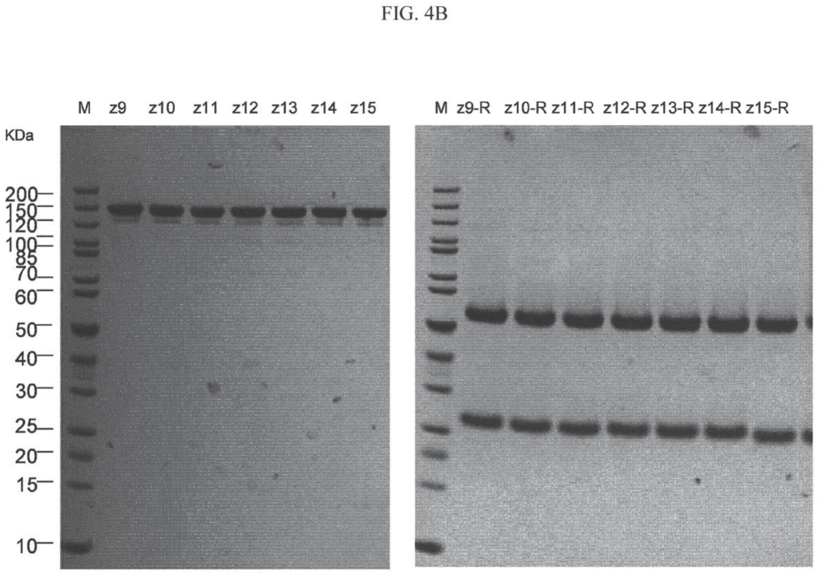

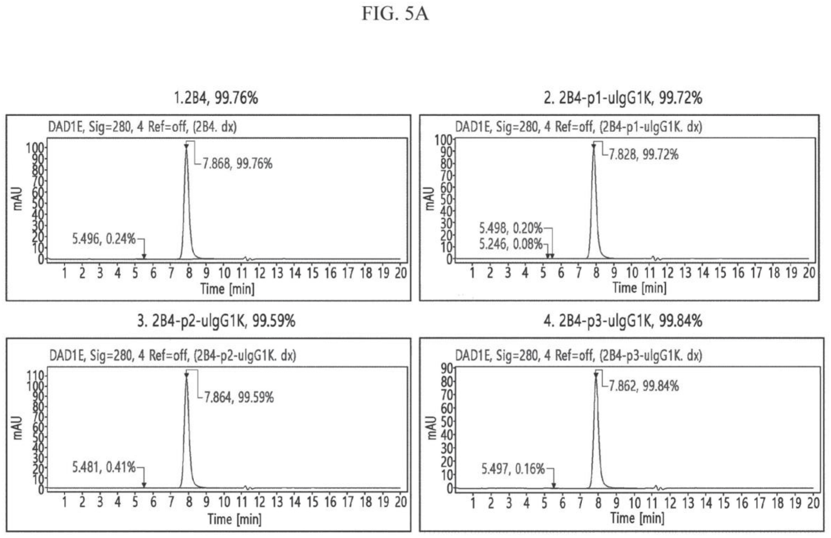

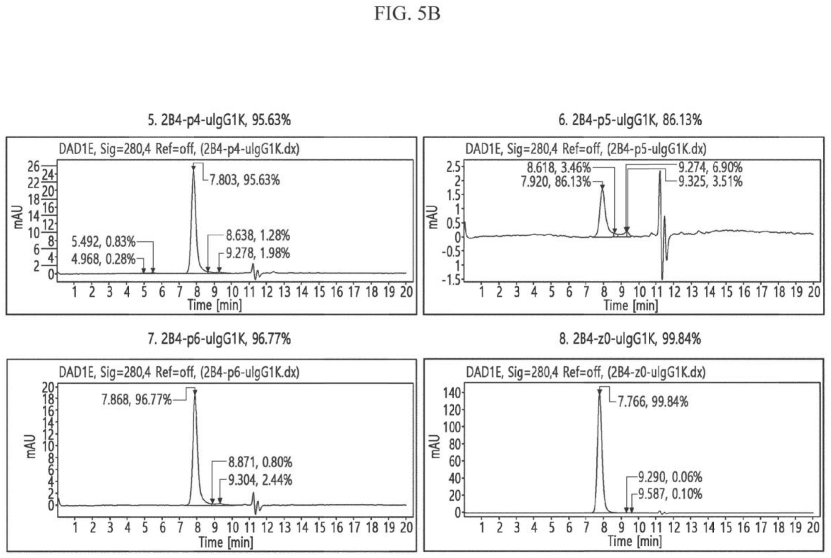

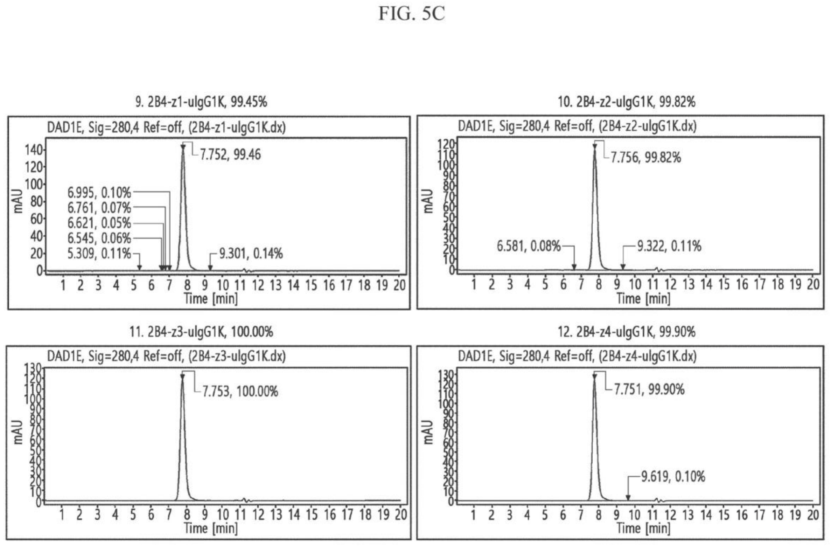

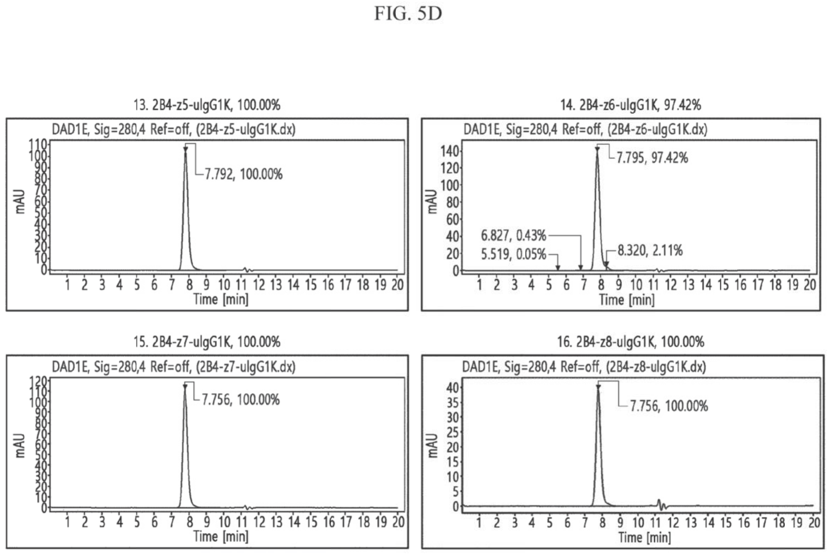

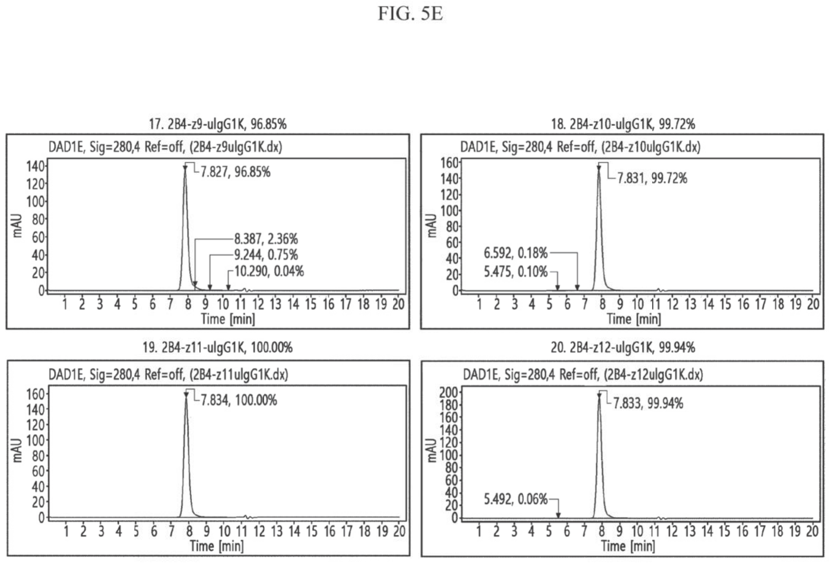

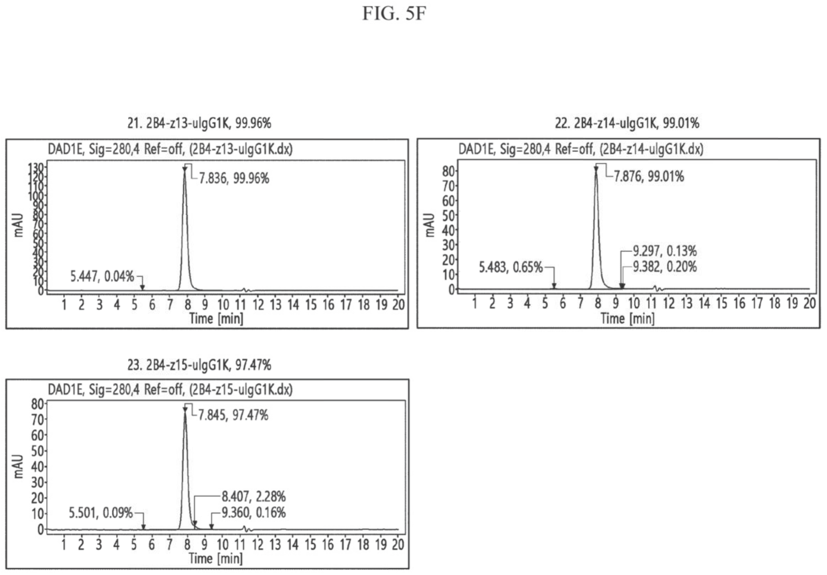

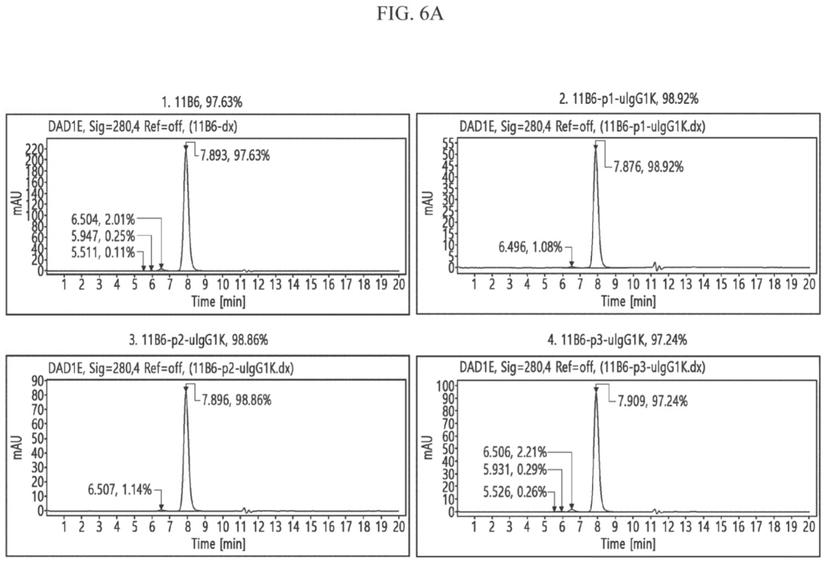

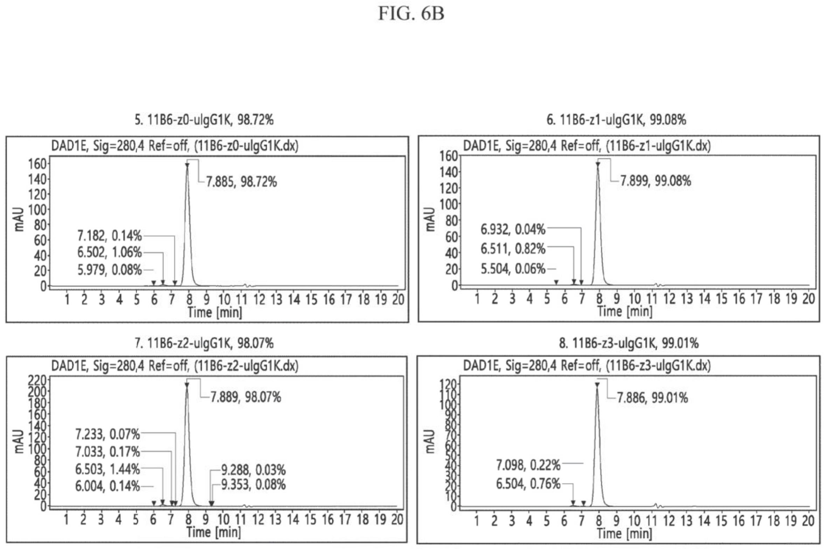

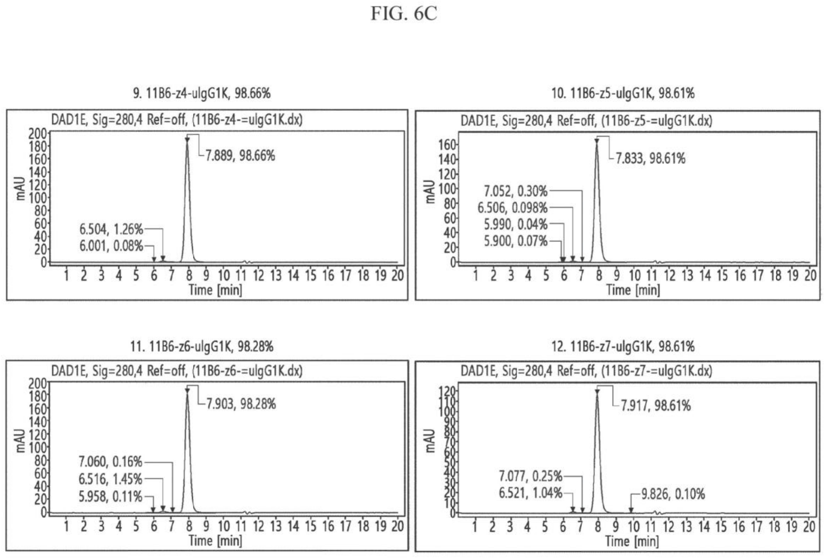

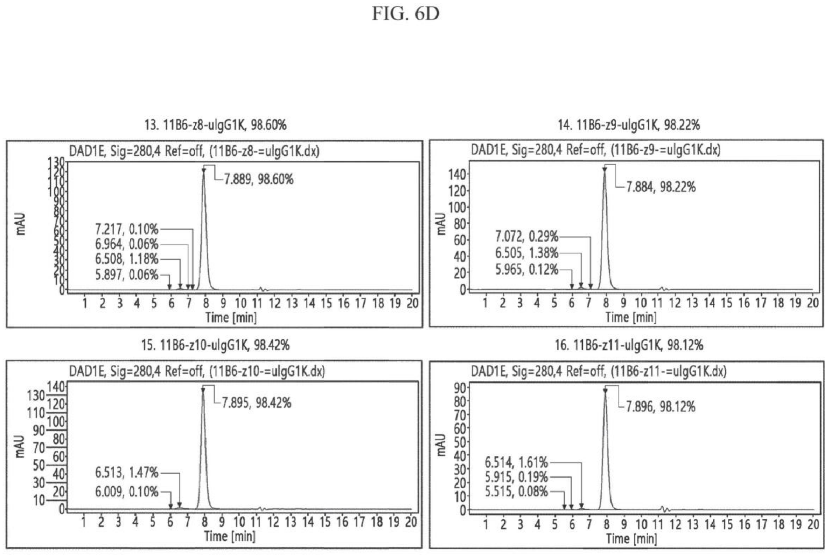

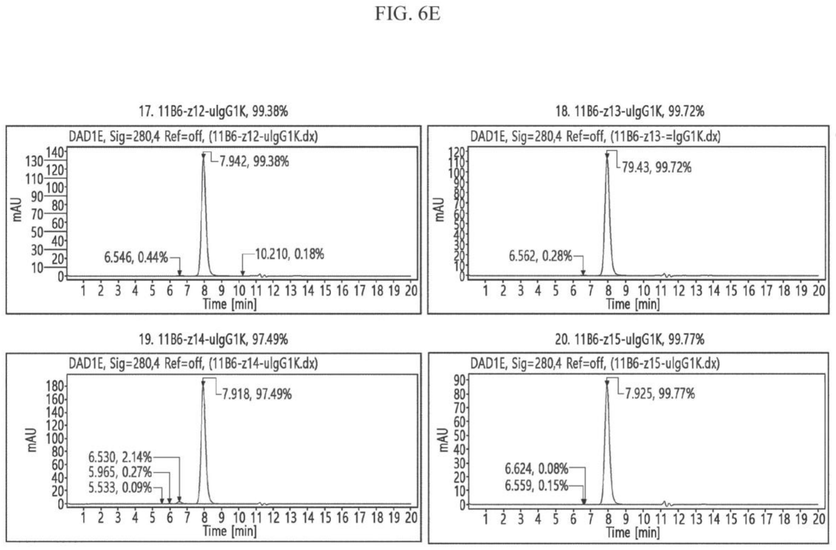

All culture supernatants containing the parent antibody, humanized antibodies, and PTM risk-eliminated variants were further purified using Protein A. Protein concentrations were detected using Nano Drop at 280 nm. Protein purity was assessed by SDS-PAGE and SEC-HPLC. After purification, the antibodies were characterized by SDS-PAGE (FIGS. 3A, 3B, 3C, 4A, and 4B) and SEC-HPLC (FIGS. 5A, 5B, 5C, 5D, 5E, 5F, 6A, 6B, 6C, 6D, and 6E). The antibody migrated on SDS-PAGE under non-reducing conditions to an apparent molecular mass of 150 kDa. The antibodies migrated on SDS-PAGE under reducing conditions to apparent molecular masses of 50 and 25 kDa, which corresponded to the heavy and light chains, respectively.

### 2-2. SPR $k_{off}$ ranking analysis of parent and humanized antibodies binding to MYCT1

SPR $k_{off}$ ranking analysis was performed to determine $k_{off}$ ratios for ranking humanized and PTM risk-eliminated variants. The data was modeled using a 1:1 binding model.

In the 2B4 clone, PTM risk-eliminated mutations were combined with humanized variants z2 and z8 to generate the final lead candidates (2B4-z2-p7-uIgG1K and 2B4-z8-p7-uIgG1K). For further evaluation, the parent antibody and two leads were expressed in 15 mL ExpiCHO cells.

In the 11B6 clone, the "NG" site PTM risk-eliminated variant exhibited excellent binding affinity to MYCT1. Among all humanized variants, z12 exhibited the highest binding affinity to MYCT1. To generate the final lead candidate molecules, humanized variants z10 and z12 and the novel variant z16 were combined with a PTM risk-eliminated mutation (p3) (11B6-z10-p3-uIgG1K, 11B6-z12-p3-uIgG1K, and 11B6-z16-p3-uIgG1K). For further evaluation, the parent antibody and three leads were expressed in 15 mL ExpiCHO cells.

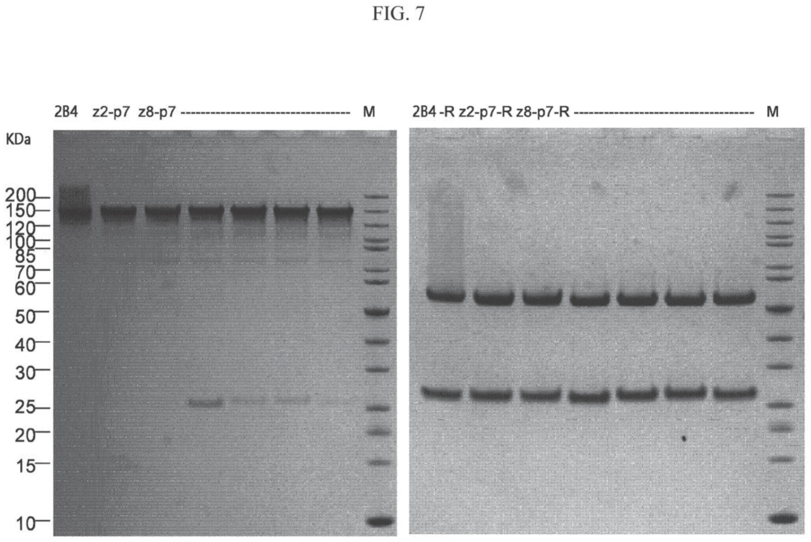

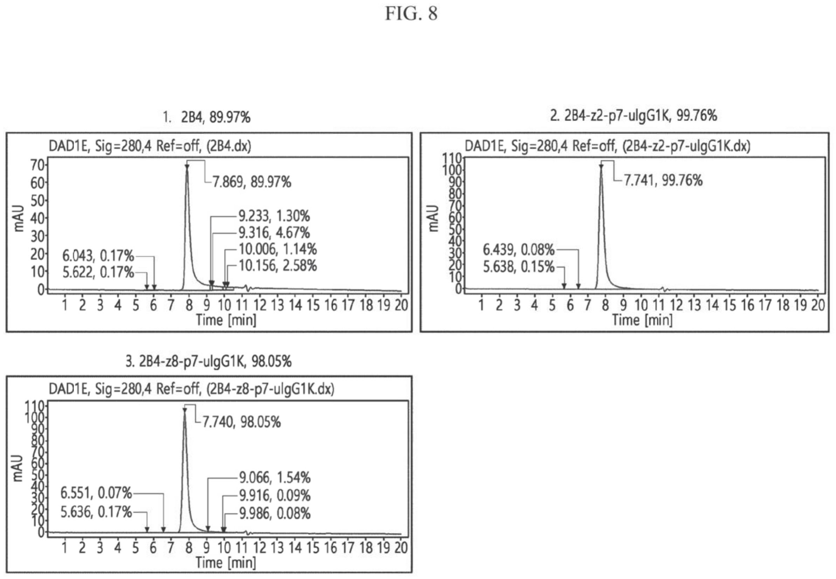

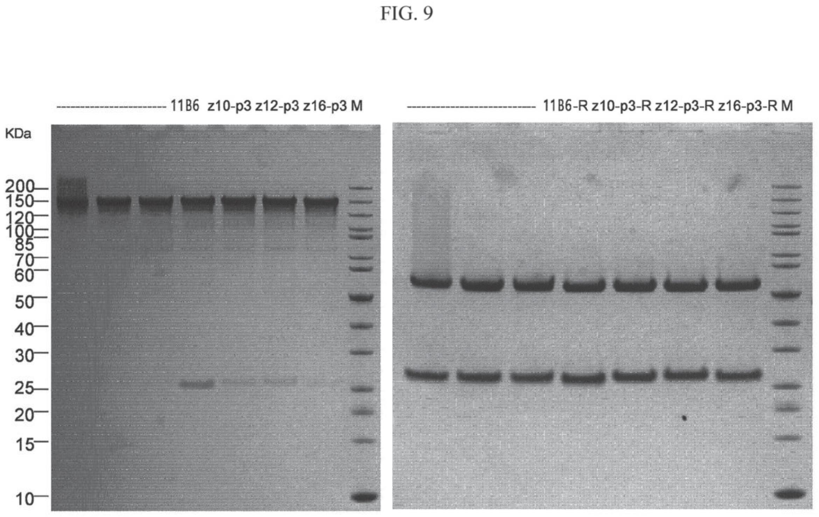

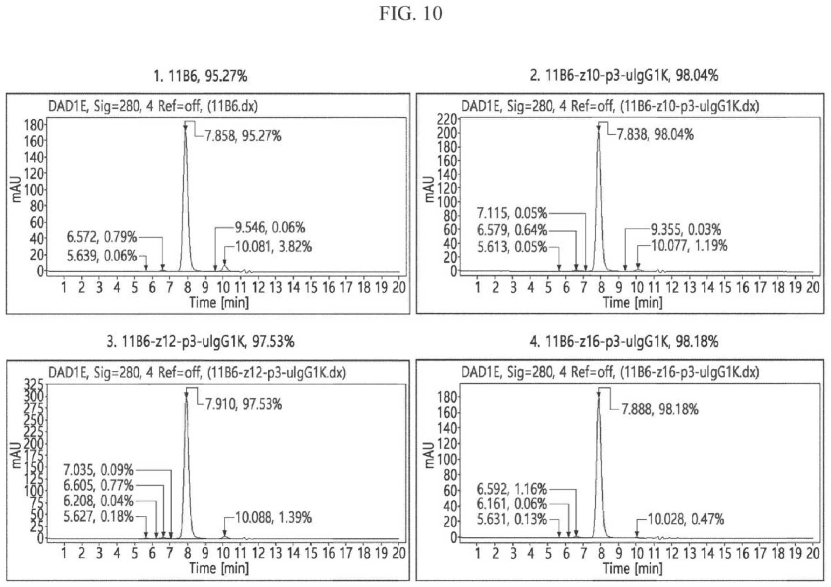

The culture supernatant was further purified using Protein A. Protein concentration was detected at 280 nm using Nano Drop. Protein purity was assessed by SDS-PAGE and SEC-HPLC. After purification, the antibody was characterized by SDS-PAGE and SEC-HPLC (FIGS. 7 to 10). The antibody migrated on SDS-PAGE under non-reducing conditions to an apparent molecular mass of 150 kDa. The antibodies migrated on SDS-PAGE under reducing conditions to apparent molecular masses of 50 and 25 kDa, which corresponded to the heavy and light chains, respectively.

## 2-3. Production of lead materials

Based on the SPR full kinetics, DSF results, and *in vitro* cell assay results, scale-up production of the parent antibody and two leads, 11B6-z12-p3-uIgG1K and 11B6-z16-p3-uIgG1K, was decided. For further evaluation, the parent antibody and two leads were expressed in ExpiCHO cells.

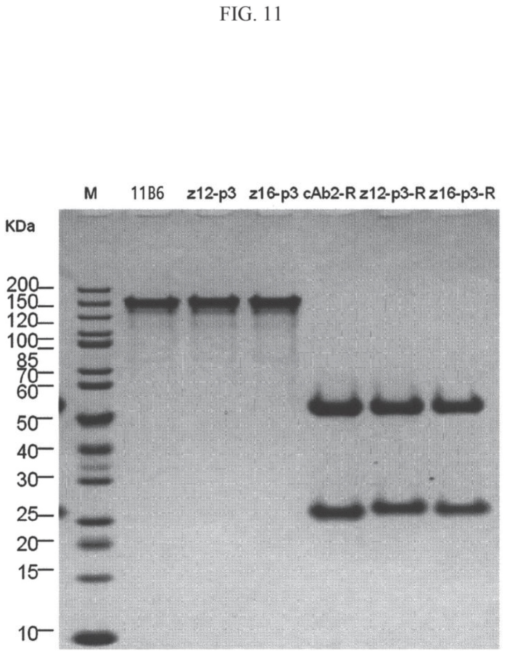

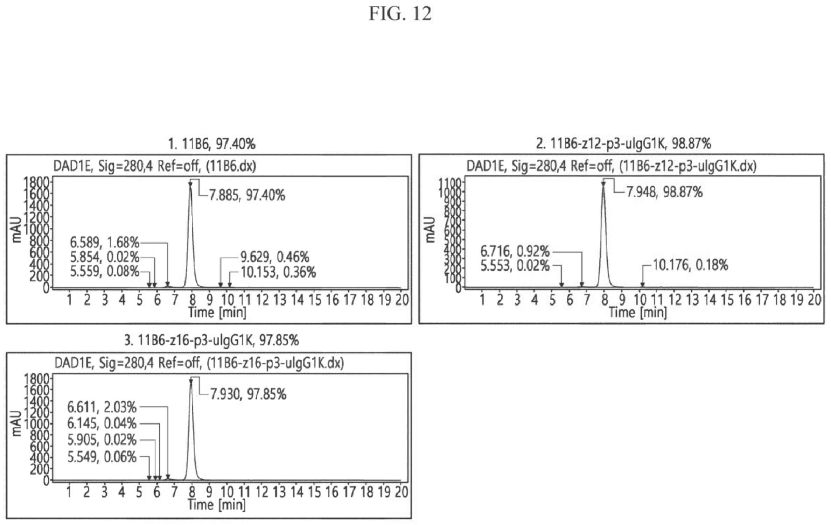

The culture supernatant was further purified using Protein A. Protein concentration was detected at 280 nm using Nano Drop. Protein purity was assessed by SDS-PAGE (FIG. 11) and SEC-HPLC (FIG. 12). After purification, the antibody was characterized by SDS-PAGE and SEC-HPLC. Non-reducing SDS-PAGE showed that the antibody had a molecular mass of 150 kDa. The SDS-PAGE under reducing conditions showed that the antibodies had apparent molecular masses of 50 and 25 kDa, corresponding to the heavy and light chains, respectively.

## 2-4. SPR full kinetics analysis

### 2-4-1. 2B4 clone

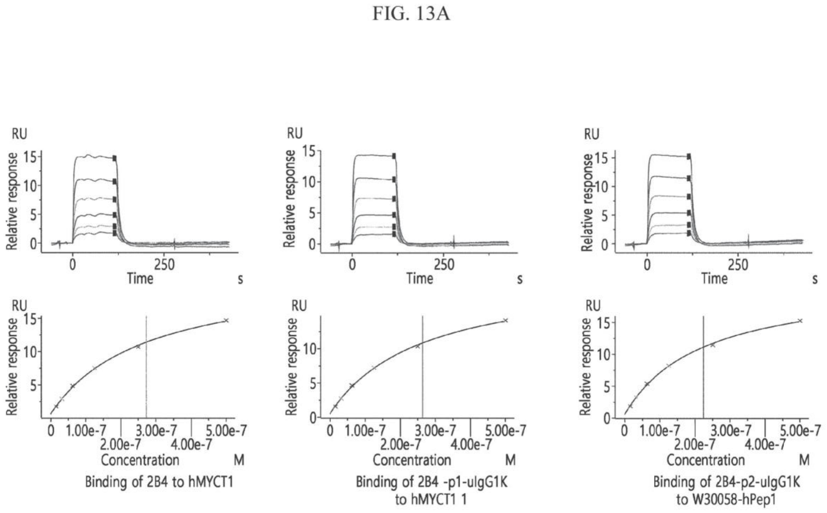

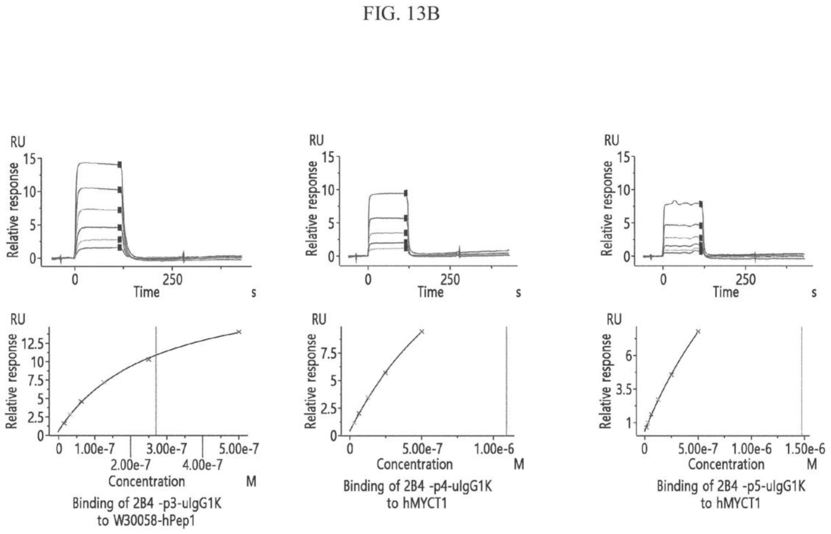

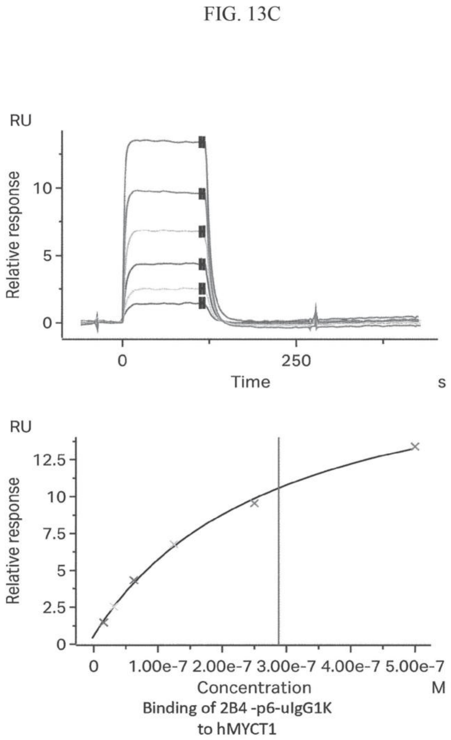

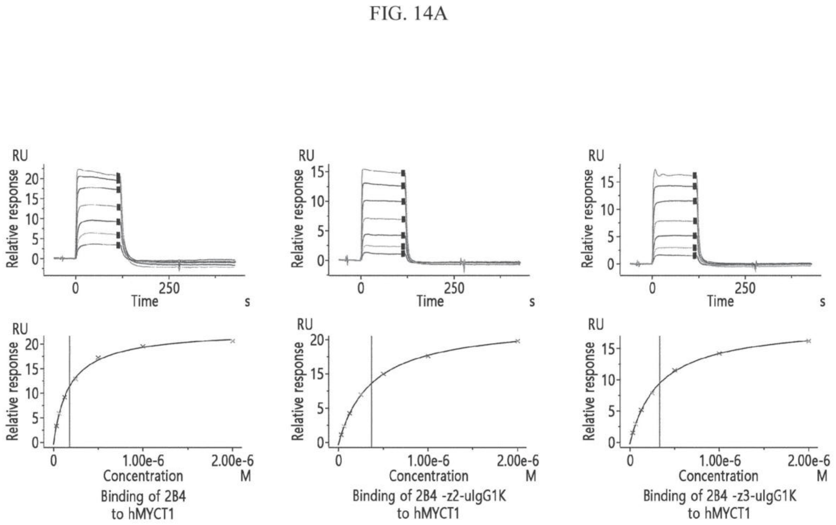

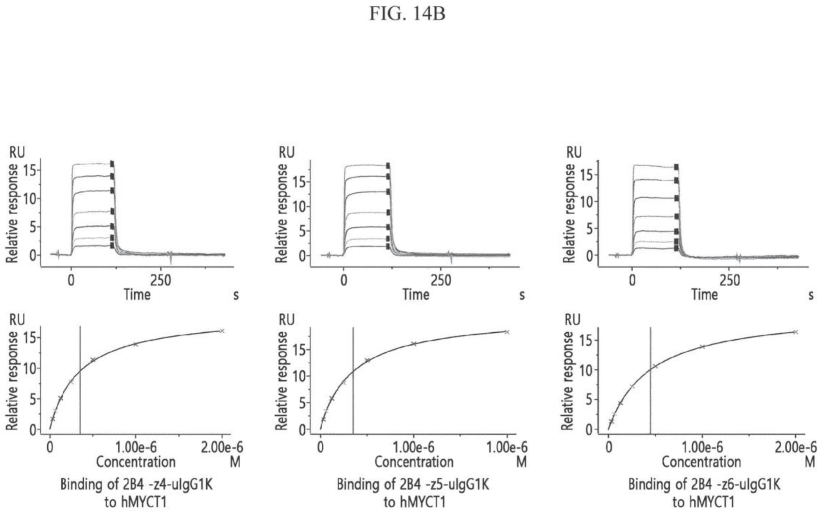

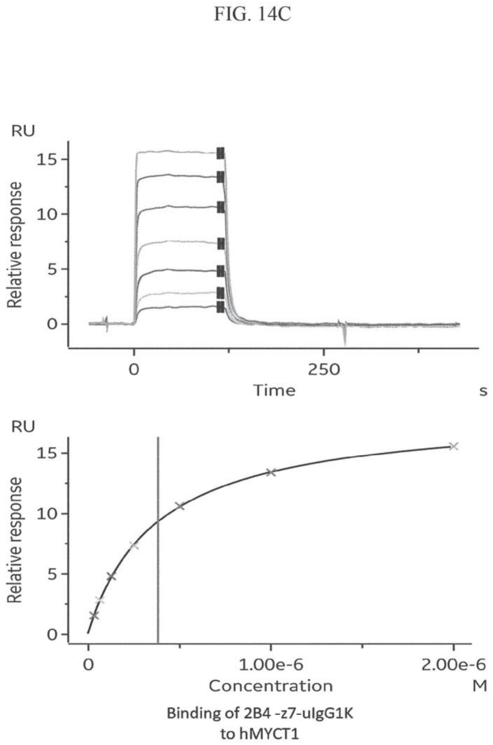

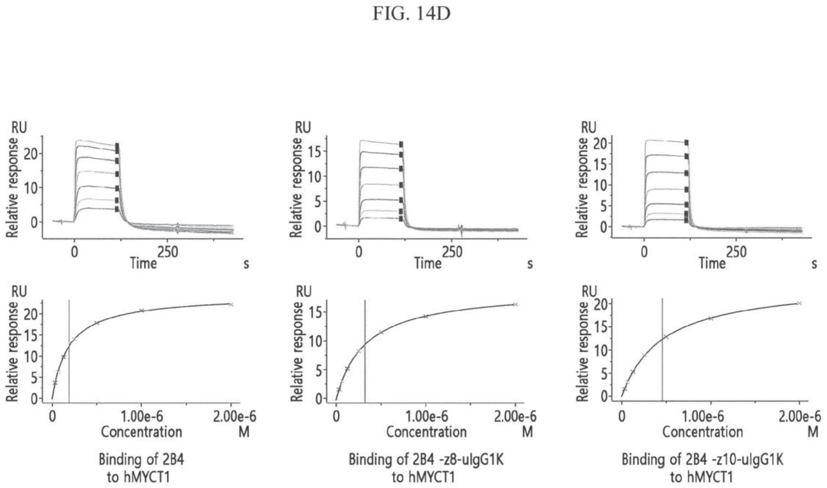

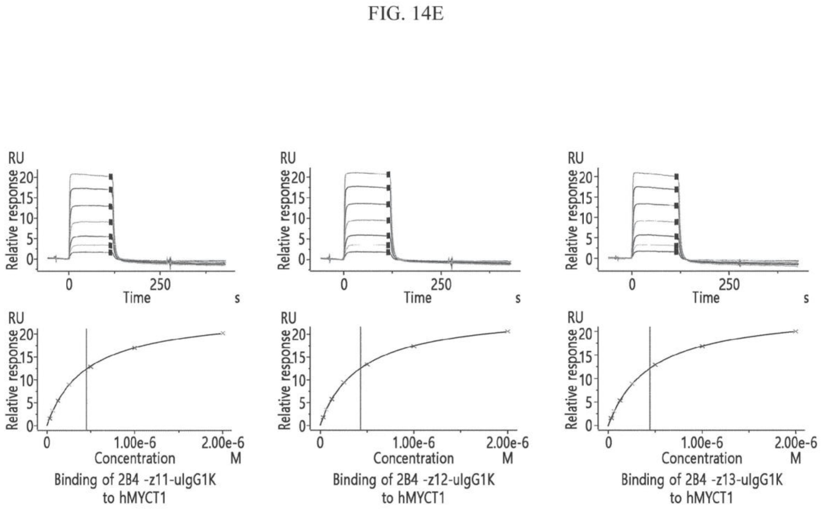

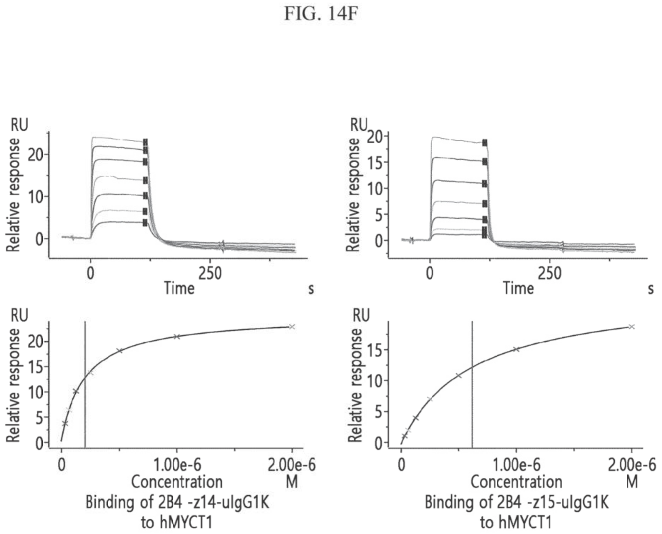

SPR full kinetics analysis was performed to determine binding affinities for ranking humanized and PTM risk-eliminated variants. The data was fitted to a steady-state affinity model (FIGS. 13A, 13B, 13C, 14A, 14B, 14C, 14D, 14E, and 14F and Tables 7 and 8).

**[Table 7]**

| Results of SPR full kinetics analysis for parent antibody (2B4) and PTM risk-eliminated variants for hMYCT1 binding (steady-state affinity model) | | |
|---|---|---|
| **Target** | **Antibody** | **$K_D$ (M)** |
| hMYCT1 | 2B4 | 2.73E-07 |
| | 2B4-p1-uIgG1K | 2.64E-07 |
| | 2B4-p2-uIgG1K | 2.24E-07 |
| | 2B4-p3-uIgG1K | 2.70E-07 |
| | 2B4-p4-uIgG1K | > 5.0E-07* |
| | 2B4-p5-uIgG1K | > 5.0E-07* |
| | 2B4-p6-uIgG1K | 2.88E-07 |

**[Table 8]**

| Results of SPR full kinetics analysis for parent antibody (2B4) and humanized antibodies for hMYCT1 binding (steady-state affinity model) | | |
|---|---|---|
| **Target** | **Antibody** | **$K_D$ (M)** |
| | 2B4 | 1.81E-07 |
| | 2B4-z0-uIgG1K | weak binding |
| | 2B4-z1-uIgG1K | weak binding |
| | 2B4-z2-uIgG1K | 3.68E-07 |
| | 2B4-z3-uIgG1K | 3.32E-07 |
| | 2B4-z4-uIgG1K | 3.54E-07 |

(continued)

| Results of SPR full kinetics analysis for parent antibody (2B4) and humanized antibodies for hMYCT1 binding (steady-state affinity model) | | |
|---|---|---|
| **Target** | **Antibody** | **$K_D$ (M)** |
| hMYCT1 | 2B4-z5-uIgG1K | 3.51E-07 |
| | 2B4-z6-uIgG1K | 4.47E-07 |
| | 2B4-z7-uIgG1K | 3.82E-07 |
| | 2B4-z8-uIgG1K | 3.21E-07 |
| | 2B4-z9-uIgG1K | weak binding |
| | 2B4-z10-uIgG1K | 4.56E-07 |
| | 2B4-z11-uIgG1K | 4.49E-07 |
| | 2B4-z12-uIgG1K | 4.28E-07 |
| | 2B4-z13-uIgG1K | 4.43E-07 |
| | 2B4-z14-uIgG1K | 2.03E-07 |
| | 2B4-z15-uIgG1K | 6.17E-07 |

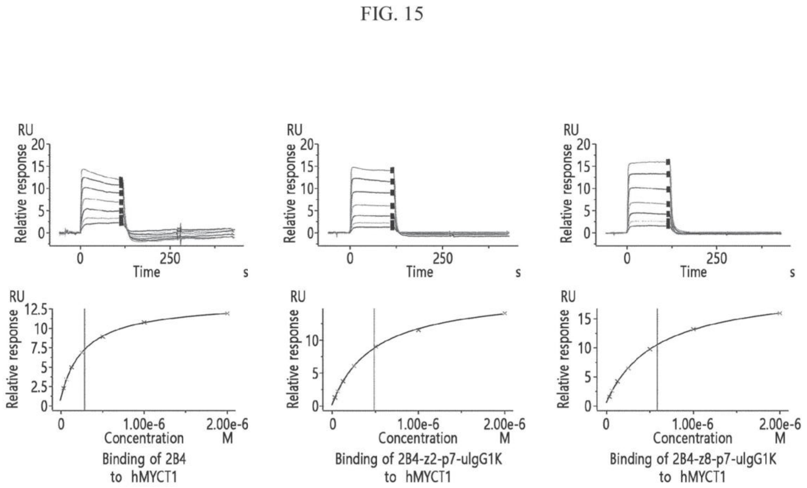

SPR $k_{off}$ ranking analysis was performed. For the humanized variants, compared to the parent antibody (KD = 0.181 mM), z2 exhibited similar binding affinity to the parent antibody, while z8 exhibited the highest binding affinity for MYCT1 among all humanized variants of the 2b4 clone. To produce the final lead molecule, humanized variants z2 and z8 were combined with the PTM risk-eliminated variant (p7: pl+p6).
SPR full kinetics analysis was performed to determine the binding affinity of the parent antibody and the humanized and PTM risk-eliminated combined lead molecules (z2-p7 and z8-p7) for hMYCT1. The data was fitted by a steady-state affinity model (FIG. 15 and Table 9).
Compared to the parent antibody (KD=0.281 mM), the two leads exhibited similar binding affinities ($K_D$ ranging from 0.484 to 0.586 mM) to the antigen.

**[Table 9]**

| Results of SPR full kinetics analysis of parent antibody (2B4) and lead molecules (z2-p7 and z8-p7) for hMYCT1 binding (steady-state affinity model) | | |
|---|---|---|
| **Target** | **Antibody** | **$K_D$ (M)** |
| hMYCT1 | 2B4 | 2.81E-07 |
| | 2B4-z2-p7-uIgG1K | 4.84E-07 |
| | 2B4-z8-p7-uIgG1K | 5.86E-07 |

**2-4-2. 11B6 clone**

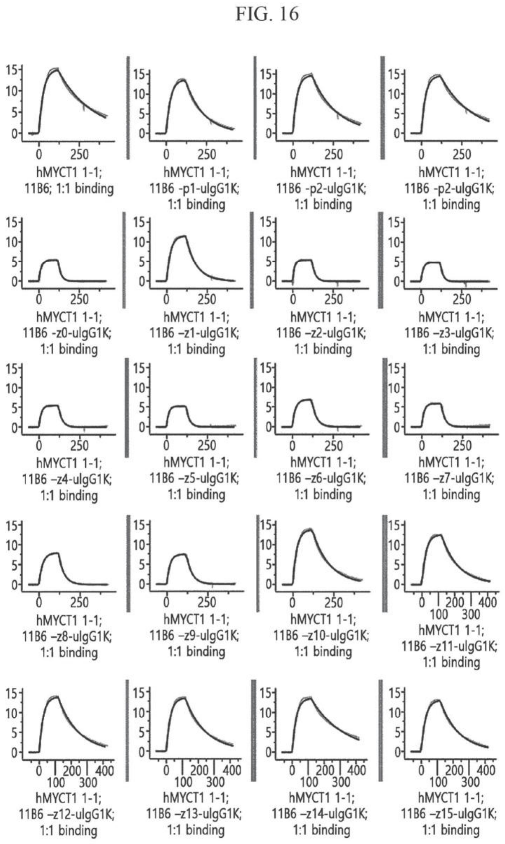

SPR $k_{off}$ ranking analysis was performed. The data was fitted to a 1:1 binding model (FIG. 16 and Table 10).

**[Table 10]**

| Single-curve SPR binding results for the parent antibody (11B6), humanized antibodies, and PTM risk-eliminated variants for hMYCT1 (1:1 binding model) | | |
|---|---|---|
| **Analyte** | **Ligand** | **$k_d$ (1/s)** |
| | 11B6 | 4.96E-03 |
| | 11B6-p1-uIgG1K | 9.33E-03 |
| | 11B6-p2-uIgG1K | 6.91E-03 |
| | 11B6-p3-uIgG1K | 5.47E-03 |

(continued)

| Single-curve SPR binding results for the parent antibody (11B6), humanized antibodies, and PTM risk-eliminated variants for hMYCT1 (1:1 binding model) | | |
|---|---|---|
| **Analyte** | **Ligand** | **$k_d$ (1/s)** |
| hMYCT1 | 11B6-z0-uIgG1K | 6.24E-02 |
| | 11B6-z1-uIgG1K | 1.69E-02 |
| | 11B6-z2-uIgG1K | 5.60E-02 |
| | 11B6-z3-uIgG1K | 6.59E-02 |
| | 11B6-z4-uIgG1K | 4.76E-02 |
| | 11B6-z5-uIgG1K | 5.21E-02 |
| | 11B6-z6-uIgG1K | 3.77E-02 |
| | 11B6-z7-uIgG1K | 4.73E-02 |
| | 11B6-z8-uIgG1K | 3.24E-02 |
| | 11B6-z9-uIgG1K | 3.11E-02 |
| | 11B6-z10-uIgG1K | 1.02E-02 |
| | 11B6-z11-uIgG1K | 1.02E-02 |
| | 11B6-z12-uIgG1K | 8.38E-03 |
| | 11B6-z13-uIgG1K | 8.38E-03 |
| | 11B6-z14-uIgG1K | 5.20E-03 |
| | 11B6-z15-uIgG1K | 9.06E-03 |

For the humanized variants, compared to the parent antibody ($k_d$ = 4.56E-03 1/s), z10 of the 11B6 clone exhibited similar affinity to the parent antibody. Among all humanized variants, z12 exhibited the highest binding affinity for MYCT1.

To produce the final lead molecule, humanized variants z10 and z12 and the novel z16 variant were combined with a PTM risk-eliminated variant (p3).

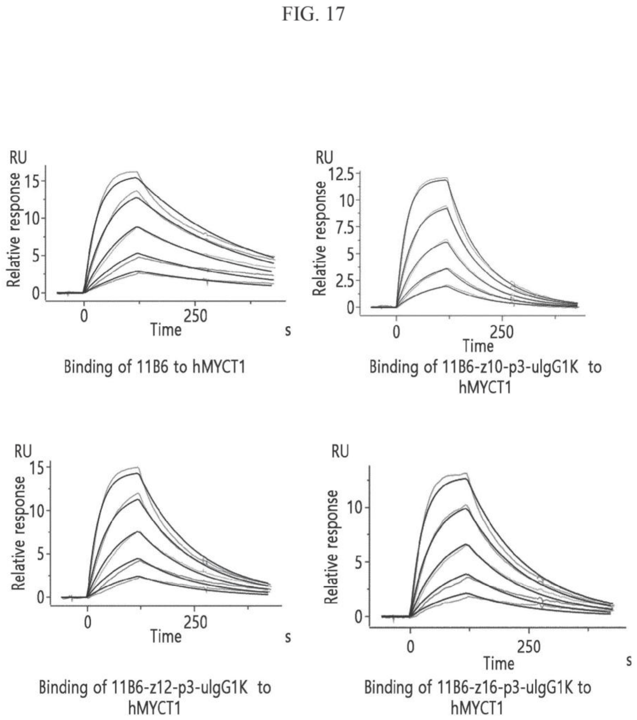

SPR full kinetics analysis was performed to determine the binding affinities of the parent antibody and the humanized and PTM risk-eliminated combined lead antibodies (z10-p3, z12-p3, and z16-p3) for MYCT1 (FIG. 17 and Table 11). The data was confirmed by a 1:1 binding model. Compared to the parent antibody (KD=1.20 nM), the three leads exhibited similar binding affinities (KD ranging from 2.35 to 3.34 nM) to the antigen.

**[Table 11]**

| Results of SPR full kinetics analysis of parent antibody (11B6) and lead antibodies (z10-p3, z12-p3, and z16-p3) for HMYCT1 (1:1 binding model) | | | | |
|---|---|---|---|---|
| **Target** | **Ligand** | **$k_a$ (1/Ms)** | **$k_d$ (1/s)** | **$K_D$ (M)** |
| hMYCT1 | 11B6 | 3.44E+06 | 4.11E-03 | 1.20E-09 |
| | 11B6-z10-p3-uIgG1K | 3.65E+06 | 1.22E-02 | 3.34E-09 |
| | 11B6-z12-p3-uIgG1K | 3.35E+06 | 7.86E-03 | 2.35E-09 |
| | 11B6-z16-p3-uIgG1K | 3.32E+06 | 8.41E-03 | 2.53E-09 |

## 2-5. Thermal stability evaluated by DSF

### 2-5-1. 2B4 clone

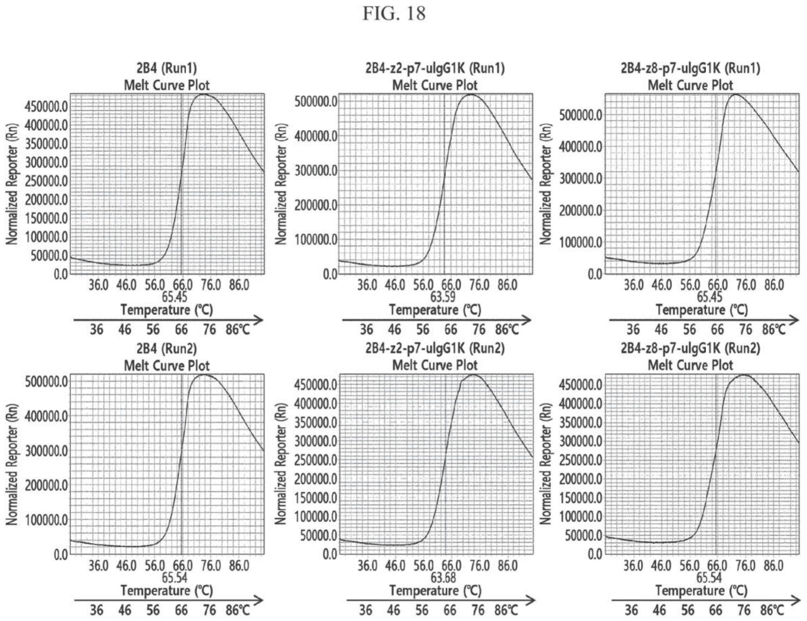

The Tm of the parent antibody and two lead molecules was investigated using the DSF test (FIG. 18 and Table 12). The Tm1 of z8-p7 was similar to that of the parent antibody. The Tm1 of z2-p7 was 2 degrees lower than that of the parent antibody.

**[Table 12]**

| Tm (2B4) confirmed by DSF | | | | | |
|---|---|---|---|---|---|
| **Name of protein** | **Conc. (mg/ml)** | **Buffer** | **Run** | **Tm1 (°C)** | **Mean Tm1 (°C)** |
| hMYCT1 | 1.0 | PBS | Run 1 | 65.5 | 65.5 |
| | | | Run 2 | 65.5 | |
| 2B4-z2-p7-uIgG1K | 1.0 | PBS | Run 1 | 63.6 | 63.7 |
| | | | Run 2 | 63.7 | |
| 2B4-z8-p7-uIgG1K | 1.0 | PBS | Run 1 | 65.5 | 65.5 |
| | | | Run 2 | 65.5 | |

**2-5-2. 11B6 clone**

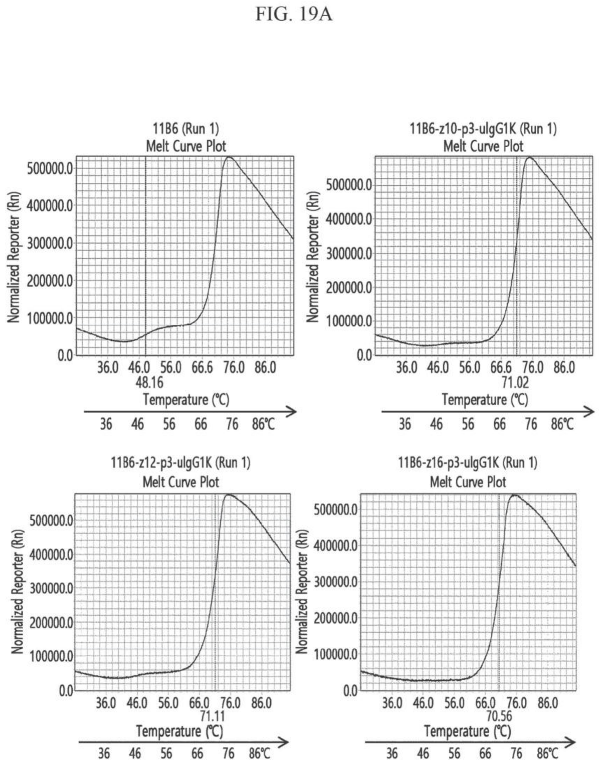

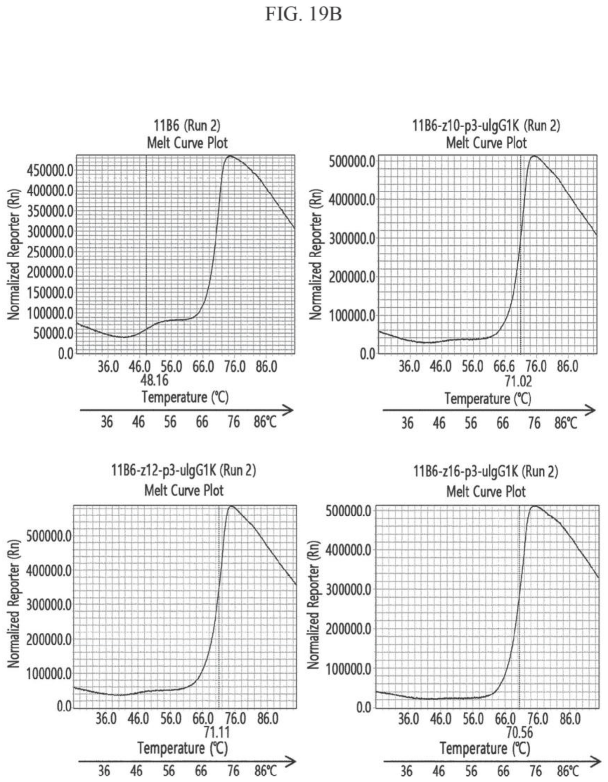

The Tm of the parent antibody and three lead molecules was investigated using the DSF test (FIGS. 19A and 19B and Table 13). The lead molecules exhibited a higher Tm1 than the parent antibody, indicating that the humanized antibody exhibits better thermal stability.

**[Table 13]**

| **Name of protein** | **Conc. (mg/ml)** | **Buffer** | **Run** | **Tm1 (°C)** | **Mean Tm1 (°C)** |
|---|---|---|---|---|---|
| 11B6 | 1.0 | PBS | Run 1 | 48.2 | 48.2 |
| | | | Run 2 | 48.2 | |
| 11B6-z10-p3-uIgG1K | 1.0 | PBS | Run 1 | 71.0 | 71.0 |
| | | | Run 2 | 71.0 | |
| 11B6-z12-p3-uIgG1K | 1.0 | PBS | Run 1 | 71.1 | 71.1 |
| | | | Run 2 | 71.1 | |
| 11B6-z16-p3-uIgG1K | 1.0 | PBS | Run 1 | 70.6 | 70.6 |
| | | | Run 2 | 70.6 | |

## Example 3. Antibody toxicity evaluation

NCI-H460 cells (lung cancer), HppG2 (liver cancer), MC38 (colon cancer), and ACHN (renal cells) were purchased from ATCC. Each cell line was plated at a density of $1 \times 10^4$ cells/well into a 96-well plate and cultured for 12 hours. After treating with various concentrations of antibodies, the cells were cultured for 24 hours. The cells were then treated with 3-(4,5-dimethylthiaol-2-yl)-2,5-diphenyltetrazolium bromide (MTT) and cultured at 37 °C for two to four hours. To determine cell viability, the absorbance at 570 nm was measured using a microplate reader (BioTek, Winooski, VT, USA).

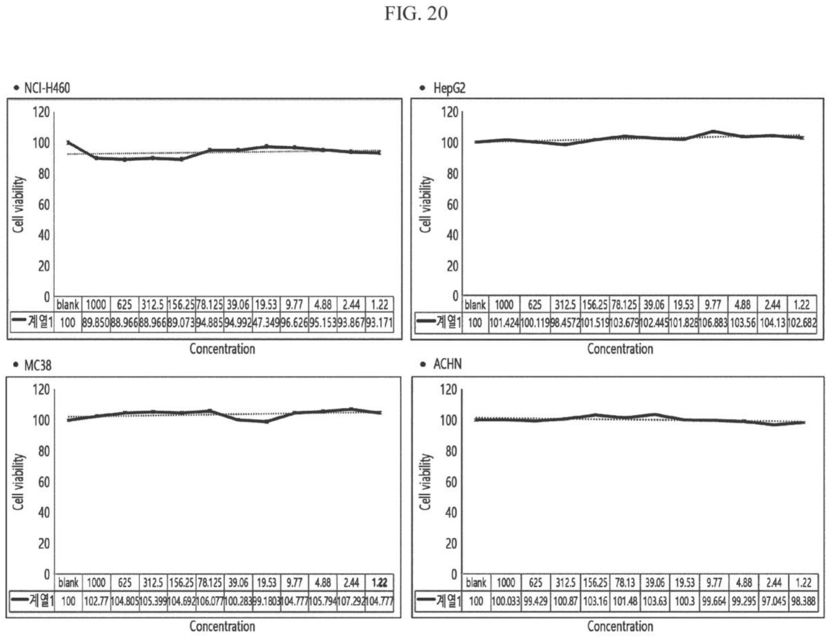

The results confirmed that ATN001 (11B4-z16-p3-uIgG1K) had no toxicity from the highest concentration of 1 mM to the lowest concentration of 1 $\mu$M through serial dilution (FIG. 20).

## Example 4. Tube formation assay

Human umbilical vein endothelial cells (HUVECs) were starved for six hours by replacing the medium with M199 medium containing 1% fetal bovine serum (FBS). 250 $\mu$L of ice-cold Matrigel (BD Biosciences) was added to each well of a 24-well plate and allowed to solidify at 37 °C for 30 minutes. After starvation, the cell density was adjusted to $1 \times 10^5$ cells/well and 1% FBS M199 medium containing antibodies (anti-human VEGF Ab (Avastin), m2B4, m11B6, 11B6-z12-p3, 11B6-z16-p3) was added. After culturing for 12 hours, tube formation was observed under an optical microscope ($\times 100$).

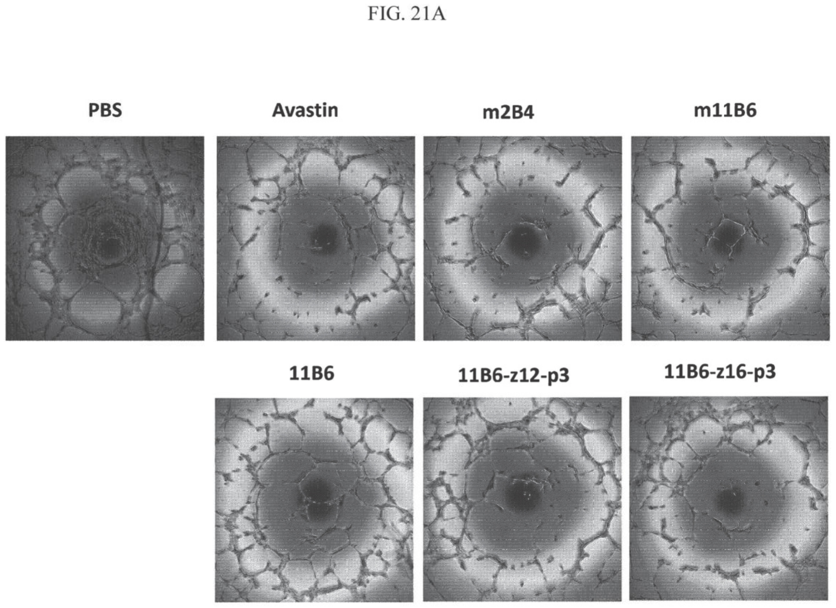

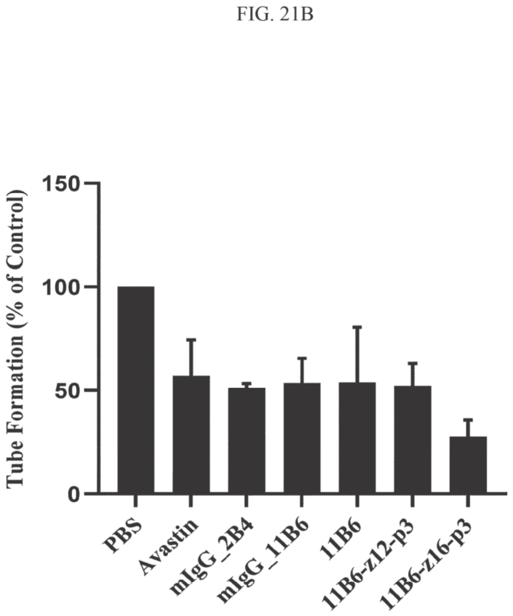

As a result, compared to the control group, treatment with 20 $\mu$M of the antibody of the present invention demonstrated efficacy greater than or equivalent to that of the control group (FIGS. 21A and 21B).

## Example 5. Antitumor effect

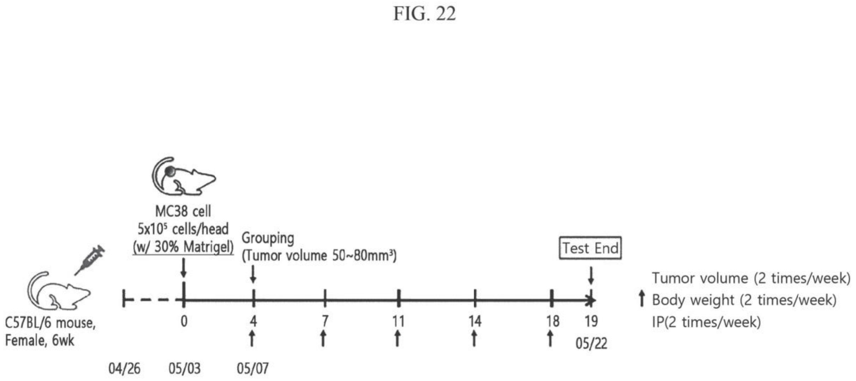

An experiment was conducted under the conditions shown in FIG. 22 and Table 14 below. Specifically, the 11B6-z16-p3 (hzATN001) antibody was administered to mice with colon cancer on days 4, 7, 11, and 18, and tumors were excised on day 19. The antitumor effect of 11B6-z16-p3 (hzATN001) was confirmed, and the tumor growth inhibition rate is shown in Table 15.

[Table 14]

| Group | Disease-causing substance | | | | Test substance | | | |
|---|---|---|---|---|---|---|---|---|
| | Cancer cell | Administration route | Administration amount | Dose | Test substance | Administration route | Administration amount (mg/kg) | Dose (mL/kg) |
| G1 (n=8) | MC38 | SC | 5x10$^5$ cell/ head | 100 μL/head | PBS | IP | - | 10 |
| G2 (n=8) | | | | | 11B6-z16-p3 | IP | 5 | 10 |
| G3 (n=8) | | | | | 11B6-z16-p3 | IP | 10 | 10 |
| G4 (n=8) | | | | | 11B6-z16-p3 | IP | 15 | 10 |
| G5 (n=8) | | | | | 11B6-z16-p3 | IP | 20 | 10 |

EP 4 737 482 A1

[Table 15]

| Tumor growth inhibition rate (%) | | |
|---|---|---|
| **Tumor growth inhibition rate (%)** | **18 days** | |
| G1 PBS | 2476.55 | 0.00 |
| G2 11B6-z16-p3 5mpk | 2473.55 | -0.12 |
| G3 11B6-z16-p3 10mpk | 2075.25 | -16.20 |
| G4 11B6-z16-p3 15mpk | 1443.13 | -41.73 |
| G5 11B6-z16-p3 20mpk | 1542.51 | -37.72 |

As can be seen from Table 15 above, the anti-MYCT1 antibody of the present invention has excellent antitumor effects.

**Example 6. Tumor growth inhibition in a syngeneic mouse model (CT26)**

To study the *in vivo* efficacy of the antibody, the antitumor efficacy was determined using the CT26 colon cancer cell line. The CT26 cell line (ATCC, CRL-2638) was cultured at 37 °C in 5% $CO_2$ using modified RPMI1640 medium containing 10% FBS and 1x antibiotic-antimycotic. For cell transplantation, CT26 cells were diluted in Dulbecco's phosphate-buffered saline (DPBS) to a concentration of $5 \times 10^6$ cells/mL and transplanted subcutaneously into the flank of each mouse with 100 μL ($5 \times 10^5$ cells). Tumor volume after CT26 cell transplantation was calculated using the formula shown below. Antibodies 11B6 (chATN001) and 11B6-Z16-p3 (hzATN001) and anti-PD-1 antibody ((anti-Mouse CD279 (PD-1) (Clone RMP1-14) - purified *in vivo* PLATINUM™ functional grade) were administered to mice under the conditions shown in Table 16.

$$\text{Tumor volume (mm}^3) = [\text{length (mm)} \times \text{width (mm)}^2] \times 0.5$$

In addition, mice were divided into groups by measuring body weight and tumor size. Tumor growth rate was determined using the following formula using tumor size at the time of group separation and tumor size on the measurement date.

Tumor growth rate (%) = (tumor size on measurement day-tumor size at group separation)/tumor size at group separation$\times$100

[Table 16]

| Group | Construct | Mouse No. | Concentration | Administration schedule | Route |
|---|---|---|---|---|---|
| 1 | Vehicle (PBS) | 12 | | | IP |
| 2 | anti-PD-1 antibody (12.1 mg/mL) | 12 | 10 mpk | 2/wk | IP |
| 3 | 11B6 (4.04 mg/mL) | 12 | 10 mpk | 3/wk | IP |
| 4 | 11B6-Z16-p3 (3.87 mg/mL) | 12 | 10 mpk | 3/wk | IP |
| 5 | 11B6 & anti-PD-1 | 12 | 10 mpk/10mpk | 3/wk/2/wk | IP/IP |
| 6 | 11B6-Z16-p3 & anti-PD-1 | 12 | 10 mpk/10mpk | 3/wk/2/wk | IP/IP |

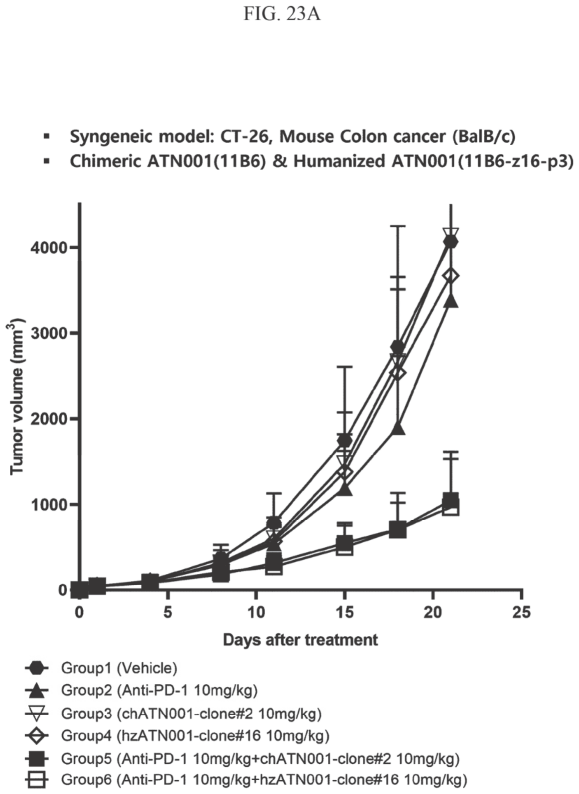

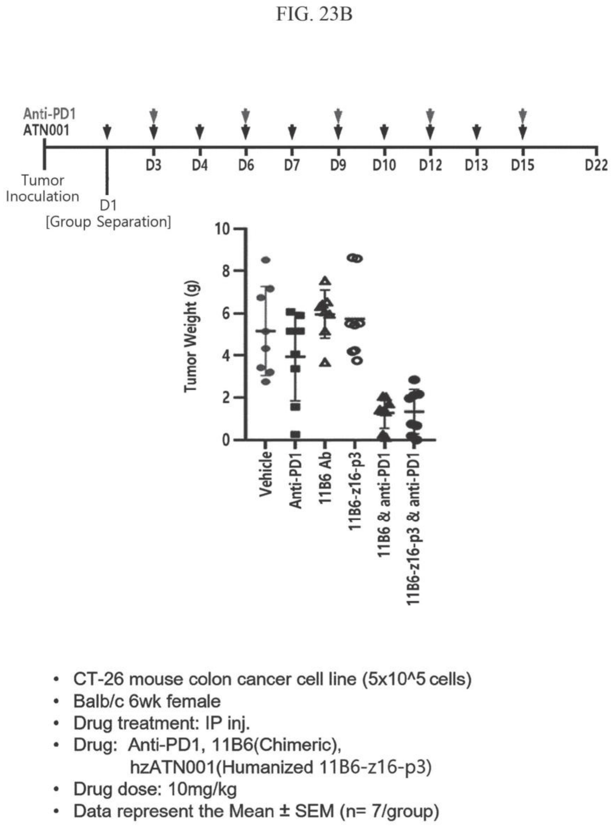

The experimental results showed that when 11B6-Z16-p3 (hzATN001) was co-administered with anti-PD-1 antibody, the tumor volume was reduced by 77.1% and the tumor weight was also significantly reduced (FIGS. 23A and 23B).

Although the above-described embodiments have been described with limited drawings, those skilled in the art will appreciate that various technical modifications and variations may be applied based on the above description. For example, appropriate results may still be achieved when the described techniques are performed in an order different from the described method, and/or the components of the described systems, structures, devices, circuits, and the like are coupled or combined in a manner different from the described method, or are replaced or substituted with other components or equivalents.

Therefore, other implementations, other embodiments, and equivalents to the claims are also within the scope of the claims described below.

The present invention was completed with support from the Korea Technology and Information Promotion Agency for SMEs and the Tech Incubator Program for Startups (TIPS) funded by the Ministry of SMEs and Startups of the Republic of Korea (Project Number: 00238993).

[Industrial Applicability]

The present invention relates to an antibody or antigen-binding fragment that specifically binds to MYCT1, and exhibits excellent affinity and binding strength for MYCT1. Therefore, the antibody or antigen-binding fragment of the present invention that specifically binds to MYCT1 can be developed into an antibody-drug conjugate, a bispecific antibody, or a chimeric antigen receptor (CAR). Furthermore, the antibody or antigen-binding fragment according to the present invention can be effectively used in the treatment or diagnosis of MYCT1-associated diseases, such as cancer or angiogenesis-associated diseases. Furthermore, the antibody or antigen-binding fragment according to the present invention can be used in combination with immunotherapy and can enhance sensitivity to the immunotherapy agent.

**Claims**

1. An isolated humanized anti-MYCT1 antibody or antigen-binding fragment thereof comprising: at least one variable heavy chain region (VH) selected from the group consisting of SEQ ID NOs: 41 and 43 to 60; and at least one variable light chain region (VL) selected from the group consisting of SEQ ID NOs: 42 and 61 to 66; or
at least one VH selected from the group consisting of SEQ ID NOs: 92 and 94 to 101; and at least one VL selected from the group consisting of SEQ ID NOs: 93 and 102 to 115.

2. The antibody or antigen-binding fragment thereof of claim 1, wherein the VH includes CDR1, CDR2, and CDR3 having amino acid sequences set forth in SEQ ID NOs: 3, 4, and 5, respectively, and the VL includes CDR1, CDR2, and CDR3 having amino acid sequences set forth in SEQ ID NOs: 6, 7, and 8, respectively.

3. The antibody or antigen-binding fragment thereof of claim 1, wherein the VH includes CDR1, CDR2, and CDR3 having amino acid sequences set forth in SEQ ID NOs: 9, 10, and 11, respectively, and the VL includes CDR1, CDR2, and CDR3 having amino acid sequences set forth in SEQ ID NOs: 12, 13, and 14, respectively.

4. The antibody or antigen-binding fragment thereof of claim 1, comprising a VH sequence of SEQ ID NO: 43 and a VL sequence of SEQ ID NO: 42;

   comprising a VH sequence of SEQ ID NO: 44 and a VL sequence of SEQ ID NO: 42;
   comprising a VH sequence of SEQ ID NO: 45 and a VL sequence of SEQ ID NO: 42;
   comprising a VH sequence of SEQ ID NO: 41 and a VL sequence of SEQ ID NO: 61;
   comprising a VH sequence of SEQ ID NO: 46 and a VL sequence of SEQ ID NO: 62;
   comprising a VH sequence of SEQ ID NO: 41 and a VL sequence of SEQ ID NO: 63;
   comprising a VH sequence of SEQ ID NO: 44 and a VL sequence of SEQ ID NO: 63;
   comprising a VH sequence of SEQ ID NO: 47 and a VL sequence of SEQ ID NO: 64;
   comprising a VH sequence of SEQ ID NO: 48 and a VL sequence of SEQ ID NO: 64;
   comprising a VH sequence of SEQ ID NO: 48 and a VL sequence of SEQ ID NO: 65;
   comprising a VH sequence of SEQ ID NO: 49 and a VL sequence of SEQ ID NO: 65;
   comprising a VH sequence of SEQ ID NO: 50 and a VL sequence of SEQ ID NO: 65;
   comprising a VH sequence of SEQ ID NO: 51 and a VL sequence of SEQ ID NO: 65;
   comprising a VH sequence of SEQ ID NO: 52 and a VL sequence of SEQ ID NO: 65;
   comprising a VH sequence of SEQ ID NO: 53 and a VL sequence of SEQ ID NO: 65;
   comprising a VH sequence of SEQ ID NO: 54 and a VL sequence of SEQ ID NO: 65;
   comprising a VH sequence of SEQ ID NO: 55 and a VL sequence of SEQ ID NO: 64;
   comprising a VH sequence of SEQ ID NO: 55 and a VL sequence of SEQ ID NO: 65;
   comprising a VH sequence of SEQ ID NO: 56 and a VL sequence of SEQ ID NO: 65;
   comprising a VH sequence of SEQ ID NO: 57 and a VL sequence of SEQ ID NO: 65;
   comprising a VH sequence of SEQ ID NO: 58 and a VL sequence of SEQ ID NO: 65;
   comprising a VH sequence of SEQ ID NO: 41 and a VL sequence of SEQ ID NO: 65;

comprising a VH sequence of SEQ ID NO: 58 and a VL sequence of SEQ ID NO: 42;
comprising a VH sequence of SEQ ID NO: 59 and a VL sequence of SEQ ID NO: 66; or
comprising a VH sequence of SEQ ID NO: 60 and a VL sequence of SEQ ID NO: 66.

5. A humanized anti-MYCT1 antibody or antigen-binding fragment thereof, comprising a VH sequence of SEQ ID NO: 92 and a VL sequence of SEQ ID NO: 102;

comprising a VH sequence of SEQ ID NO: 92 and a VL sequence of SEQ ID NO: 103;
comprising a VH sequence of SEQ ID NO: 92 and a VL sequence of SEQ ID NO: 104;
comprising a VH sequence of SEQ ID NO: 94 and a VL sequence of SEQ ID NO: 105;
comprising a VH sequence of SEQ ID NO: 95 and a VL sequence of SEQ ID NO: 105;
comprising a VH sequence of SEQ ID NO: 96 and a VL sequence of SEQ ID NO: 105;
comprising a VH sequence of SEQ ID NO: 97 and a VL sequence of SEQ ID NO: 105;
comprising a VH sequence of SEQ ID NO: 98 and a VL sequence of SEQ ID NO: 105;
comprising a VH sequence of SEQ ID NO: 99 and a VL sequence of SEQ ID NO: 106;
comprising a VH sequence of SEQ ID NO: 99 and a VL sequence of SEQ ID NO: 107;
comprising a VH sequence of SEQ ID NO: 99 and a VL sequence of SEQ ID NO: 108;
comprising a VH sequence of SEQ ID NO: 99 and a VL sequence of SEQ ID NO: 109;
comprising a VH sequence of SEQ ID NO: 99 and a VL sequence of SEQ ID NO: 110;
comprising a VH sequence of SEQ ID NO: 95 and a VL sequence of SEQ ID NO: 110;
comprising a VH sequence of SEQ ID NO: 100 and a VL sequence of SEQ ID NO: 110;
comprising a VH sequence of SEQ ID NO: 100 and a VL sequence of SEQ ID NO: 111;
comprising a VH sequence of SEQ ID NO: 101 and a VL sequence of SEQ ID NO: 111;
comprising a VH sequence of SEQ ID NO: 92 and a VL sequence of SEQ ID NO: 111;
comprising a VH sequence of SEQ ID NO: 101 and a VL sequence of SEQ ID NO: 112;
comprising a VH sequence of SEQ ID NO: 95 and a VL sequence of SEQ ID NO: 113;
comprising a VH sequence of SEQ ID NO: 100 and a VL sequence of SEQ ID NO: 114; or
comprising a VH sequence of SEQ ID NO: 95 and a VL sequence of SEQ ID NO: 115.

6. The humanized anti-MYCT1 antibody or antigen-binding fragment thereof of claim 1, wherein the antigen binding fragment includes Fd, Fab, Fab', F(ab')2, dsFv, scFv, and a single domain antibody (sdAb).

7. A nucleic acid molecule encoding the antibody or antigen-binding fragment thereof of any one of claims 1 to 6.

8. A recombinant expression vector comprising the nucleic acid molecule of claim 7.

9. A host cell transfected with the expression vector of claim 8.

10. An immunoconjugate comprising the antibody or antigen-binding fragment of any one of claims 1 to 6 and a cytotoxic agent.

11. A pharmaceutical composition for preventing or treating an MYCT1-associated disease, comprising the antibody or antigen-binding fragment of any one of claims 1 to 6 as an active ingredient.

12. The pharmaceutical composition of claim 11, wherein the MYCT1-associated disease is an angiogenesis-associated disease or cancer.

13. The pharmaceutical composition of claim 12, wherein the pharmaceutical composition is administered in combination with at least one additional therapeutic agent or therapeutic procedure.

14. The pharmaceutical composition of claim 13, wherein the at least one additional therapeutic agent or therapeutic procedure is selected from one or more of chemotherapy, targeted anticancer therapy, an oncolytic drug, a cytotoxic agent, immune-based therapy, a cytokine, a surgical procedure, a radiation procedure, a vaccine, and cell therapy.

15. A composition for diagnosing an MYCT1-associated disease, comprising the antibody or antigen-binding fragment of any one of claims 1 to 6 as an active ingredient.

16. A pharmaceutical composition for enhancing sensitivity to an immunotherapy agent in cancer, comprising the antibody or antigen-binding fragment of any one of claims 1 to 6 as an active ingredient.

17. A chimeric antigen receptor (CAR) comprising an antigen-binding domain that specifically binds to MYCT1, wherein the antigen-binding domain includes an antigen-binding fragment including: at least one variable heavy chain (VH) region selected from the group consisting of SEQ ID NOs: 41 and 43 to 60 and SEQ ID NOs: 92 and 94 to 101; and at least one variable light chain (VL) region selected from the group consisting of SEQ ID NOs: 42 and 61 to 66 and SEQ ID NOs: 93 and 102 to 115.

18. An immune effector cell expressing the chimeric antigen receptor (CAR) of claim 17.

19. A method of treating an MYCT1-associated disease, comprising a step of administering to a subject a composition including a therapeutically effective amount of the antibody or antigen-binding fragment of any one of claims 1 to 6.

20. A method of enhancing sensitivity of a subject to an immunotherapy agent, comprising a step of administering to the subject a composition including a therapeutically effective amount of the antibody or antigen-binding fragment of any one of claims 1 to 6.

FIG. 1

FIG. 2

FIG. 3A

FIG. 3B

FIG. 3C

FIG. 4A

FIG. 4B

FIG. 5A

FIG. 5B

FIG. 5C

FIG. 5D

13. 2B4-z5-ulgG1K, 100.00%

14. 2B4-z6-ulgG1K, 97.42%

15. 2B4-z7-ulgG1K, 100.00%

16. 2B4-z8-ulgG1K, 100.00%

EP 4 737 482 A1

FIG. 5E

17. 2B4-z9-uIgG1K, 96.85%

DAD1E, Sig=280,4 Ref=off, (2B4-z9uIgG1K.dx)

7.827, 96.85%
8.387, 2.36%
9.244, 0.75%
10.290, 0.04%

18. 2B4-z10-uIgG1K, 99.72%

DAD1E, Sig=280,4 Ref=off, (2B4-z10uIgG1K.dx)

7.831, 99.72%
6.592, 0.18%
5.475, 0.10%

19. 2B4-z11-uIgG1K, 100.00%

DAD1E, Sig=280,4 Ref=off, (2B4-z11uIgG1K.dx)

7.834, 100.00%

20. 2B4-z12-uIgG1K, 99.94%

DAD1E, Sig=280,4 Ref=off, (2B4-z12uIgG1K.dx)

7.833, 99.94%
5.492, 0.06%

EP 4 737 482 A1

FIG. 5F

FIG. 6A

1. 11B6, 97.63%

DAD1E, Sig=280,4 Ref=off, (11B6-dx)

7.893, 97.63%

6.504, 2.01%
5.947, 0.25%
5.511, 0.11%

2. 11B6-p1-uIgG1K, 98.92%

DAD1E, Sig=280,4 Ref=off, (11B6-p1-uIgG1K.dx)

7.876, 98.92%

6.496, 1.08%

3. 11B6-p2-uIgG1K, 98.86%

DAD1E, Sig=280,4 Ref=off, (11B6-p2-uIgG1K.dx)

7.896, 98.86%

6.507, 1.14%

4. 11B6-p3-uIgG1K, 97.24%

DAD1E, Sig=280,4 Ref=off, (11B6-p3-uIgG1K.dx)

7.909, 97.24%

6.506, 2.21%
5.931, 0.29%
5.526, 0.26%

FIG. 6B

5. 11B6-z0-uIgG1K, 98.72%

DAD1E, Sig=280,4 Ref=off, (11B6-z0-uIgG1K.dx)

6. 11B6-z1-uIgG1K, 99.08%

DAD1E, Sig=280,4 Ref=off, (11B6-z1-uIgG1K.dx)

7. 11B6-z2-uIgG1K, 98.07%

DAD1E, Sig=280,4 Ref=off, (11B6-z2-uIgG1K.dx)

8. 11B6-z3-uIgG1K, 99.01%

DAD1E, Sig=280,4 Ref=off, (11B6-z3-uIgG1K.dx)

EP 4 737 482 A1

FIG. 6C

FIG. 6D

FIG. 6E

FIG. 7

FIG. 8

1. 2B4, 89.97%

DAD1E, Sig=280,4 Ref=off, (2B4.dx)

7.869, 89.97%
9.233, 1.30%
9.316, 4.67%
10.006, 1.14%
10.156, 2.58%
6.043, 0.17%
5.622, 0.17%

Time [min]

2. 2B4-z2-p7-uIgG1K, 99.76%

DAD1E, Sig=280,4 Ref=off, (2B4-z2-p7-uIgG1K.dx)

7.741, 99.76%
6.439, 0.08%
5.638, 0.15%

Time [min]

3. 2B4-z8-p7-uIgG1K, 98.05%

DAD1E, Sig=280,4 Ref=off, (2B4-z8-p7-uIgG1K.dx)

7.740, 98.05%
9.066, 1.54%
9.916, 0.09%
9.986, 0.08%
6.551, 0.07%
5.636, 0.17%

Time [min]

FIG. 9

# FIG. 10

1. 11B6, 95.27%

2. 11B6-z10-p3-ulgG1K, 98.04%

3. 11B6-z12-p3-ulgG1K, 97.53%

4. 11B6-z16-p3-ulgG1K, 98.18%

EP 4 737 482 A1

FIG. 11

FIG. 12

FIG. 13A

Binding of 2B4 to hMYCT1

Binding of 2B4 -p1-uIgG1K
to hMYCT1 1

Binding of 2B4-p2-uIgG1K
to W30058-hPep1

EP 4 737 482 A1

FIG. 13B

FIG. 13C

Binding of 2B4 -p6-uIgG1K
to hMYCT1

FIG. 14A

FIG. 14B

Binding of 2B4 -z4-uIgG1K
to hMYCT1

Binding of 2B4 -z5-uIgG1K
to hMYCT1

Binding of 2B4 -z6-uIgG1K
to hMYCT1

EP 4 737 482 A1

FIG. 14C

Binding of 2B4 -z7-uIgG1K
to hMYCT1

FIG. 14D

Binding of 2B4
to hMYCT1

Binding of 2B4 -z8-ulgG1K
to hMYCT1

Binding of 2B4 -z10-ulgG1K
to hMYCT1

FIG. 14E

Binding of 2B4 -z11-uIgG1K
to hMYCT1

Binding of 2B4 -z12-uIgG1K
to hMYCT1

Binding of 2B4 -z13-uIgG1K
to hMYCT1

EP 4 737 482 A1

FIG. 14F

FIG. 15

FIG. 16

hMYCT1 1-1;
11B6; 1:1 binding

hMYCT1 1-1;
11B6 -p1-ulgG1K;
1:1 binding

hMYCT1 1-1;
11B6 -p2-ulgG1K;
1:1 binding

hMYCT1 1-1;
11B6 -p2-ulgG1K;
1:1 binding

hMYCT1 1-1;
11B6 -z0-ulgG1K;
1:1 binding

hMYCT1 1-1;
11B6 -z1-ulgG1K;
1:1 binding

hMYCT1 1-1;
11B6 -z2-ulgG1K;
1:1 binding

hMYCT1 1-1;
11B6 -z3-ulgG1K;
1:1 binding

hMYCT1 1-1;
11B6 -z4-ulgG1K;
1:1 binding

hMYCT1 1-1;
11B6 -z5-ulgG1K;
1:1 binding

hMYCT1 1-1;
11B6 -z6-ulgG1K;
1:1 binding

hMYCT1 1-1;
11B6 -z7-ulgG1K;
1:1 binding

hMYCT1 1-1;
11B6 -z8-ulgG1K;
1:1 binding

hMYCT1 1-1;
11B6 -z9-ulgG1K;
1:1 binding

hMYCT1 1-1;
11B6 -z10-ulgG1K;
1:1 binding

hMYCT1 1-1;
11B6 -z11-ulgG1K;
1:1 binding

hMYCT1 1-1;
11B6 -z12-ulgG1K;
1:1 binding

hMYCT1 1-1;
11B6 -z13-ulgG1K;
1:1 binding

hMYCT1 1-1;
11B6 -z14-ulgG1K;
1:1 binding

hMYCT1 1-1;
11B6 -z15-ulgG1K;
1:1 binding

FIG. 17

Binding of 11B6 to hMYCT1

Binding of 11B6-z10-p3-ulgG1K to hMYCT1

Binding of 11B6-z12-p3-ulgG1K to hMYCT1

Binding of 11B6-z16-p3-ulgG1K to hMYCT1

FIG. 18

FIG. 19A

FIG. 19B

11B6 (Run 2)
Melt Curve Plot
48.16

11B6-z10-p3-uIgG1K (Run 2)
Melt Curve Plot
71.02

11B6-z12-p3-uIgG1K (Run 2)
Melt Curve Plot
71.11

11B6-z16-p3-uIgG1K (Run 2)
Melt Curve Plot
70.56

FIG. 20

EP 4 737 482 A1

- NCI-H460

| | blank | 1000 | 625 | 312.5 | 156.25 | 78.125 | 39.06 | 19.53 | 9.77 | 4.88 | 2.44 | 1.22 |
|---|---|---|---|---|---|---|---|---|---|---|---|---|
| 계열1 | 100 | 89.850 | 88.966 | 88.966 | 89.073 | 94.885 | 94.992 | 47.349 | 96.626 | 95.153 | 93.867 | 93.171 |

- HepG2

| | blank | 1000 | 625 | 312.5 | 156.25 | 78.125 | 39.06 | 19.53 | 9.77 | 4.88 | 2.44 | 1.22 |
|---|---|---|---|---|---|---|---|---|---|---|---|---|
| 계열1 | 100 | 101.424 | 100.119 | 98.4572 | 101.519 | 103.679 | 102.445 | 101.828 | 106.883 | 103.56 | 104.13 | 102.682 |

- MC38

| | blank | 1000 | 625 | 312.5 | 156.25 | 78.125 | 39.06 | 19.53 | 9.77 | 4.88 | 2.44 | 1.22 |
|---|---|---|---|---|---|---|---|---|---|---|---|---|
| 계열1 | 100 | 102.77 | 104.805 | 105.399 | 104.692 | 106.077 | 100.283 | 99.1803 | 104.777 | 105.794 | 107.292 | 104.777 |

- ACHN

| | blank | 1000 | 625 | 312.5 | 156.25 | 78.13 | 39.06 | 19.53 | 9.77 | 4.88 | 2.44 | 1.22 |
|---|---|---|---|---|---|---|---|---|---|---|---|---|
| 계열1 | 100 | 100.033 | 99.429 | 100.87 | 103.16 | 101.48 | 103.63 | 100.3 | 99.664 | 99.295 | 97.045 | 98.388 |

FIG. 21A

FIG. 21B

FIG. 22

EP 4 737 482 A1

FIG. 23A

- Syngeneic model: CT-26, Mouse Colon cancer (BalB/c)
- Chimeric ATN001(11B6) & Humanized ATN001(11B6-z16-p3)

Group1 (Vehicle)
Group2 (Anti-PD-1 10mg/kg)
Group3 (chATN001-clone#2 10mg/kg)
Group4 (hzATN001-clone#16 10mg/kg)
Group5 (Anti-PD-1 10mg/kg+chATN001-clone#2 10mg/kg)
Group6 (Anti-PD-1 10mg/kg+hzATN001-clone#16 10mg/kg)

FIG. 23B

- CT-26 mouse colon cancer cell line (5x10^5 cells)
- Balb/c 6wk female
- Drug treatment: IP inj.
- Drug: Anti-PD1, 11B6(Chimeric),
        hzATN001(Humanized 11B6-z16-p3)
- Drug dose: 10mg/kg
- Data represent the Mean ± SEM (n= 7/group)

## INTERNATIONAL SEARCH REPORT

International application No.

**PCT/KR2024/008897**

| A. | CLASSIFICATION OF SUBJECT MATTER |
|---|---|

**C07K 16/30**(2006.01)i; **C07K 14/725**(2006.01)i; **G01N 33/574**(2006.01)i; **A61P 35/00**(2006.01)i; **A61K 47/68**(2017.01)i; **C12N 15/113**(2010.01)i; **A61K 39/00**(2006.01)i

According to International Patent Classification (IPC) or to both national classification and IPC

| B. | FIELDS SEARCHED |
|---|---|

Minimum documentation searched (classification system followed by classification symbols)

C07K 16/30(2006.01); A61K 38/01(2006.01); A61K 39/395(2006.01); A61K 45/06(2006.01); A61K 48/00(2006.01); A61P 35/00(2006.01); C07K 14/47(2006.01); C12N 15/113(2010.01); C12Q 1/68(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: MYC 표적-1(MYC target-1, MYCT1), 항-MYCT1 항체(anti-MYCT1 antibody), 가변 중쇄 영역(variable heavy chain region), 가변 경쇄 영역(variable light chain region), 인간화 항체(humanized antibody), 혈관신생(angiogenesis)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | US 2022-0143220 A1 (WASHINGTON UNIVERSITY) 12 May 2022 (2022-05-12)<br>See paragraphs [0003]-[0057] and [0157]-[0174]. | 1-18 |
| A | EP 2962694 A1 (UNIVERSITÉ DE LAUSANNE) 06 January 2016 (2016-01-06)<br>See paragraphs [0024] and [0071]. | 1-18 |
| A | US 2011-0117083 A1 (BAIS, Carlos et al.) 19 May 2011 (2011-05-19)<br>See paragraph [0006]. | 1-18 |
| A | US 2022-0025369 A1 (CUREVAC AG) 27 January 2022 (2022-01-27)<br>See paragraphs [0064], [0078] and [0081]. | 1-18 |
| A | KABIR, Ashraf Ul et al. Dual role of endothelial Myct1 in tumor angiogenesis and tumor immunity. Science Translational Medicine. 03 March 2021 (publication date), article no. eabb6731, inner pp. 1-18.<br>See abstract. | 1-18 |

☐ Further documents are listed in the continuation of Box C.       ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **30 September 2024** | **02 October 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

| International application No. |
|---|
| **PCT/KR2024/008897** |

**Box No. I        Nucleotide and/or amino acid sequence(s) (Continuation of item 1.c of the first sheet)**

1. With regard to any nucleotide and/or amino acid sequence disclosed in the international application, the international search was carried out on the basis of a sequence listing:

   a.  ☑  forming part of the international application as filed.

   b.  ☐  furnished subsequent to the international filing date for the purposes of international search (Rule 13*ter*.1(a)),

      ☐  accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

| International application No. |
| --- |
| **PCT/KR2024/008897** |

**Box No. II    Observations where certain claims were found unsearchable (Continuation of item 2 of first sheet)**

This international search report has not been established in respect of certain claims under Article 17(2)(a) for the following reasons:

1. ☑ Claims Nos.: **19,20**
   because they relate to subject matter not required to be searched by this Authority, namely:

   Claims 19 and 20 pertain to a method for treatment of the human body by therapy (PCT Article 17(2)(a)(i) and PCT Rule 39.1(iv)).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

## INTERNATIONAL SEARCH REPORT
### Information on patent family members

| International application No. |
| --- |
| **PCT/KR2024/008897** |

| Patent document cited in search report | | | Publication date (day/month/year) | Patent family member(s) | | | Publication date (day/month/year) |
| --- | --- | --- | --- | --- | --- | --- | --- |
| US | 2022-0143220 | A1 | 12 May 2022 | None | | | |
| EP | 2962694 | A1 | 06 January 2016 | None | | | |
| US | 2011-0117083 | A1 | 19 May 2011 | CN | 102575298 | A | 11 July 2012 |
| | | | | EP | 2464744 | A1 | 20 June 2012 |
| | | | | JP | 2013-501526 | A | 17 January 2013 |
| | | | | KR | 10-2012-0059553 | A | 08 June 2012 |
| | | | | WO | 2011-020049 | A1 | 17 February 2011 |
| US | 2022-0025369 | A1 | 27 January 2022 | EP | 3452101 | A2 | 13 March 2019 |
| | | | | US | 11078247 | B2 | 03 August 2021 |
| | | | | US | 2019-0241633 | A1 | 08 August 2019 |
| | | | | WO | 2017-191274 | A2 | 09 November 2017 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20230082857
- KR 20230094413
- WO 2001077342 A
- WO 2009080251 A
- WO 2009080252 A
- WO 2009080253 A
- WO 2009080254 A
- WO 2010112193 A
- WO 2010115589 A

- WO 2010136172 A
- WO 2010145792 A
- WO 2010145793 A
- WO 2011117330 A
- US 20170368169 A
- WO 2004010957 A
- WO 2015182984 A
- US 4737456 A

**Non-patent literature cited in the description**

- *J. Cell. Mol. Med*, 2016, vol. 20 (3), 471-481
- **CHOTHIA et al.** *Nature*, 1989, vol. 342, 877-883
- **AL-LAZIKANI et al.** Standard conformations for the canonical structures of immunoglobulins. *Journal of Molecular Biology*, 1997, vol. 273, 927-948

- **KABAT et al.** Sequences of Proteins of Immunological Interest. *U.S. Department of Health and Human Services, National Institutes of Health*, 1987